# EUROPEAN PATENT APPLICATION

(11) **EP 4 793 641 A1**
(43) Date of publication of application: **19.08.2026**
(21) Application number: 24877256.8
(22) Date of filing: 10.10.2024
(51) Int. Cl.: G01N 33/50, C12N 9/00, C12N 11/02, C12N 15/09, C12Q 1/00, C12Q 1/68, G01N 21/64, G01N 33/15, G01N 33/68, G01N 33/557

(54) **NOVEL METHOD FOR ANALYZING BIOMOLECULAR INTERACTIONS**

(30) Priority: 10.10.2023 JP 2023175595; 10.10.2023 JP 2023175598; 18.12.2023 JP 2023213310
(71) Applicant: Kyoto University, Kyoto-shi, Kyoto 606-8501 (JP)
(72) Inventor: FUJITA, Daishi, Kyoto-shi, Kyoto 606-8501 (JP); YOSHIMURA, Masahiko, Kyoto-shi, Kyoto 606-8501 (JP); MATSUDA, Fuyuki, Kyoto-shi, Kyoto 606-8501 (JP); IKEDA, Yoshiki, Kyoto-shi, Kyoto 606-8501 (JP)
(74) Representative: Murgitroyd & Company
(86) International application number: PCT/JP2024/036365
(87) International publication number: WO 2025/079662

(57) **Abstract**

The present disclosure provides a method for designing structures useful for forming interaction complexes, manufactured structures, and uses thereof. One aspect of the present disclosure provides a design and a fabrication of the structure described in the present specification. Since a unit molecule is to be observed or used, the structure can be designed to fit the unit molecule. In one embodiment, the structure for causing formation of complexes among the unit molecules can be designed. The structures described in the present specification may have a function of promoting the formation of complexes among the unit molecules.

## Description

### [Technical Field]

The present disclosure relates to a method of designing a structure that is useful in forming an interaction complex, the fabricated structure, and use thereof. The present disclosure also relates to a novel method for analyzing a complex using such a structure, especially to the search and development of drugs.

### [Background Art]

Many molecules such as biomolecules exert their function through an interaction with another molecule. An interaction between molecules results in the formation of a complex, although this can be for a long or short period of time. Thus, complex formation can be critical for a molecule to exert its function, but the development of technologies for observing or utilizing complex formation is still ongoing. For example, elemental analysis, mass spectrometry, nuclear magnetic resonance (NMR), powder X-ray analysis, etc. are utilized as methods of identifying the structure of a complex. Each of such methods obtains information from different aspects. For example, Patent Literature 1 describes analysis on complexes of an IgE antibody and a target molecule thereof through microscopy. Since interaction complexes are comprised of multiple molecules, the size would be large, rendering analysis thereon to be challenging. Further, many drugs exert their action by intervening with intermolecular interactions (complex formation).

### [Citation List]

### [Patent Literature]

[PTL 1] EP Patent Application Publication No. 18215047

### [Summary of Invention]

### [Solution to Problem]

The inventors have developed a method of designing and making a novel structure for causing the formation of an interaction complex and found that the structure is useful in various uses. In particular, a complex can be analyzed by using a structure to cause formation of a complex at a predefined position and measuring the predefined position. This can be useful in the search and development of drugs.

The present disclosure provides, for example, the following items.

### (Item A1)

A method of making a structure for causing the formation of an interaction complex,
wherein the structure comprises a substrate comprising a first linker immobilization point and a second linker immobilization point,
wherein the first linker immobilization point and the second linker immobilization point can bind to or are attached to a first linker and a second linker, respectively, and the first linker and the second linker can bind to or are attached to a first unit molecule and a second unit molecule, respectively,
wherein a relative positional relationship between the first linker immobilization point and the second linker immobilization point is fixed,
wherein the first unit molecule and the second unit molecule can together form an interaction complex, and
wherein the method comprises a step of designing the structure based on the interaction complex.

### (Item A2)

The method of any preceding item A, wherein the step of designing the structure comprises designing the structure which is structured so that a crossing index for the first unit molecule and the second unit molecule is -5 *≤* D *≤* 15.

### (Item A3)

The method of any preceding item A, wherein the step of designing the structure comprises designing the structure based on sizes of the first unit molecule and the second unit molecule.

### (Item A4)

The method of any preceding item A, wherein the step of designing the structure comprises:
designing the structure based on a predicted value of a binding dissociation constant between the first unit molecule and the second unit molecule.

### (Item A5)

The method of any preceding item A, wherein the step of designing the structure comprises determining lengths of the first linker and the second linker based on at least one of: sizes of the first unit molecule and the second unit molecule; and a binding dissociation constant between the first unit molecule and the second unit molecule.

### (Item A6)

The method of any preceding item A, wherein the step of designing the structure comprises determining a distance between the first linker immobilization point and the second linker immobilization point based on at least one of: sizes of the first unit molecule and the second unit molecule; and a binding dissociation constant between the first unit molecule and the second unit molecule.

### (Item A7)

The method of any preceding item A, wherein the step of designing the structure comprises choosing the substrate from a plurality of options based on sizes of the first unit molecule and the second unit molecule.

### (Item A8)

The method of any preceding item A, wherein the step of designing the structure comprises:
choosing the substrate from a plurality of options;
determining a distance between the first linker immobilization point and the second linker immobilization point; and
determining lengths of the first linker and the second linker.

### (Item A9)

A method of analyzing an interaction complex, comprising the steps of:
making the structure according to the method of item A1;
preparing a structure comprising the first unit molecule attached to the first linker immobilization point via the first linker and the second unit molecule attached to the second linker immobilization point via the second linker; and
analyzing the interaction complex.

### (Item A10)

The method of any preceding item A, of determining a binding dissociation constant between the first unit molecule and the second unit molecule.

### (Item A11)

The method of any preceding item A, wherein the binding dissociation constant (K_{d}) is within a range of 10⁻² to 10⁻⁶ M.

### (Item A12)

The method of any preceding item A, of determining the binding dissociation constant based on calibration curves created from a result for a reference unit molecule with a known binding dissociation constant.

### (Item A13)

The method of any preceding item A, of determining a structure of the interaction complex.

### (Item A14)

A method of conducting an immobilized enzymatic reaction, comprising the steps of:
making the structure according to the method of item A1; and
contacting the structure with a substrate for an enzymatic reaction;
wherein the interaction complex is an enzyme.

### (Item A15)

A structure for causing the formation of an interaction complex,
wherein the structure comprises a substrate comprising a first linker immobilization point and a second linker immobilization point,
wherein the first linker immobilization point and the second linker immobilization point can bind to or are attached to a first linker and a second linker, respectively, and the first linker and the second linker can bind to or are attached to a first unit molecule and a second unit molecule, respectively,
wherein a relative positional relationship between the first linker immobilization point and the second linker immobilization point is fixed, and
wherein the first unit molecule and the second unit molecule can together form an interaction complex.

### (Item A16)

The structure of any preceding item A, wherein the structure is structured so that a crossing index for the first unit molecule and the second unit molecule is -5 *≤* D *≤* 15.

### (Item A17)

The structure of any preceding item A made according to the method of item A1.

### (Item B1)

A method of analyzing an interaction complex, comprising the steps of:
(A) providing a structure for causing the formation of an interaction complex at a predefined position, the structure comprising:
   a substrate comprising a first linker immobilization point and a second linker immobilization point;
   a first linker and a second linker attached to the substrate via the first linker immobilization point and the second linker immobilization point, respectively; and
   a first unit molecule and a second unit molecule attached to the first linker and the second linker, respectively;
   wherein a relative positional relationship between the first linker immobilization point and the second linker immobilization point is fixed,
   wherein the first unit molecule and the second unit molecule can together form an interaction complex,
   wherein the predefined position is defined as a relative position with respect to the substrate, and
   wherein the interaction complex is formed at the predefined position;
(B) causing the formation of the interaction complex; and
(C) analyzing data derived from the interaction complex at the predefined position to analyze the interaction complex.

### (Item B2)

The method of any preceding item B, further comprising the step of irradiating a particle beam or an electromagnetic wave at the predefined position.

### (Item B3)

The method of any preceding item B, further comprising the step of collecting a particle beam or an electromagnetic wave from the interaction complex.

### (Item B4)

The method of any preceding item B, wherein the step of collecting an irradiated particle beam or an electromagnetic wave comprises using a cryogenic electron microscope.

### (Item B5)

The method of any preceding item B, of analyzing a three-dimensional structure of the interaction complex.

### (Item B6)

The method of any preceding item B, wherein data collected from a plurality of the structures is used to analyze a three-dimensional structure of the interaction complex.

### (Item B7)

The method of any preceding item B, further comprising the step of distinguishing the structures comprising the interaction complex and the structures free of the interaction complex.

### (Item B8)

The method of any preceding item B, of analyzing a attaching force between the first unit molecule and the second unit molecule.

### (Item B9)

The method of any preceding item B, further comprising the step of:
attaching the first unit molecule to the first linker; and/or
attaching the second unit molecule to the second linker.

### (Item B10)

The method of any preceding item B,
wherein the substrate further comprises a third linker immobilization point, a third linker is attached to the substrate via the third linker immobilization point, and a third unit molecule is attached to the third linker,
wherein a relative positional relationship between the first linker immobilization point, the second linker immobilization point, and the third linker immobilization point is fixed, and
wherein the first unit molecule, the second unit molecule, and the third unit molecule together form the interaction complex at the predefined position.

### (Item B11)

The method of any preceding item B, wherein the linker comprises a nucleic acid material.

### (Item B12)

The method of any preceding item B, wherein at least one of the first linker and the second linker has a capture moiety that non-covalently binds to the first unit molecule and/or the second unit molecule.

### (Item B13)

The method of any preceding item B, wherein the substrate is comprised of a nucleic acid material.

### (Item B14)

The method of any preceding item B, wherein the substrate comprises an opening.

### (Item B15)

The method of any preceding item B, wherein the predefined position is located at the opening.

### (Item B16)

The method of any preceding item B, wherein the substrate has a tubular structure or a sheet-like structure.

### (Item B17)

The method of any preceding item B, wherein the substrate comprises a cap.

### (Item B18)

The method of any preceding item B, wherein the cap is a polymer with a mesh structure.

### (Item B19)

The method of any preceding item B, wherein at least one of the first linker immobilization point and the second linker immobilization point is on the cap.

### (Item B20)

The method of any preceding item B, wherein the first unit molecule and the second unit molecule are linked by a linker.

### (Item B21)

The method of any preceding item B, wherein the structure is structured so that a crossing index for the first unit molecule and the second unit molecule is 0 *≤* D *≤* 10.

### (Item C1)

A method of screening for a drug, comprising the steps of:
(A) providing a structure for causing the formation of an interaction complex at a predefined position, the structure comprising:
   a substrate comprising a first linker immobilization point and a second linker immobilization point;
   a first linker and a second linker attached to the substrate via the first linker immobilization point and the second linker immobilization point, respectively; and
   a first unit molecule and a second unit molecule attached to the first linker and the second linker, respectively;
   wherein a relative positional relationship between the first linker immobilization point and the second linker immobilization point is fixed,
   wherein the first unit molecule and the second unit molecule can together form an interaction complex,
   wherein the predefined position is defined as a relative position with respect to the substrate, and
   wherein the interaction complex is formed at the predefined position;
(B) causing the formation of the interaction complex in the presence of a candidate drug; and
(C) analyzing data derived from the interaction complex at the predefined position to analyze an interaction between the candidate drug and the interaction complex.

### (Item C2)

A method of screening for a drug, comprising the steps of:
(A) providing a structure for causing the formation of an interaction complex at a predefined position, the structure comprising:
   a substrate comprising a first linker immobilization point and a second linker immobilization point;
   a first linker and a second linker attached to the substrate via the first linker immobilization point and the second linker immobilization point, respectively; and
   a first unit molecule and a second unit molecule attached to the first linker and the second linker, respectively;
   wherein the first unit molecule comprises a candidate drug,
   wherein a relative positional relationship between the first linker immobilization point and the second linker immobilization point is fixed,
   wherein the first unit molecule and the second unit molecule can together form an interaction complex,
   wherein the predefined position is defined as a relative position with respect to the substrate, and
   wherein the interaction complex is formed at the predefined position;
(B) causing the formation of the interaction complex; and
(C) analyzing data derived from the interaction complex at the predefined position to analyze the interaction complex.

### (Item C3)

The method of any preceding item C, comprising the steps of:
attaching the first unit molecule to a linker comprising a capture moiety capable of attaching to the first linker immobilization point; and
attaching the first linker attached to the first unit molecule to the first linker immobilization point to form the structure.

### (Item C4)

The method of any preceding item C, further comprising the step of collecting a particle beam or an electromagnetic wave from the interaction complex.

### (Item C5)

The method of any preceding item C, wherein a state where the interaction complex is formed is distinguished from a state where the interaction complex is not formed.

### (Item C6)

The method of any preceding item C, wherein the first linker or the first unit molecule comprises a first functional group, the second linker or the second unit molecule comprises a second functional group, and an interaction between the first functional group and the second functional group generates light or light of a specific wavelength.

### (Item C7)

The method of any preceding item C, wherein the first linker or the first unit molecule, or the second linker or the second unit molecule, comprises a first functional group, a third linker comprising a second functional group is immobilized to a third linker immobilization point on the substrate, and an interaction between the first functional group and the second functional group generates light or light of a specific wavelength.

### (Item C8)

The method of any preceding item C, of screening for a drug that inhibits or promotes the formation of the interaction complex.

### (Item C9)

A method of designing a drug, comprising the steps of:
(A) providing a structure for causing the formation of an interaction complex at a predefined position, the structure comprising:
   a substrate comprising a first linker immobilization point and a second linker immobilization point;
   a first linker and a second linker attached to the substrate via the first linker immobilization point and the second linker immobilization point, respectively; and
   a first unit molecule and a second unit molecule attached to the first linker and the second linker, respectively;
   wherein a relative positional relationship between the first linker immobilization point and the second linker immobilization point is fixed,
   wherein the first unit molecule and the second unit molecule can together form an interaction complex,
   wherein the predefined position is defined as a relative position with respect to the substrate,
   wherein the interaction complex is formed at the predefined position, and
   wherein the first unit molecule comprises a starting drug molecule;
(B) causing the formation of the interaction complex;
(C) analyzing data derived from the interaction complex at the predefined position to extract a pharmacophore of the starting drug molecule; and
(D) designing a drug by modifying the pharmacophore.

### (Item C10)

The method of any preceding item C, wherein the substrate comprises the first linker immobilization point at a plurality of different positions.

### (Item C11)

The method of any preceding item C, wherein the substrate comprises a plurality of types of substrates comprising the first linker immobilization point at positions that are different from each other.

### (Item C12)

The method of any preceding item, further comprising the step of classifying data for the particle beam or electromagnetic wave by each substrate having a linker attached thereto via the first linker immobilization point at different positions.

### [Advantageous Effects of Invention]

The present disclosure provides novel methods for analyzing a complex and can promote the elucidation of functions of a biomolecule, etc. The present disclosure can also promote the search or development of drugs, etc.

### [Brief Description of Drawings]

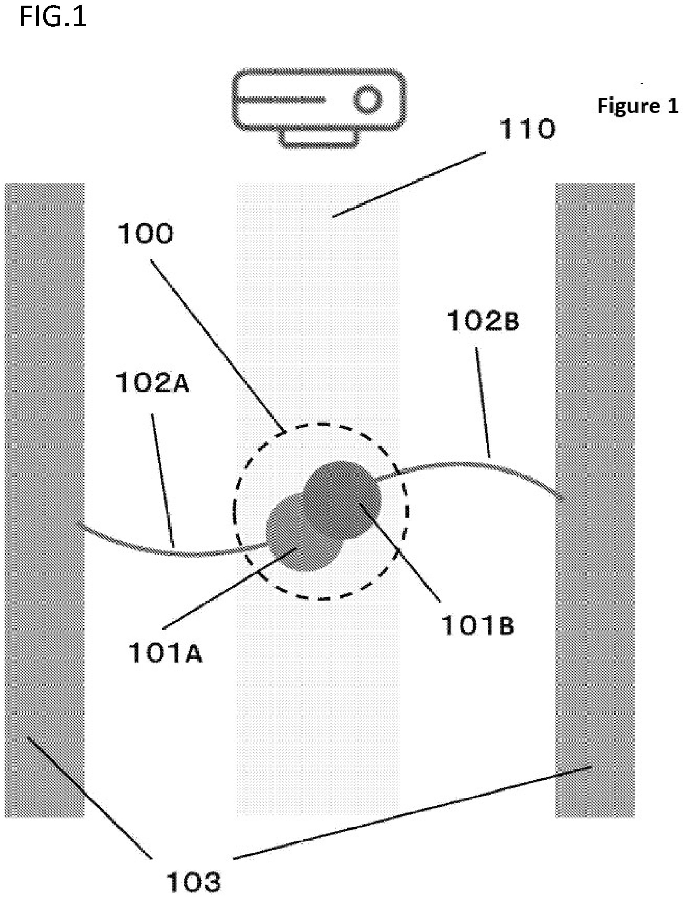

[Figure 1] Figure **1** shows an exemplary system for causing the formation of an interaction complex comprised of two unit molecules (**101A** and **101B**) and analyzing the complex. The unit molecules are attached to a substrate (**103**) via respective linkers (**102A** and **102B**). When an interaction complex is formed, two unit molecules are positioned at a predefined position (**100**). An interaction complex can be analyzed by measuring an electromagnetic wave or particle beam (**110**) irradiated to pass through the predefined position (**100**).

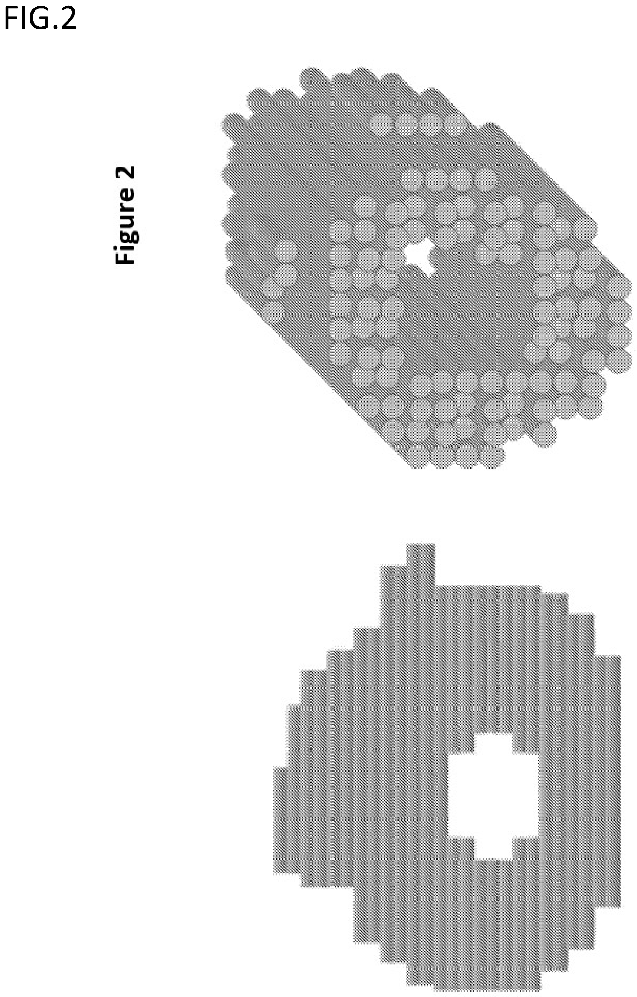

[Figure 2] Figure **2** shows an exemplary structure of a substrate.

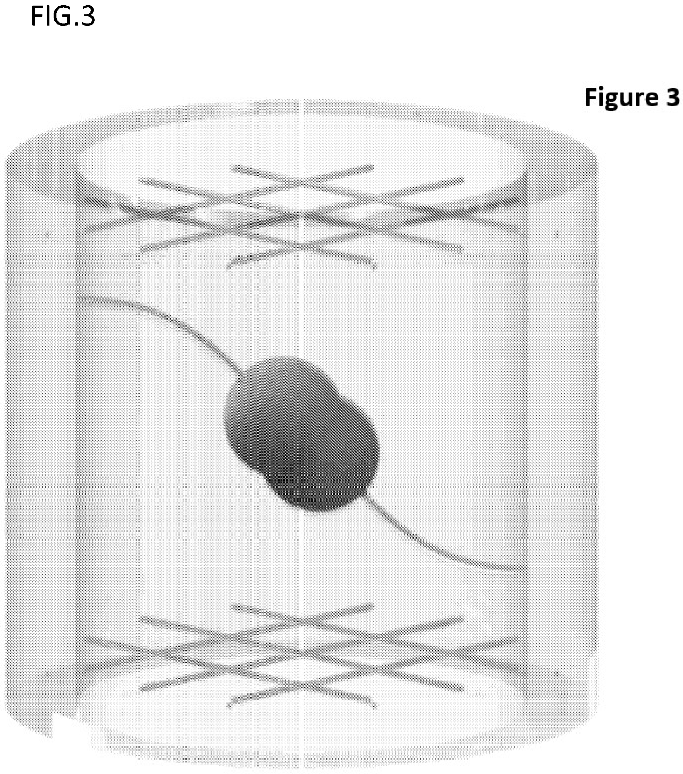

[Figure 3] Figure **3** shows an exemplary structure of a substrate comprising a cap.

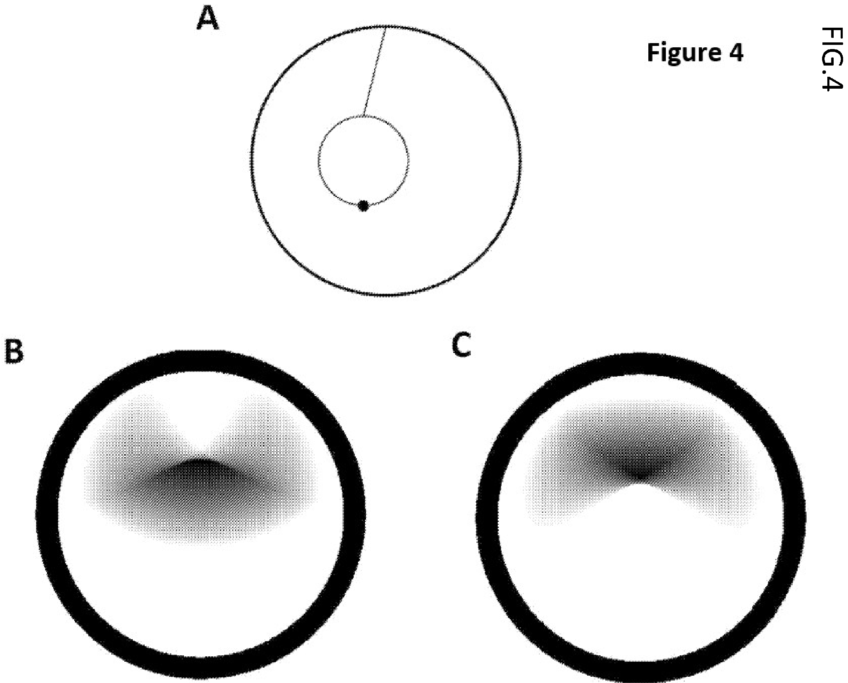

[Figure 4] A) is a schematic diagram of a system envisioned in a specific example of calculating a crossing index. B) is the probability distribution of an interaction point of particle **1** calculated by numerical simulation. C) is the probability distribution of an interaction point of particle **2** calculated by numerical simulation.

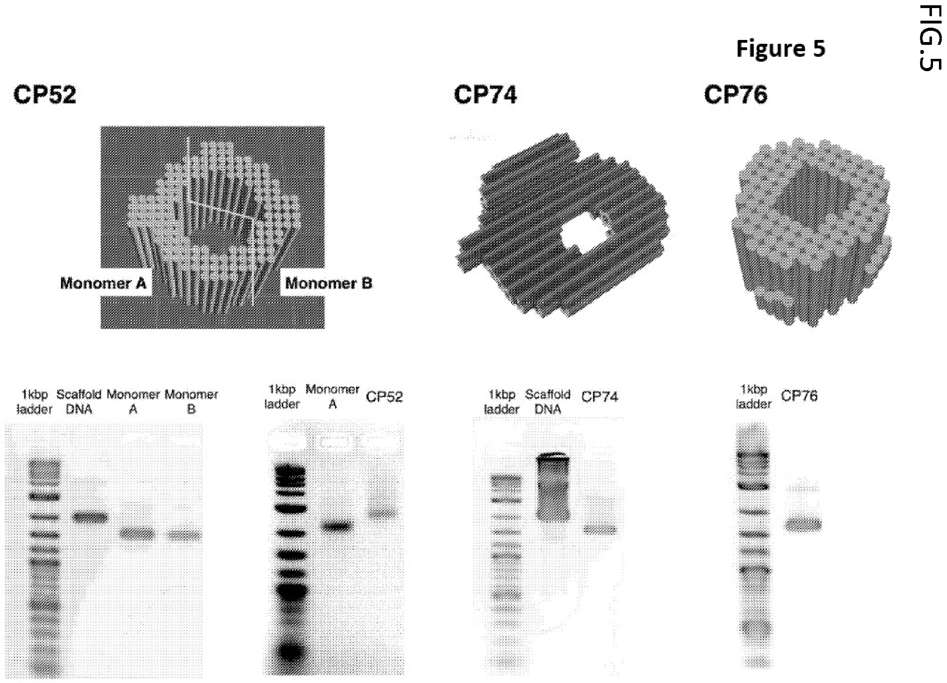

[Figure 5] The top half shows the structures of substrates constructed by using DNA origami in the Examples. The bottom half shows the results of gel electrophoresis confirming that each substrate was able to be formed.

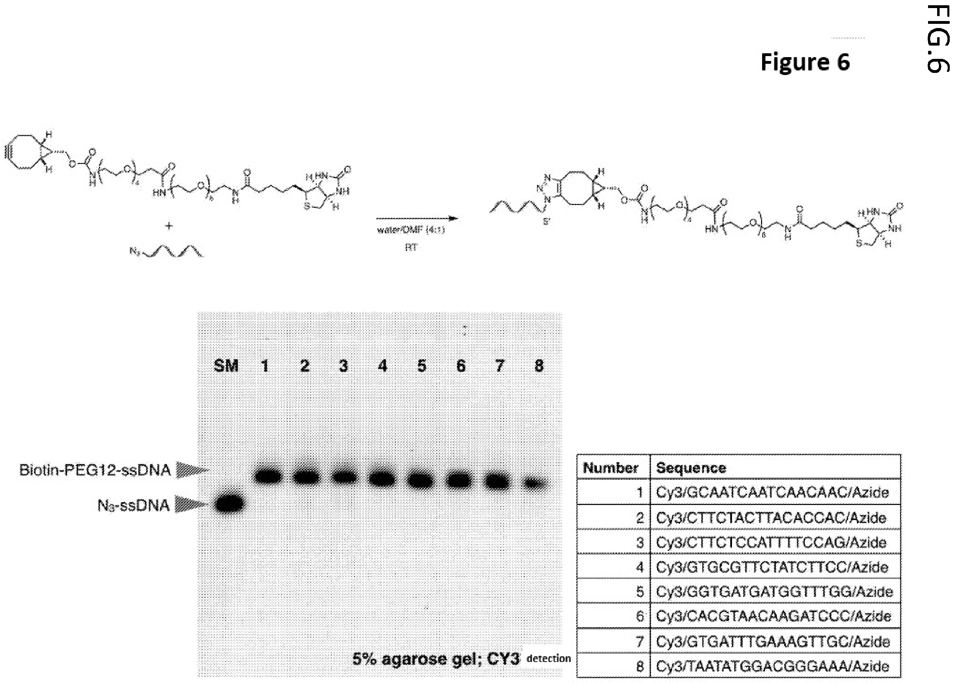

[Figure 6] The top half shows the outline of synthesis of unit molecule (biotin) + linker that can be attached to DNA origami (substrate) by utilizing the complementarity of nucleic acid bases. This is directed to a reaction for adding a complementary base sequence. The bottom half shows the results of gel electrophoresis confirming that each unit molecule + linker with additions of eight different base sequences was able to be formed.

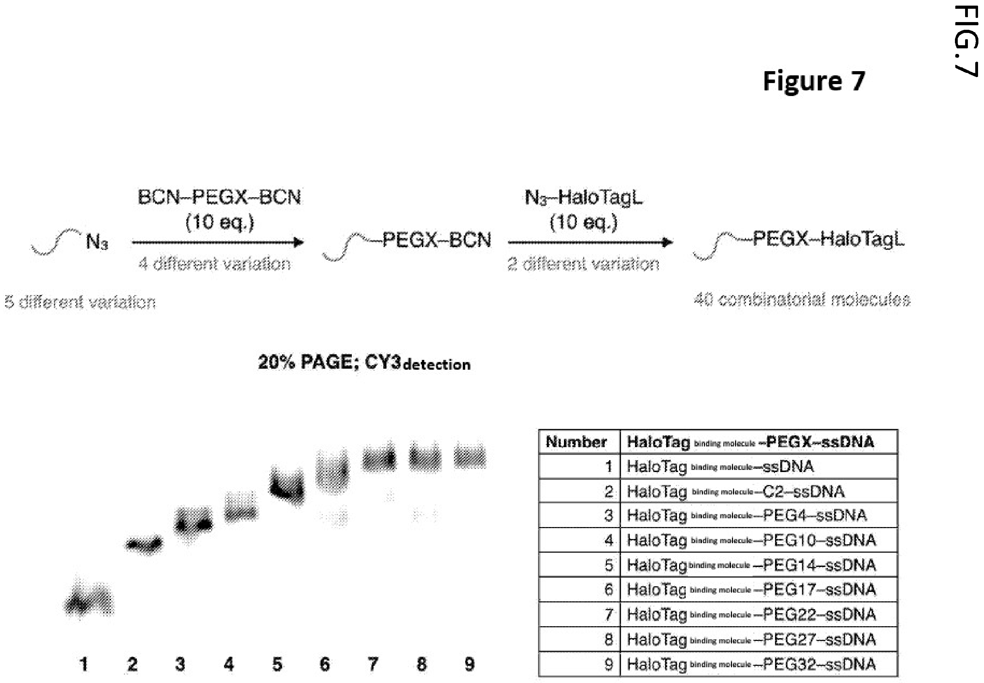

[Figure 7] The top half shows the outline of synthesis of unit molecule (HaloTag) + linker that can be attached to DNA origami (substrate) by utilizing the complementarity of nucleic acid bases. This is directed to a reaction for adding PEG with various lengths to a complementary base sequence and a reaction for attaching HaloTag to the PEG. The bottom half shows the results of gel electrophoresis confirming that each unit molecule + linker with HaloTag linked to a base sequence via PEG with various lengths was able to be formed.

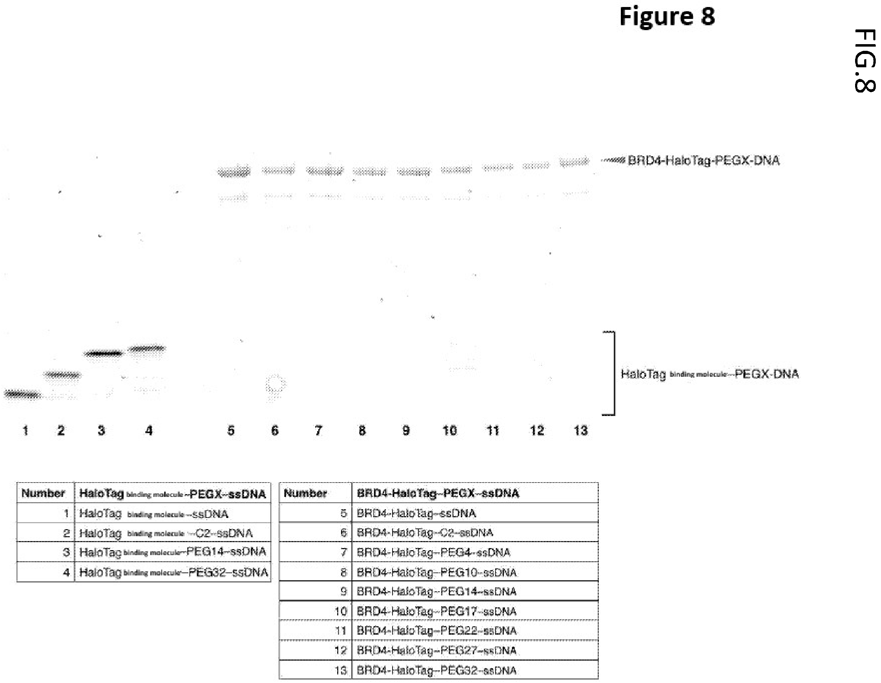

[Figure 8] Figure **8** shows the results of gel electrophoresis confirming that each unit molecule + linker with BRD4 further attached to the HaloTag moiety in the unit molecule (HaloTag) + linker in Figure **7** was able to be formed.

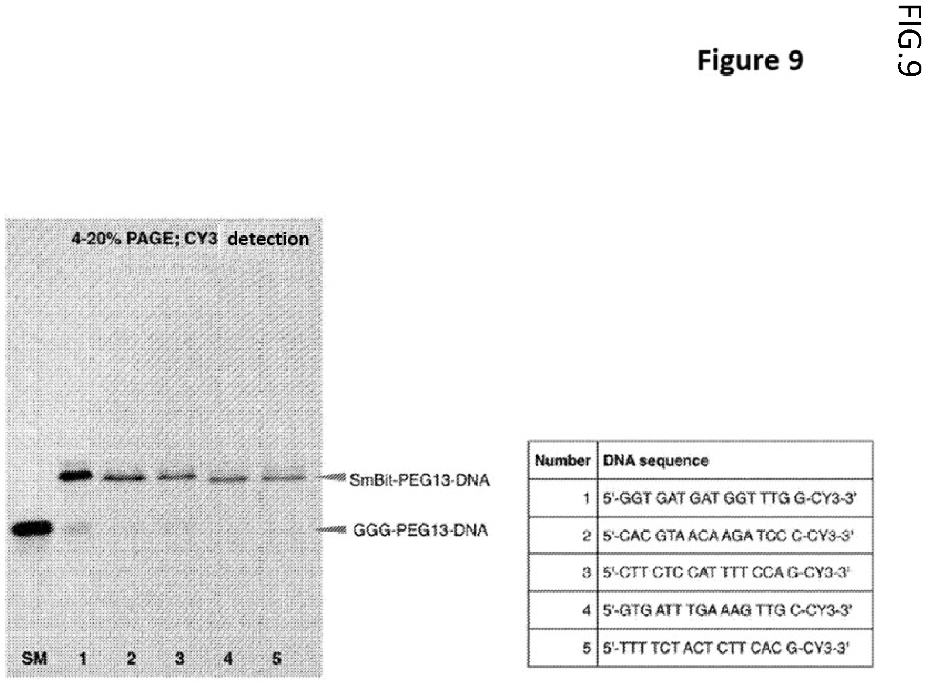

[Figure 9] Figure **9** shows the results of gel electrophoresis confirming that each unit molecule + linker of SmBit-PEG13-DNA (5 different base sequences) was able to be formed.

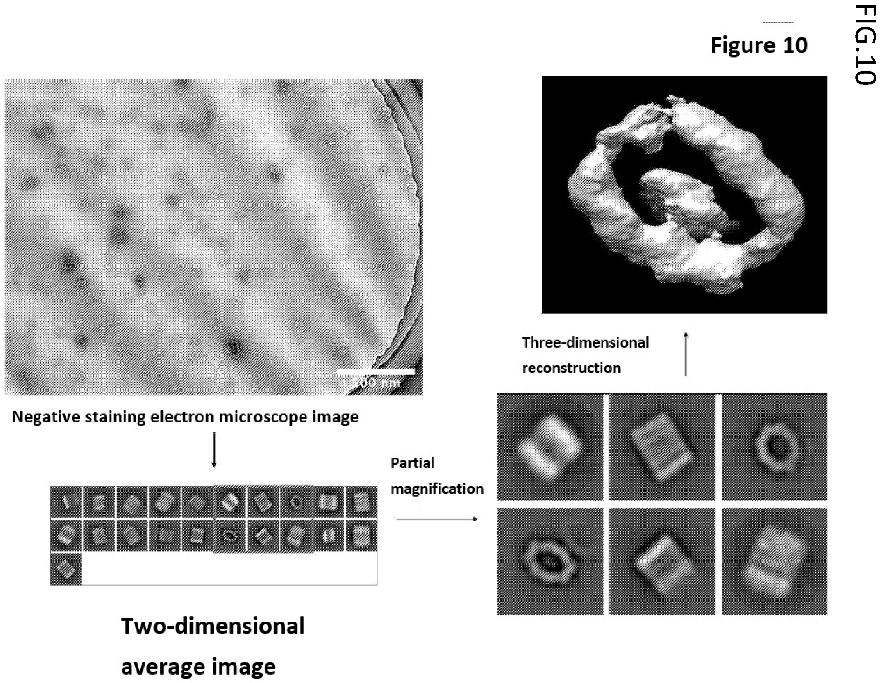

[Figure 10] Figure **10** is an example of an image of a structure observed through a cryo-transmission electron microscope. An image in which an interaction complex is formed in a structure is found.

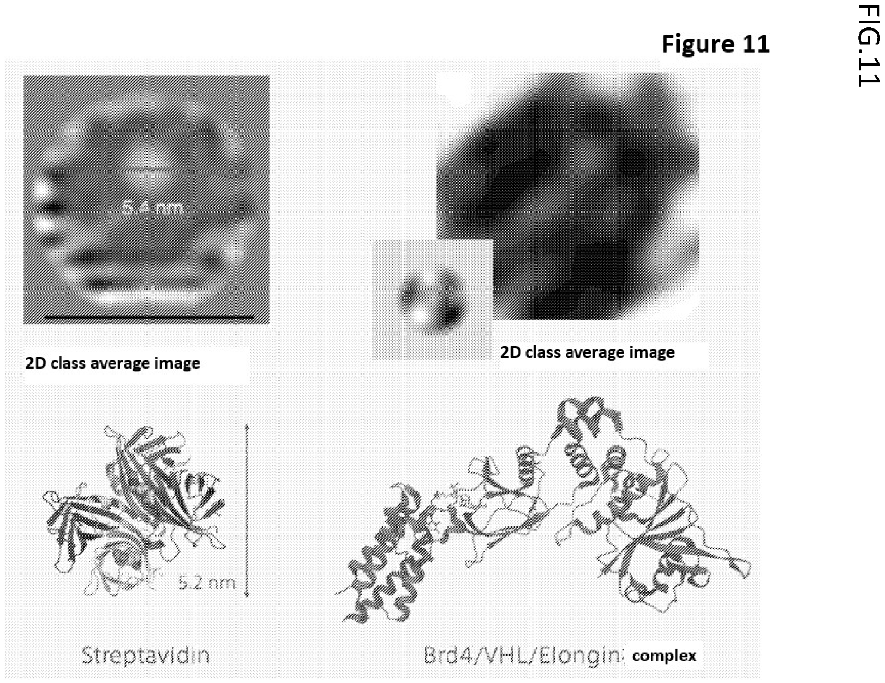

[Figure 11] Figure **11** is an example of an image of a structure observed through a cryo-transmission electron microscope. It can be observed that a biotin-streptavidin interaction complex (left) and Brd4/VHL/Elongin interaction complex (right), respectively, is formed in a structure and has a different shape from each other.

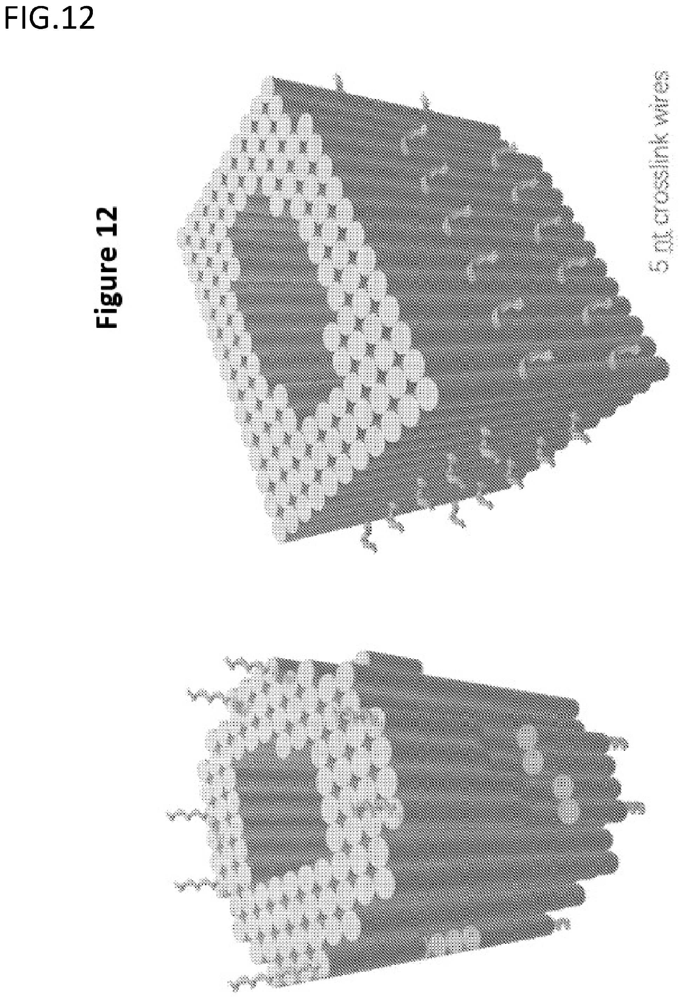

[Figure 12] Figure **12** is a schematic diagram of DNA origami configured to be able to link to each other. The DNA origami on the left can be linked to each other by using a linker, and the DNA origami on the right can be linked to each other without a linker.

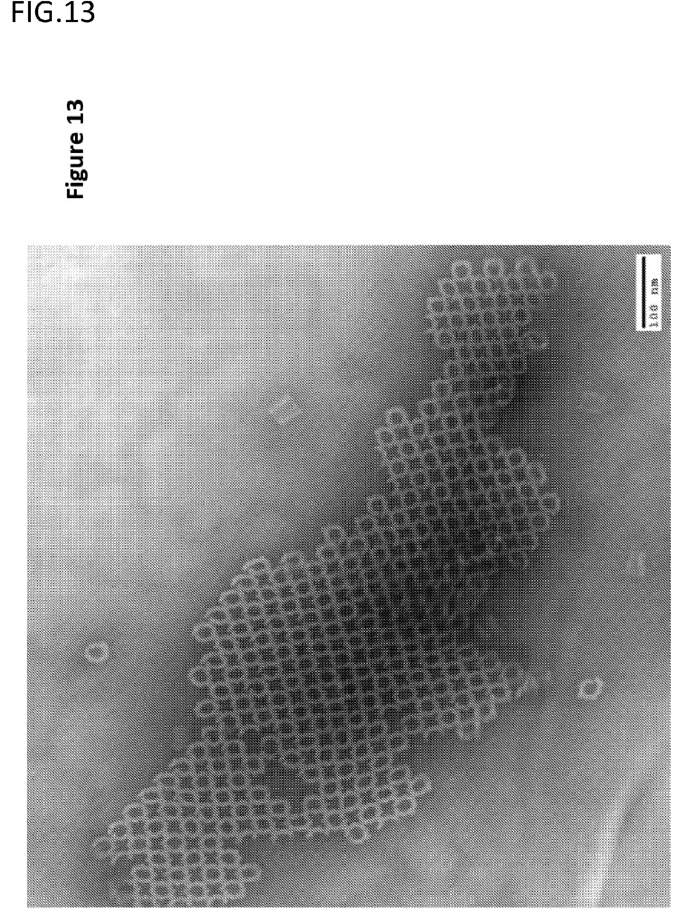

[Figure 13] Figure **13** is a cryo-transmission electron microscope picture of a cluster formed by linking DNA origami substrates in side by side manner.

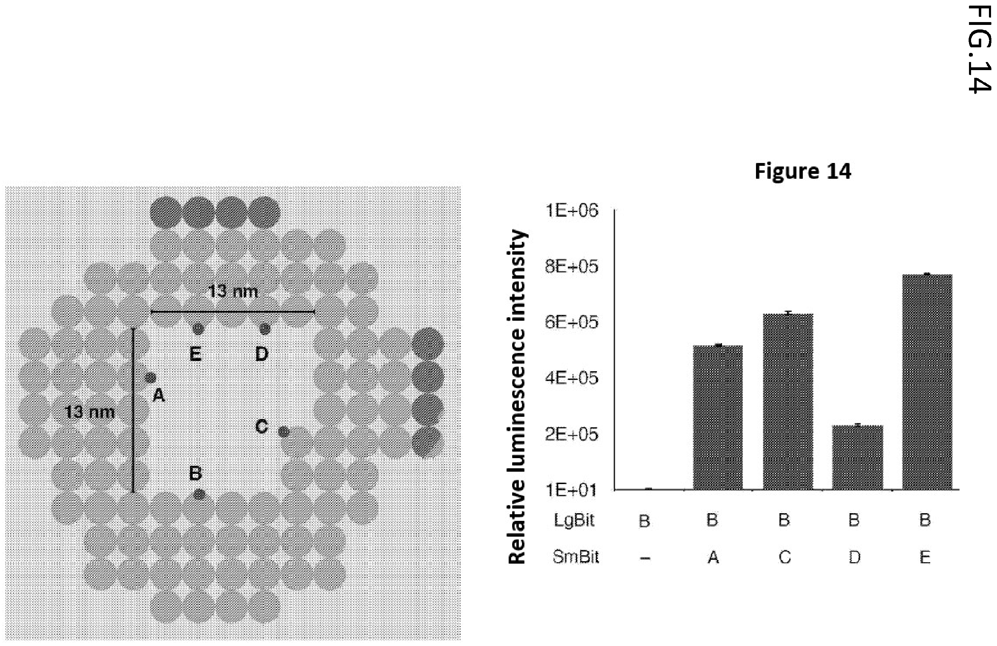

[Figure 14] The left side is a schematic diagram showing attaching sites for unit molecule + linker in a DNA origami substrate. The attaching sites in the substrate vary depending on the base sequence of ssDNA in the unit molecule + linker. The right side shows the relative luminescence intensity observed when using a combination of unit molecule (LgBit) + linker constructed to bind to attaching site B and unit molecule (SmBit) + linker constructed to bind to attaching site A, C, D, or E.

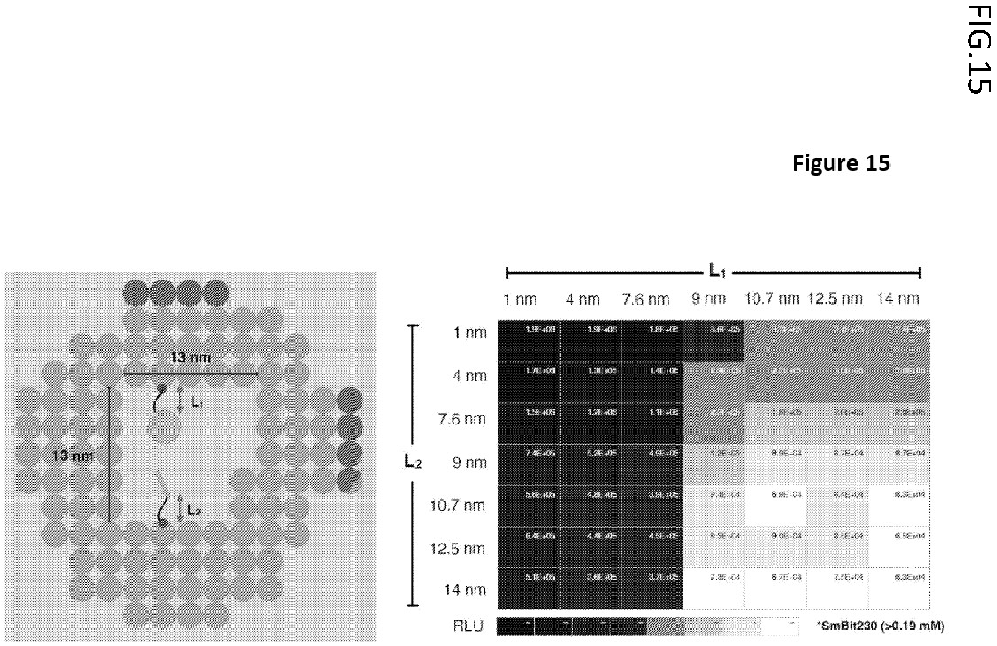

[Figure 15] The left side is a schematic diagram showing a system wherein two types of unit molecules forming an interaction complex are attached to a DNA origami substate via linkers with various lengths. The right side shows the relative luminescence intensity observed when using combinations of respective linker lengths. Darker colors indicate stronger luminescence intensities.

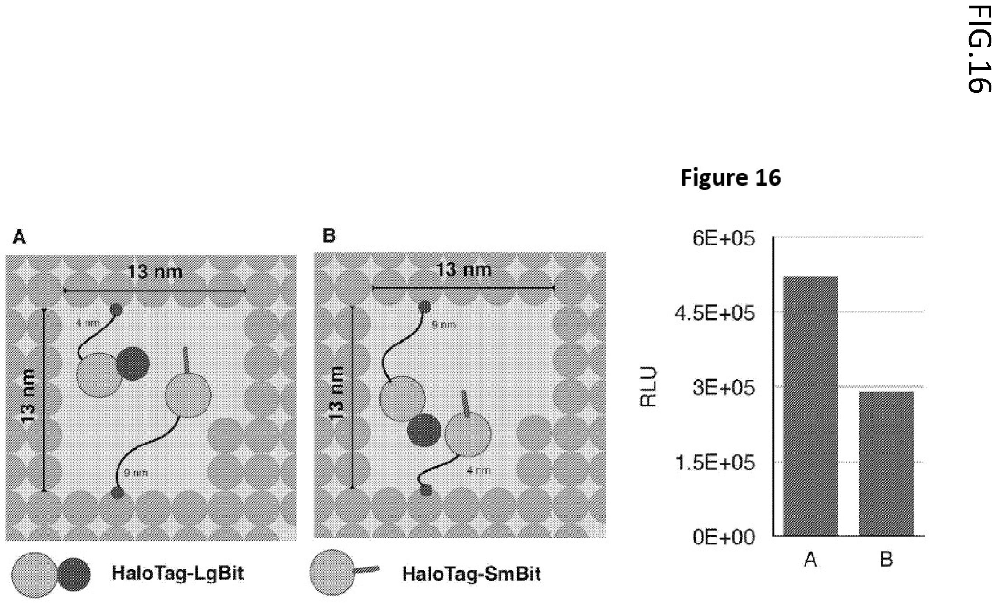

[Figure 16] Figure **16** is a diagram showing that the binding state varies when the attaching positions of the unit molecules are interchanged, even for the same attaching points and linkers.

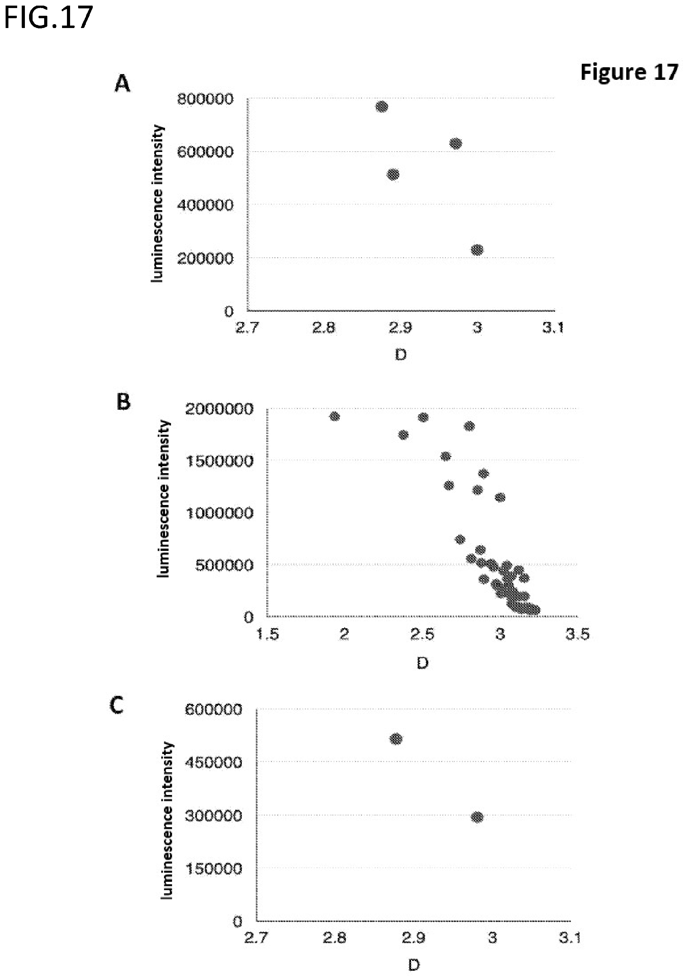

[Figure 17] Figure **17** is a diagram plotting the results of Figure **14** (A), Figure **15** (B), and Figure **16** (C) in relation to D.

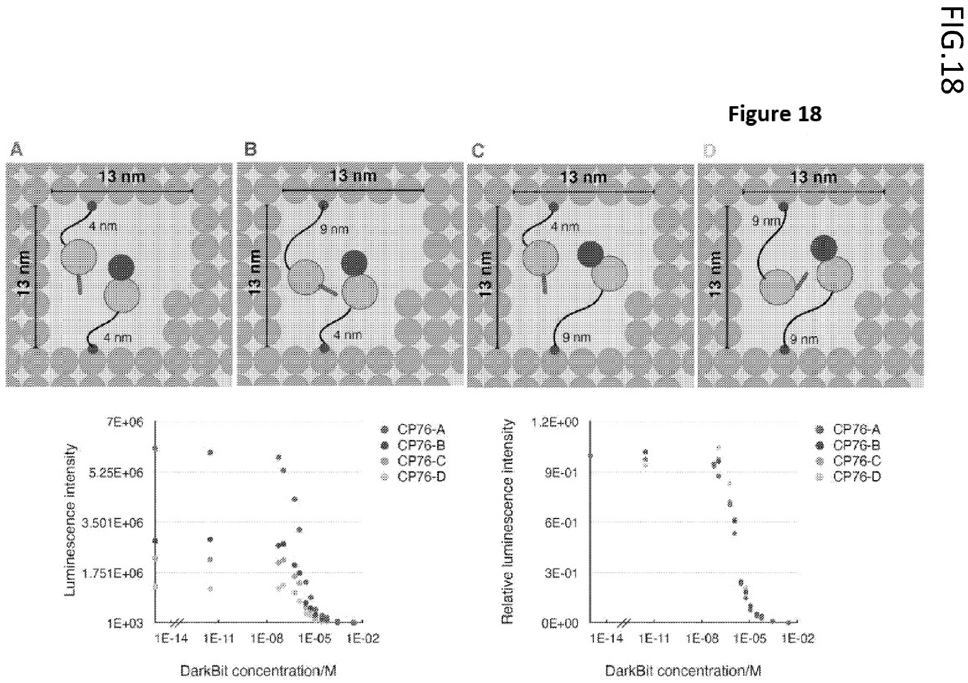

[Figure 18] Figure **18** shows results of studying the effect of an inhibitor by using complex formation in a substrate of the present disclosure. The top half is a schematic diagram showing four systems with varying linker lengths. The bottom left graph shows the correlation between the concentration of added inhibitor and luminescence intensity in each system. The bottom right graph shows the results of the bottom left graph converted to relative luminescence intensity, with the luminescence intensity upon no addition of inhibitor as 1.

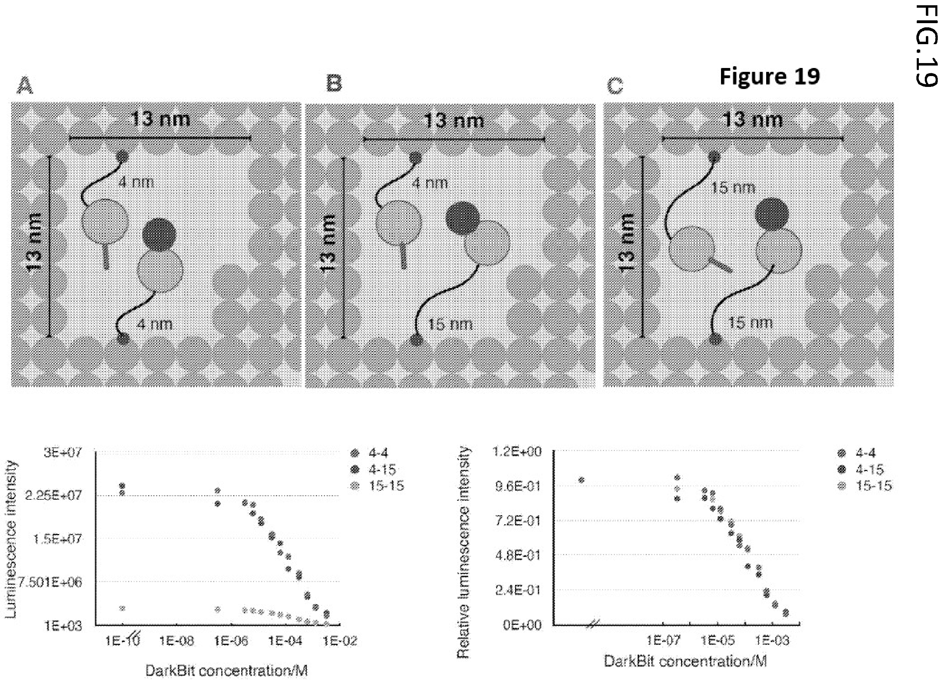

[Figure 19] Figure **19** shows results of conducting a similar experiment as Figure **17** by using a pair of unit molecules having different binding strengths.

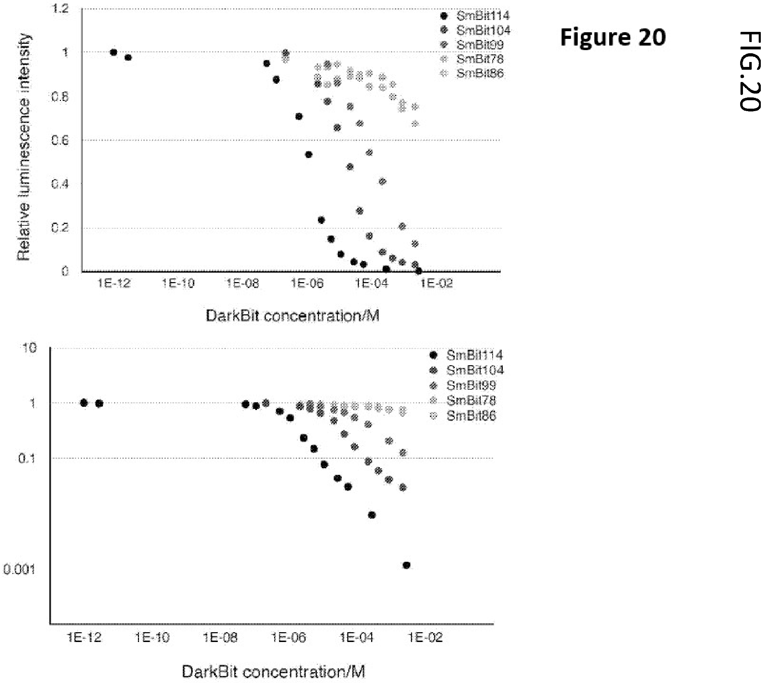

[Figure 20] Figure **20** shows a comparison of correlation between the concentration of added inhibitor and luminescence intensity using a pair of unit molecules having various binding strengths with fixed conditions of the substrate, attaching point, and linker. The plots appeared on the graph in order of, from the top (strong luminescence intensity), for SmBit86, SmBit78, SmBit99, SmBit104, and SmBit114.

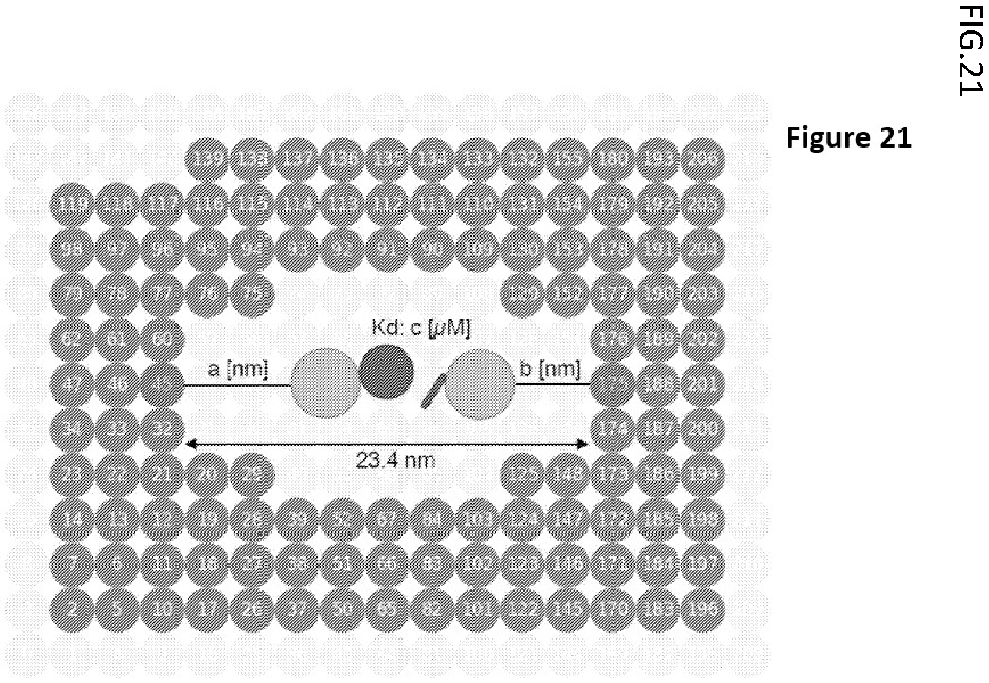

[Figure 21] Figure **21** shows the outline of a construct used for the measurement in Example 10.

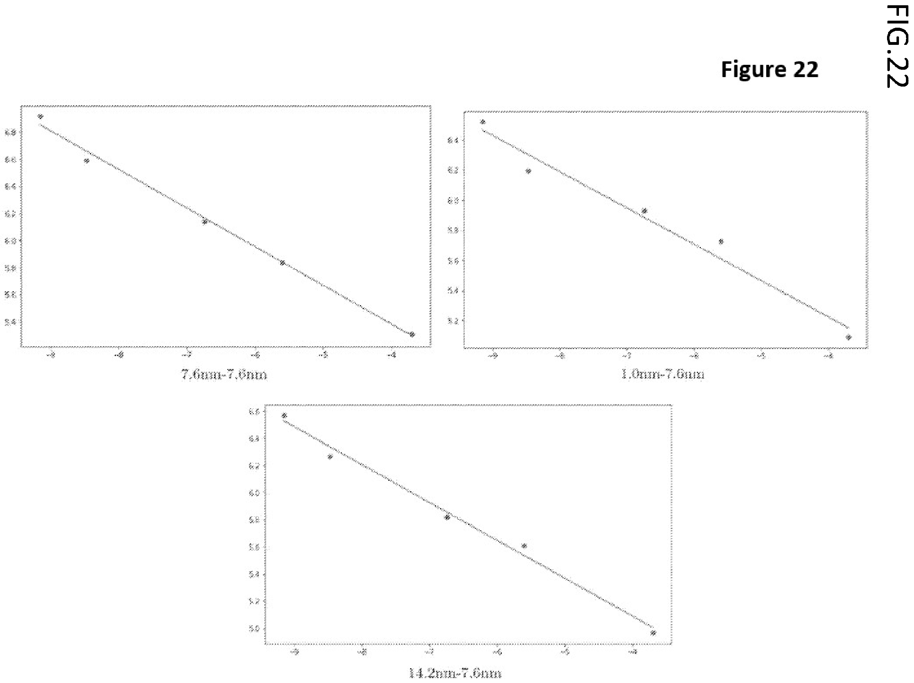

[Figure 22] Figure **22** is a diagram from calculating K_{d} for SmBit99 (provided as having K_{d} = 1.8 × 10⁻⁷ M) by utilizing the luminescence intensity measured through the use of the construct of the present disclosure. The horizontal axis is x = log₁₀K_{d} and the vertical axis is y = log₁₀(experimentally obtained luminescence value). The center plot, among the five plots, is plotted with the luminescence value for SmBit99 on the vertical axis and the provided binding constant on the horizontal axis. The results from using three structures with linkers having linker lengths of 7.6 nm + 7.6 nm, 1.0 nm + 7.6 nm, and 14.2 nm + 7.6 nm, respectively, are shown.

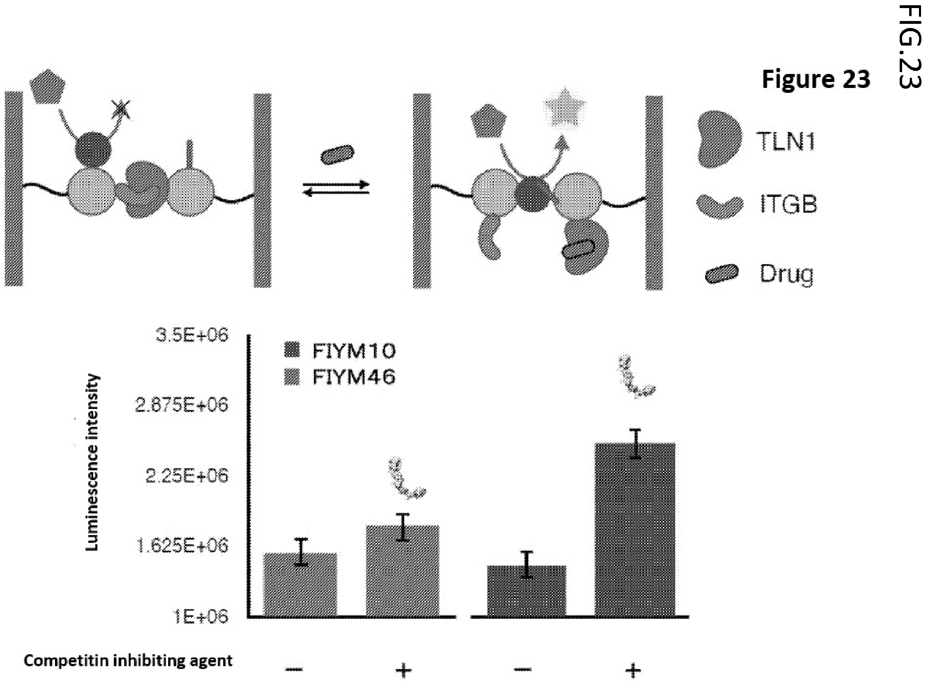

[Figure 23] The top half is a schematic diagram showing a system for detecting the effect of an added agent on complex formation based on the change in the luminescence intensity in a flip-flop configuration. The bottom half shows results indicating that an increase in luminescence intensity is observed when an inhibitor for complex formation is added in this system.

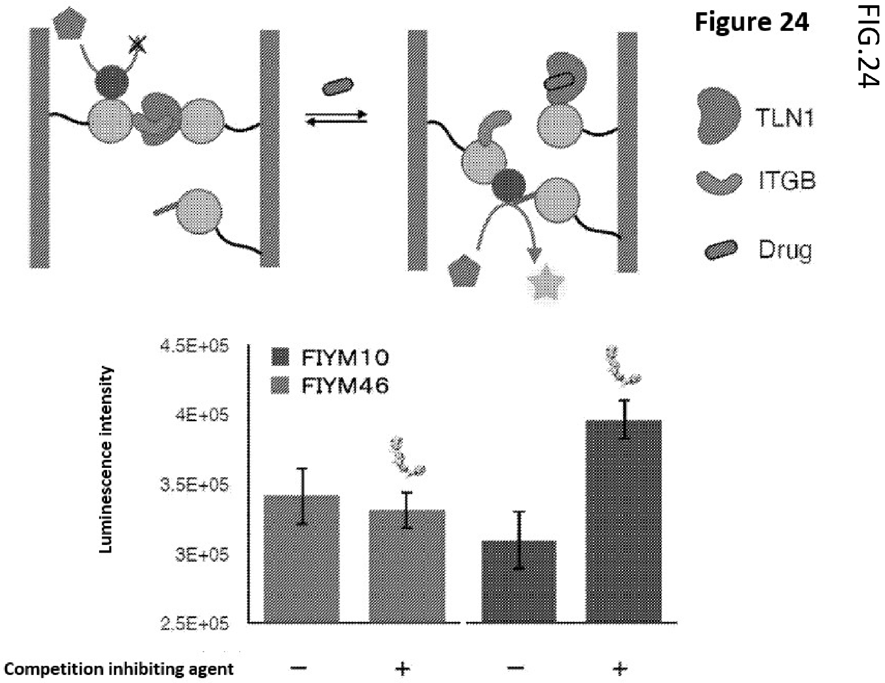

[Figure 24] The top half is a schematic diagram showing a system for detecting the effect of an added agent on complex formation based on the change in the luminescence intensity in a switch configuration. The bottom half shows results indicating that an increase in luminescence intensity is observed when an inhibitor for complex formation is added in this system.

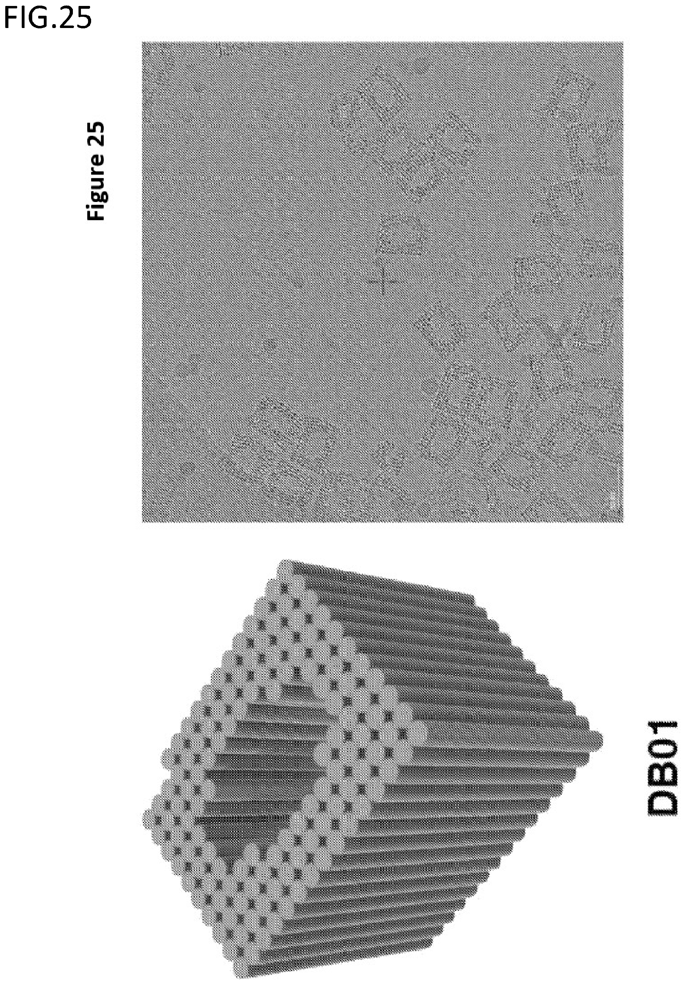

[Figure 25] The left side shows the structure of DNA origami (DB01) having a rectangular opening (used in Figure **21**). The right side is an example of an image of a substrate observed through a cryo-transmission electron microscope.

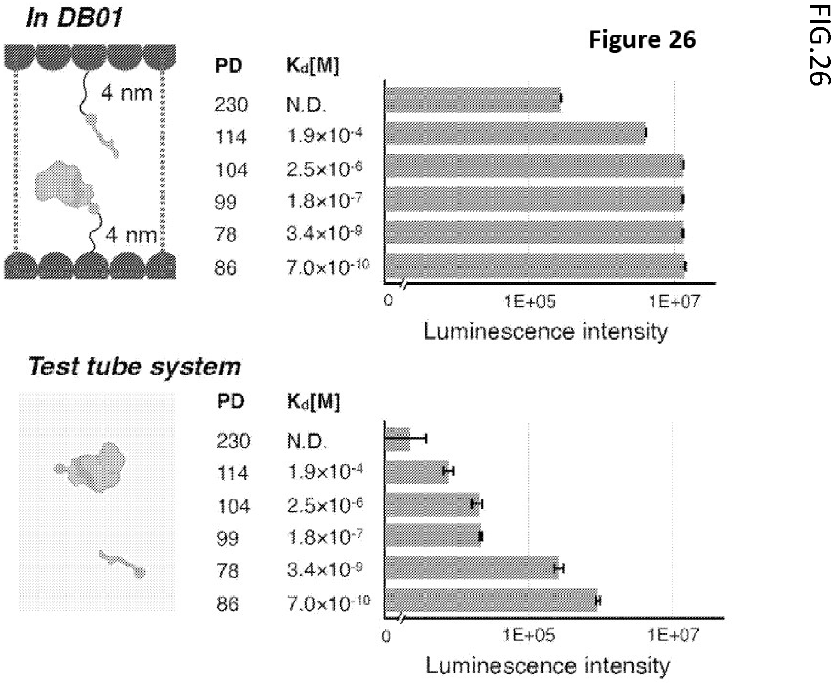

[Figure 26] Figure **26** shows results of measuring luminescence intensity in the cases wherein LgBit and SmBit with various K_{d} are immobilized via a linker to DB01 or in a free state. The top side shows results from measurement using DB01, and the bottom side shows results of measurement in a free state in a solution.

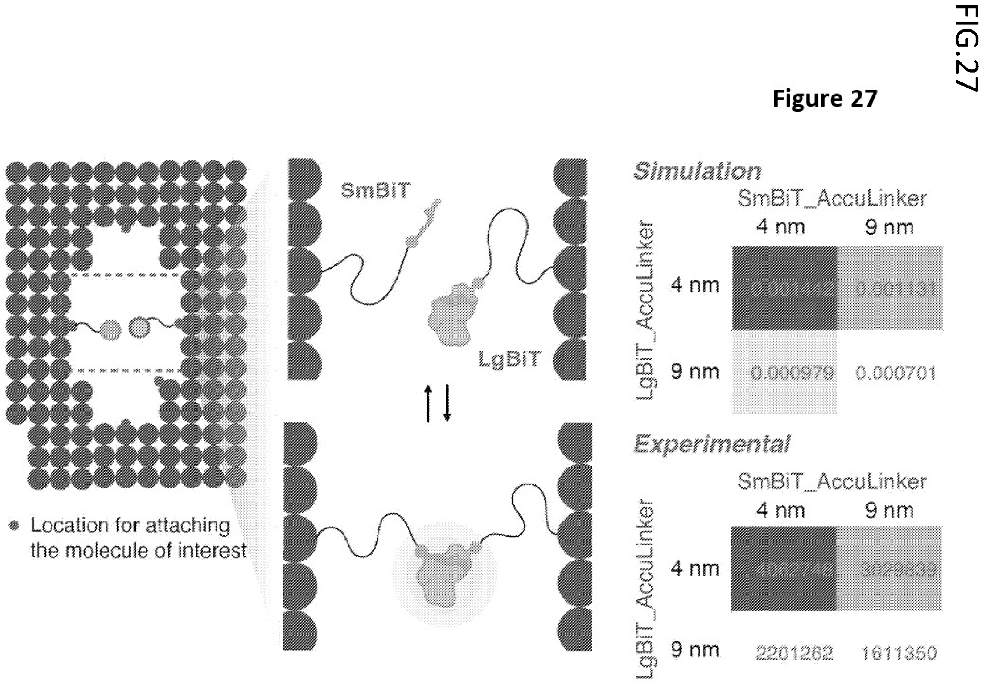

[Figure 27] Figure **27** shows the luminescence intensity measured by immobilizing LgBit and SmBit via a linker with a length of 4 nm or 9 nm at different positions in DB01. The left and center diagrams show schematic diagrams of this measurement system, the top right diagram shows the theoretical values of relative luminescence intensity, and the bottom right diagram shows the actual measurement values of luminescence intensity.

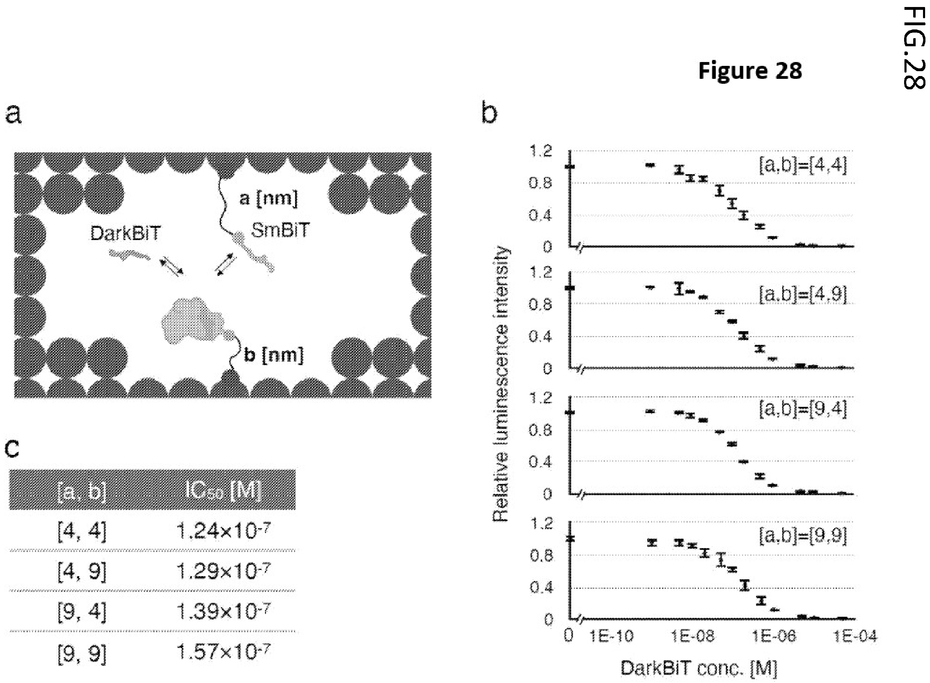

[Figure 28] Figure **28** shows results of studying the effect of an inhibitor by using complex formation in a substrate of the present disclosure. a shows a schematic diagram of the measurement system. b shows the change in the relative value of luminescence intensity (when the maximum intensity in the measurement is considered to be 1) associated with the change in the concentration of DarkBit at each combination of linker lengths. c shows the IC₅₀ values calculated based on the results of b.

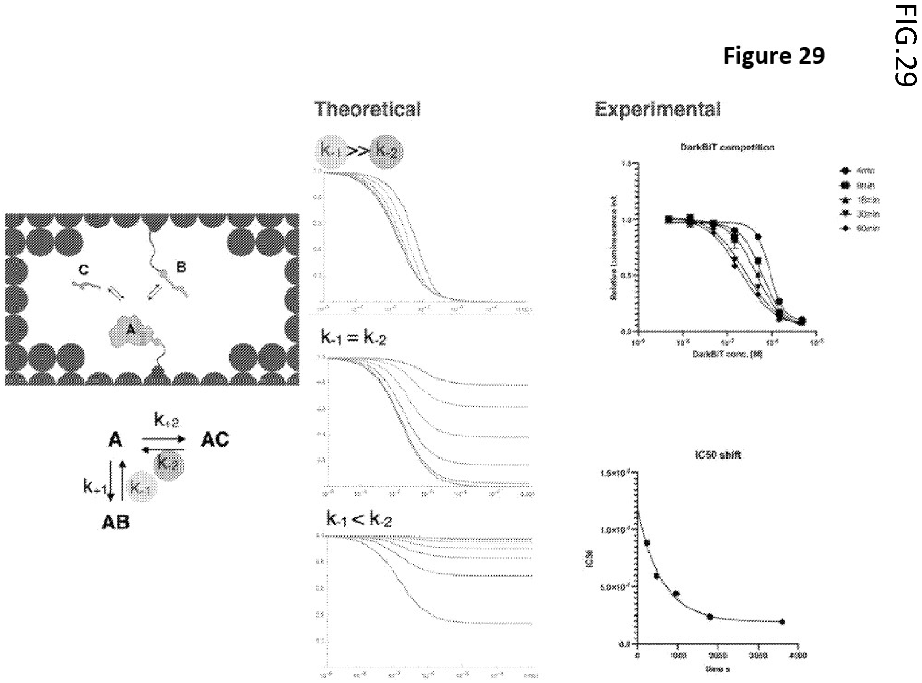

[Figure 29] Figure **29** shows the relationship between the theoretical prediction and actual measurement values of the inhibition mode in Figure **28**. The left side shows a summary of binding reactions of LgBit with SmBit or DarkBit. The difference in elapsed time from the addition of DarkBiT to measurement of inhibition rate (such as time described in the graph) was examined to study the time-dependent transition in inhibition curves upon addition of free DarkBiT from the outside to DB01 with LgBiT and SmBiT attached thereto. The center column shows the change in theoretical relative luminescence intensity when assuming each relationship of reaction rate constants for ligand (SmBit) and inhibitor (DarkBit). The right side shows the time-dependent transition in the inhibition curves upon actually adding free DarkBiT from the outside.

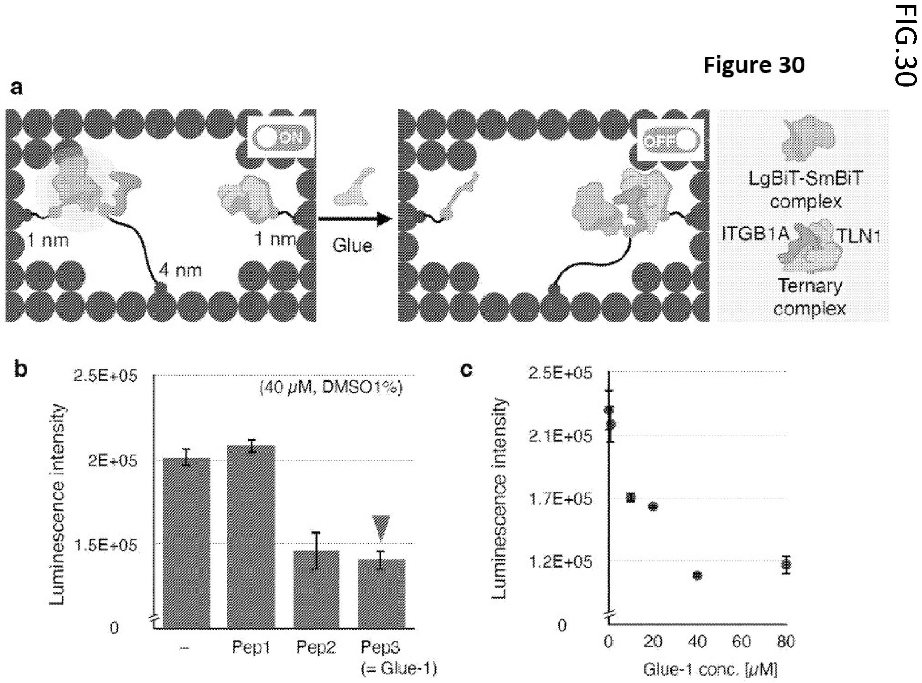

[Figure 30] a shows a schematic diagram representing a system for identifying a glue molecule by a decrease in luminescence intensity. b shows luminescence intensity upon addition of Pep1, Pep2, or Pep3 in this system. c shows the change in luminescence intensity upon a change in the concentration of Pep3 (Glue-1) added.

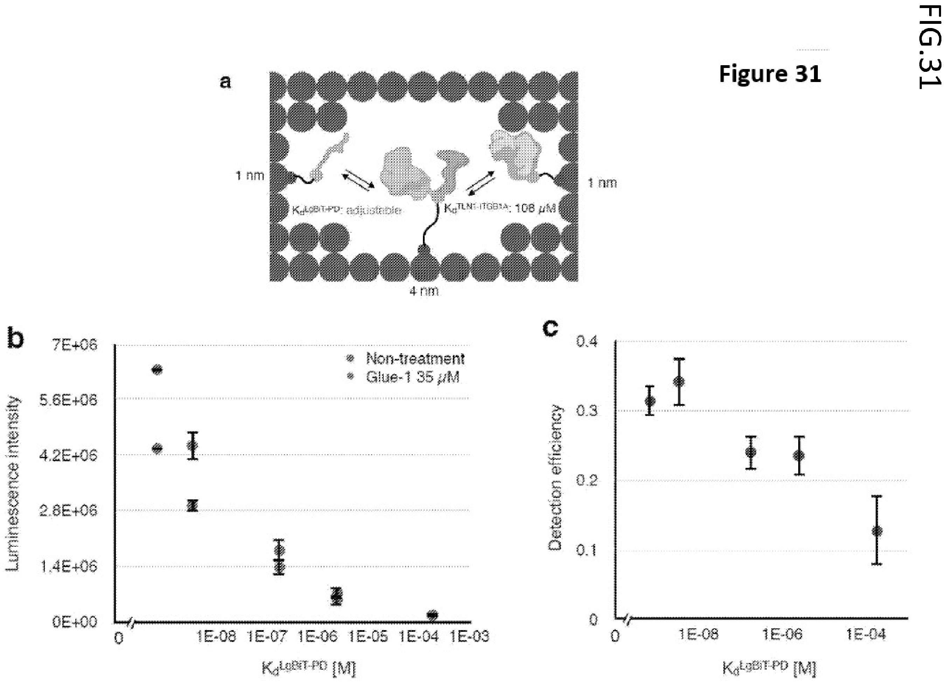

[Figure 31] Figure **31** shows the change in luminescence intensity upon a change in the binding strength between LgBit and SmBit by changing the type of SmBit. b shows the binding strength between LgBit and SmBit on the horizontal axis and the luminescence intensity on the vertical axis, for addition of a glue molecule and a control without the addition. c shows the result of quantifying the relationship of detection sensitivity (vertical axis) with binding strength between LgBit and SmBit based on the results of b.

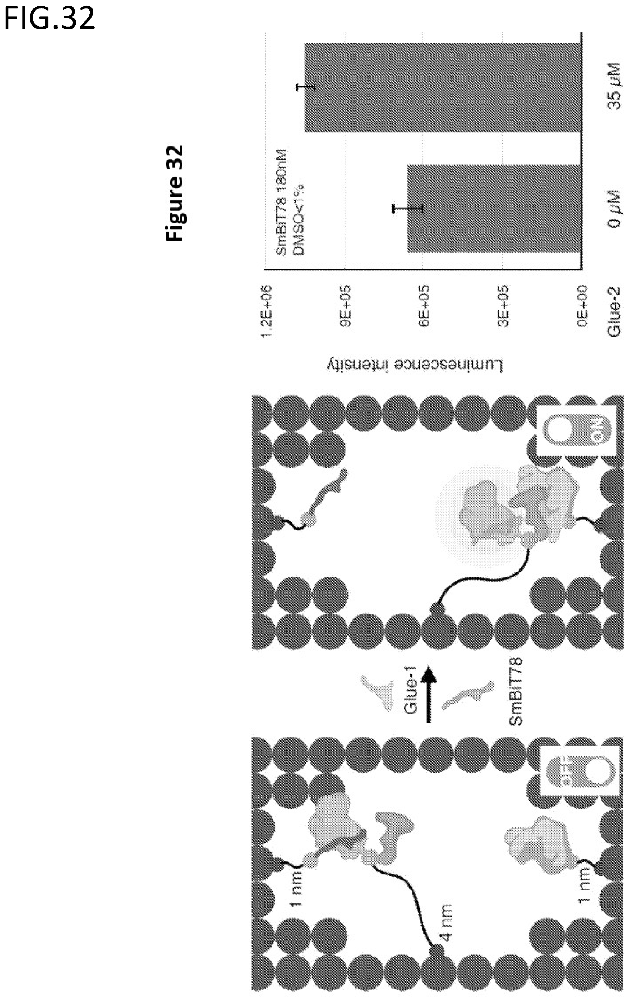

[Figure 32] The left side shows a schematic diagram representing a system for identifying a glue molecule by enhancement in luminescence intensity. The right side shows results of comparing luminescence intensity with addition of glue molecule and that without addition thereof.

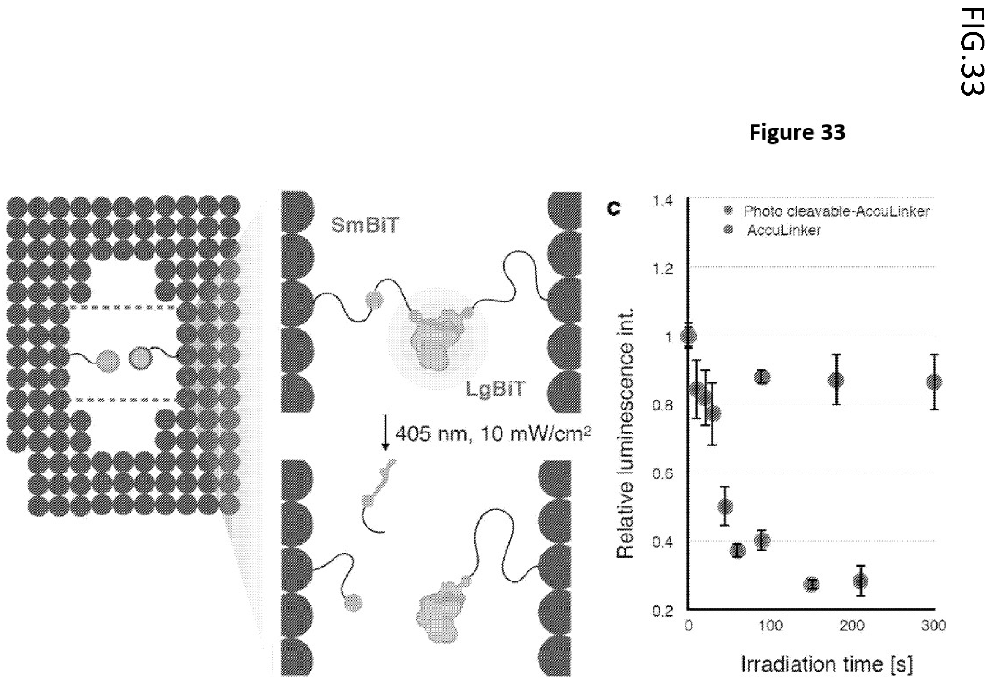

[Figure 33] a and b show a schematic diagram of a system using a photocleavable group-containing linker. c shows the light irradiation time on the horizontal axis and the luminescence intensity on the vertical axis for a system using a photocleavable group-containing linker and a system using a photocleavable group-free linker.

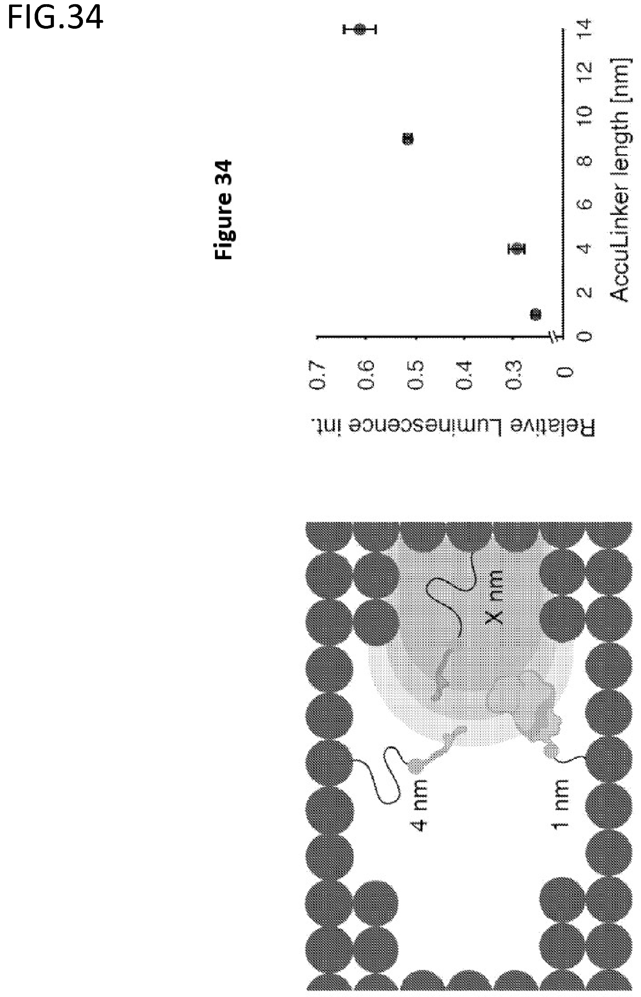

[Figure 34] a shows a schematic diagram of a three-way competition system. b shows the linker for DarkBit on the horizontal axis and the luminescence intensity on the vertical axis.

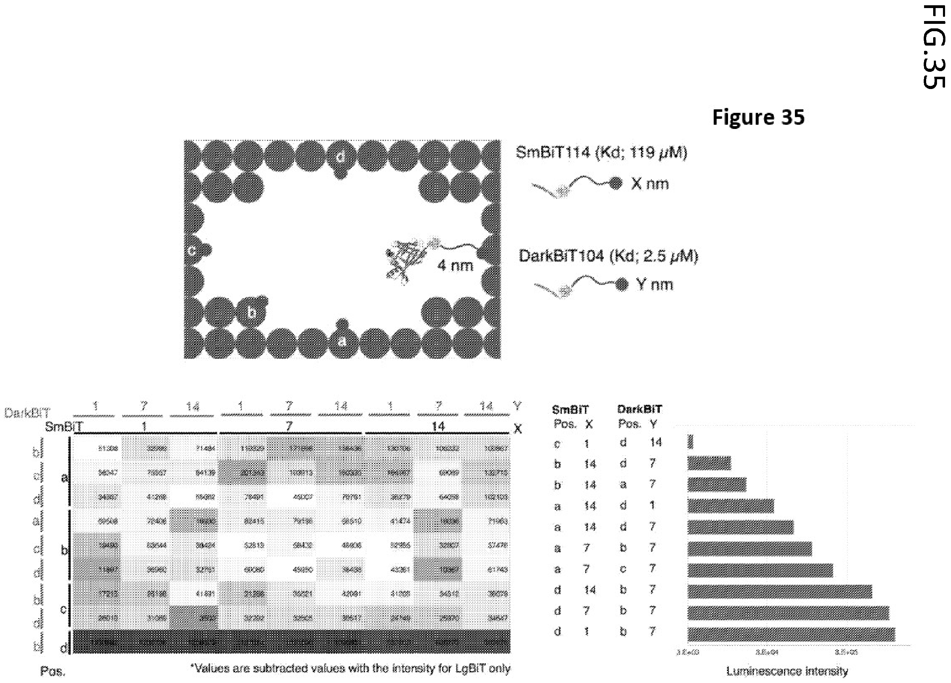

[Figure 35] The top side shows a structural schematic diagram of a construct using various attaching positions for a ligand (SmBit) and inhibitor (DarkBit) and linker lengths. The bottom side shows the luminescence intensity when using the respective attaching positions and linker lengths shown.

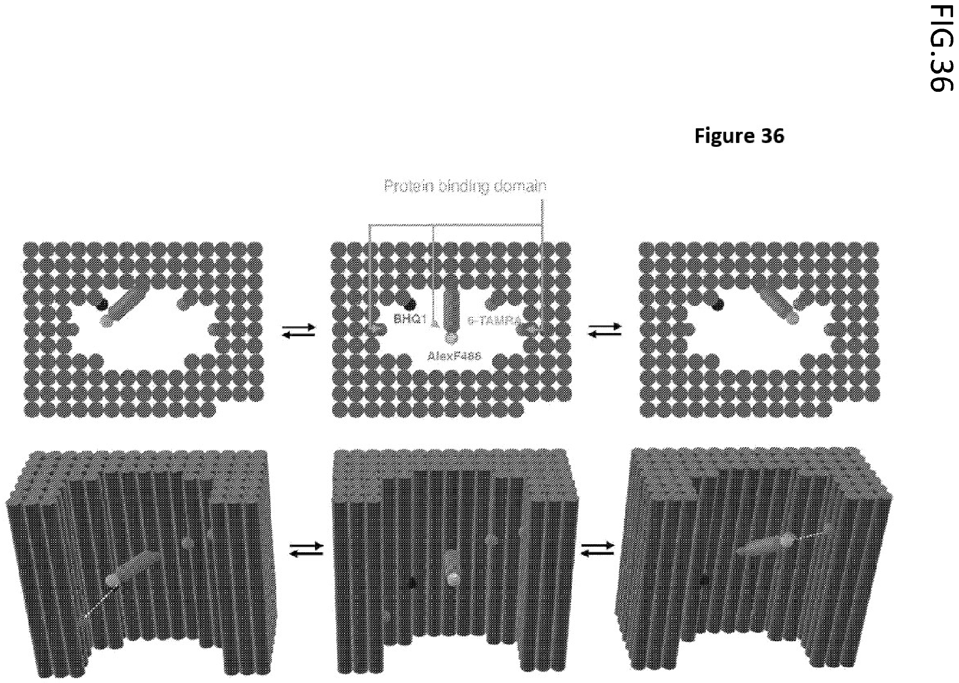

[Figure 36] Figure **36** shows a schematic diagram of a system for detecting an interprotein binding by fluorescence, which is constructed by using the substrate in Figure **25**. Either a FRET donor or acceptor is attached to a unit molecule attached to a substrate via a linker, and the other one of the donor or acceptor and a fluorescence quencher are attached to another location on the substrate. The other unit molecule forming a pair with the unit molecules is further attached to another location on the substrate. Structure a shows a schematic diagram of a three-way competition system. b shows a linker for DarkBit on the horizontal axis and the luminescence intensity on the vertical axis.

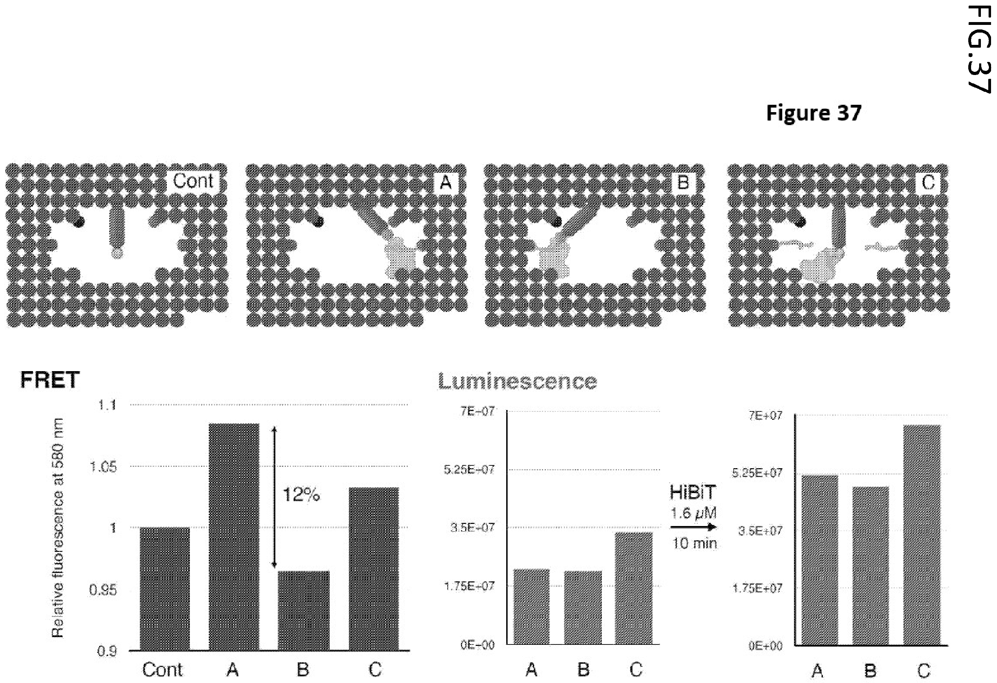

[Figure 37] The top side is a schematic diagram of each construct (Cont, A, B, and C). The bottom left side shows results of measuring fluorescence intensity for each construct. The center of the bottom side shows results of measuring luminescence intensity for each construct when using SmBit99. The bottom right side shows results of measuring luminescence intensity for each construct when HiBit was used instead of SmBit99.

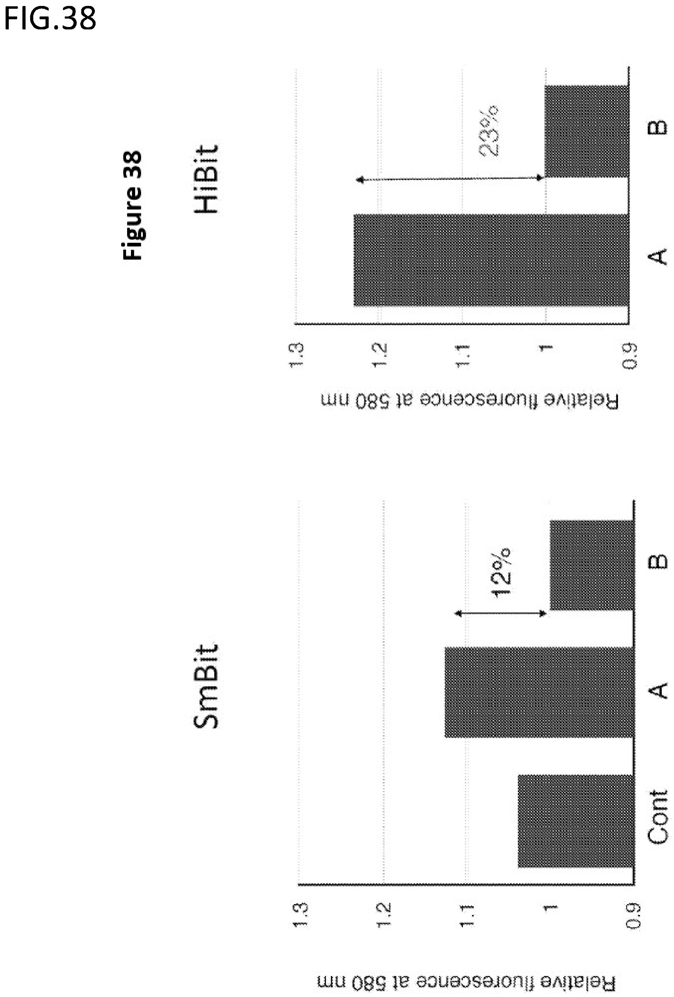

[Figure 38] Figure **38** shows a comparison of results of measuring fluorescence intensity by using constructs A and B in Figure **37** when using SmBit99 or HiBit as a ligand. The left side shows results upon using SmBit99 (corresponding to the bottom left side of Figure **37**) and the right side shows results upon using HiBit.

### [Description of Embodiments]

The present invention is described hereinafter with exemplary Examples while referring to the appended drawings as needed. Throughout the entire specification, a singular expression should be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

### (Definitions)

As used herein, "molecule" refers to not only a series of atom groups linked by a covalent bond but also a group of atoms integrated by any interaction such as a coordinate bond, ionic bond, or intermolecular force. Thus, the term "molecule" includes salts, coordination complexes, protein complexes, etc. The "structure" of a molecule includes the "three-dimensional structure" of the molecule as well as the "chemical structure" of the molecule.

As used herein, "unit molecule" refers to a target molecule for observation or use in the structure described herein (including components of an interaction complex) and is intended to be attached to a structure. Unless specifically noted otherwise, an interaction complex consists of unit molecules. A unit molecule may not be in a free state. For example, a unit molecule can be present in a state of being attached to a linker, a state of being incorporated into an interaction complex, etc. A unit molecule may be linked to another unit molecule (e.g., via a linker). In such a case, it is preferable to be linked in a form where there is a binding state (e.g., a state with no part bound in plane by plane manner) between unit molecules that is different from a binding state in an interaction complex of interest.

As used herein, "interaction complex" (or simply "complex") refers to an object formed by multiple components (unit molecules) binding to one another. In this regard, components may be the same or different from one another. Upon measurement for performing analysis described herein, a unit molecule is generally in an equilibrium state between a state of forming an interaction complex and a state of not forming an interaction complex.

As used herein, "kit" generally refers to a unit providing parts to be provided, generally in two or more separate segments. A kit advantageously comprises an instruction or user manual describing how the provided parts, etc. are to be used or processed.

As used herein, the term "about" refers to the indicated value plus or minus 10%, unless specifically noted otherwise. When used in the context of a temperature, "about" refers to the indicated temperature plus or minus 5°C. When used in the context of pH, "about" refers to the indicated pH plus or minus 0.5.

### (Preferred embodiments)

Preferred embodiments of the present disclosure are described hereinafter. Embodiments provided hereinafter provide better understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

While the present disclosure will be described herein mainly through descriptions regarding a method of making a structure, various embodiments such as the fabricated structures and use thereof, elements or a combination thereof for use in the method (such as composition, kit), systems for performing the method, and programs for performing the method are also intended as an invention. A description for a certain embodiment such as a method is similarly applicable to other embodiments. For example, it will be appreciated that the descriptions regarding a method are also intended as descriptions regarding elements used in the method, descriptions regarding the operation of a system for performing the method, descriptions regarding instructions encoded into the program for performing the method, etc.

### (Design and preparation of a structure for causing the formation of an interaction complex)

In one aspect, the present disclosure provides design and preparation of a structure described herein. Since unit molecules are targeted for observation or use, a structure can be designed to be compatible with unit molecules.

In one embodiment, a structure for causing the formation of a complex between unit molecules can be designed. The structure described herein can have a function of promoting the formation of a complex between unit molecules. In particular, complex formation can be promoted if a structure is configured so that the crossing index described herein (which can be determined based on a wall of a substrate, first and second linker attaching points, first and second linkers, and first and second unit molecules) is a value within a certain range. In one embodiment, a structure can be designed to be structured so that a crossing index for the first unit molecule and the second unit molecule is -5 *≤* D *≤* 15 such as -2 *≤* D *≤* 12, 0 *≤* D *≤* 10, 1 *≤* D *≤* 10, 1 *≤* D *≤* 9, 1 *≤* D *≤* 7, 1 *≤* D *≤* 5, 1 *≤* D *≤* 4, 2 *≤* D *≤* 10, 2 *≤* D *≤* 9, 2 *≤* D *≤* 7, 2 *≤* D *≤* 5, or 2 *≤* D *≤* 4. In general, for a lower value of D, the degree of freedom in movement of a first unit molecule and a second unit molecule is lower, and the number of cases of forming a complex would be lower. When observation of a complex is intended, such a complex usually has not been fully studied, and thus it is preferable to design a structure so that D has a value greater than the minimum value of D at which it is expected to be able to form a complex.

As described elsewhere herein, simulation under the following conditions may be used for the calculation of D:
- a system with a particle attached to the inside of a cylindrical space via a linker is considered;
- it is assumed that the particle is spherical and the point of interaction with another particle is located on the opposite side of the linker attaching point;
- the volume per one residue of amino acid is calculated as 0.141 nm³ (or volume of molecular weight (kDa) × 1 nm³) and the volume of the particle is calculated therefrom to calculate the particle diameter while assuming that the particle is spherical;
- it is assumed that the orientation of the particle is random, and the linker wrapping around the particle and a wall of a cylindrical substrate are taken into consideration to calculate the probability distribution of possible positions of an interaction point for a particle (particle **1**) connected to a linker;
- the same processing is also performed for the particle on the opposite side (particle **2**) attached to an attaching point at the same height to calculate the probability distribution; and
- crossing index D is calculated by using the probability distributions of particle **1** and particle **2**.

*It is assumed that: the distance between a linker attaching point on the substrate and a contact point between the linker and the particle excluding the wrapping around portion of the linker is distributed at a constant probability from length 0 to the length of the linker excluding the wrapping around portion; the particle is oriented in any direction at the same probability; and an interparticle interaction point is present on the opposite side of a linker attaching point on the particle.

In one embodiment, D may be calculated by setting a space that better reflects the structure of a wall of a substrate instead of a cylindrical space and/or setting the position of a linker attaching point to a position that better reflects a linker attaching point in a construct in the simulation described above. As is understood by those skilled in the art, parameters such as the shape of the particle, probability of the orientation of the particle, degree of freedom in movement of the linker, or position of interparticle interaction point may be adjusted to configure the simulation to be more in line with the actual circumstances.

When it is preferable that both a structure comprising a complex and a structure free of a complex (unit molecules do not form a complex) are present, such as in the case of determining a dissociation constant between unit molecules, a structure can be designed to be structured so that a crossing index for the first unit molecule and the second unit molecule is 1 *≤* D *≤* 9 or 2 *≤* D *≤* 9. When the binding constant (estimated K_{d}) between the first unit molecule and the second unit molecule can be roughly predicted, the structure can be designed based thereon.

In one embodiment, use of a structure where D is within the range of $- log \left(\frac{{p}_{max}}{1 - {p}_{max}}{K}_{d}\right) \leq D \leq - log \left(\frac{{p}_{min}}{1 - {p}_{min}}{K}_{d}\right)$ by setting the lower limit of binding ratio p that can be detected by a measurement instrument to pₘᵢₙ and the upper limit to p_{max,} for the estimated value of K_{d} of the binding constant between the first unit molecule and second unit molecule, allows utilization of linear approximation on a log-log graph. Thus, calculation of the actual K_{d} can be simple. For the calculation of K_{d}, it is not essential that linear approximation on a log-log graph is possible, so that a structure where D is within this range does not need to be necessarily used. Even if the estimated value of K_{d} is completely unknown or the actual K_{d} is significantly different from the estimated value, it is understood that K_{d} can be measured, if a signal is detected in multiple structures having different D and a structure providing a signal with a value away from the maximum and minimum values of signal detection limits for the measurement instrument is chosen, by using a structure having the same or similar (e.g., ± 1, ± 2) D as the D of said structure.

When a structure free of a complex is not required such as when using a structure as a reaction site for enzymes forming a complex, the structure may be designed to be structured so that a crossing index for the first unit molecule and the second unit molecule would be D such as -5 *≤* D *≤* 9. In the case of forming a constricted interaction complex such as with the orientation of the interaction complex controlled, it can be advantageous to design a structure to have a low crossing index such as D *≤* 0.

In one embodiment, a structure is designed based on at least one of sizes of the first unit molecule and the second unit molecule, and a binding dissociation constant between the first unit molecule and the second unit molecule. For example, D can be calculated by deeming the sizes of the first unit molecule and second unit molecule as spheres with a volume of molecular weight (kDa) × 1 nm³. The binding dissociation constant between the first unit molecule and the second unit molecule, which is referenced for designing a structure, may be a predicted value, and may have an error (e.g., about 10³, about 10², or about 10¹) from the actual binding dissociation constant. Those skilled in the art can predict the approximate value of the binding dissociation constant between unit molecules of interest.

In one embodiment, the step of designing the structure comprises determining lengths of the first linker and second linker. In one embodiment, the step of designing the structure comprises determining the distance between the first linker immobilization point and the second linker immobilization point. In one embodiment, the step of designing the structure comprises choosing the substrate from a plurality of options. Since it can be easier to change the length of a linker and/or the position of a linker immobilization point than changing the design of a substrate, a substrate to be used may be chosen from a plurality of predesigned substrates. In particular, it is preferable to predesign substrates with different sizes so that unit molecules or interaction complexes of different sizes can be enclosed. In one embodiment, the step of designing the structure comprises choosing the substrate from a plurality of options, determining the distance between the first linker immobilization point and the second linker immobilization point, and determining lengths of the first linker and the second linker.

### (Structure)

As described herein, a structure comprises a substrate comprising a linker attaching point described herein, optionally comprising a linker described herein, and optionally comprising a unit molecule described herein. While various uses of structures are described herein, both objects prior to comprising all of the elements for exerting a function of the structure in such uses and objects after comprising all of the elements are described as a structure herein. Representative examples of structures for causing the formation of an interaction complex comprise a substrate comprising a first linker immobilization point and a second linker immobilization point, wherein the relative positional relationship of the first immobilization point and the second immobilization point is fixed. A first linker and a second linker can be attached to or are attached to the first linker immobilization point and the second linker immobilization point, respectively. A first unit molecule and a second unit molecule can be attached to or are attached to the first linker and the second linker, respectively. The first unit molecule and the second unit molecule bind to each other to form an interaction complex at a predefined position.

A structure may be provided as a system. A system is a collection of components for achieving a specific purpose. Components may or may not be bound to each other. When, for example, a linker and a unit molecule are bound upon measurement, a linker and a unit molecule that are separate prior to being bound to each other can also be components of a system.

In one embodiment, the structure described herein can have a unit molecule (or interaction complex) positioned at a predefined position with respect to the structure. For example, a structure can be constructed so that at least one unit molecule which is a component of an interaction complex of interest can move within a specific region (defined by the length of a linker, etc.) when an interaction complex of interest is not formed, and the unit molecule is only present in a more limited region (predefined position) of the specific region when an interaction complex of interest is formed.

A predefined position can be mostly determined based on the structure of a unit molecule, attaching point on a substrate, and linker structure. While other factors such as the strength of interaction between unit molecules forming an interaction complex can also be a factor defining the predefined position, the contribution thereof is relatively small in many cases.

When unit molecule A is attached to attaching point A on a substrate via linker A and unit molecule B is attached to attaching point B on a substrate via linker B to form a structure for causing the formation of an interaction complex at a predefined position, the predefined position can be approximated as a region of overlap between a sphere with a radius of (UA + LA) and a sphere with a radius of (UB + LB) disposed away by a center distance of D:
wherein
D is the distance between attaching point A and attaching point B,
UA and UB are each distances from a point (or region) at which unit molecule A and unit molecule B are bound to an attaching point between a unit molecule and a linker, and
LA and LB are lengths of linker A and linker B, respectively.
The length of a linker may be approximated by multiplying the number of interatomic bonds on the shortest path between an attaching point between the linker and unit molecule and the attaching point between the linker and substrate by a specific coefficient or may be accurately computed while taking the structure of the linker into consideration.

In a representative embodiment, a line segment connecting attaching point A and attaching point B passes through an interaction complex at a position with the highest probability of an interaction complex being formed. In a representative embodiment, the midpoint between attaching point A and attaching point B is located inside an interaction complex that can be formed. In one embodiment, the total of the lengths of linker A and linker B can be about 200% or less, about 150% or less, about 100% or less, about 90% or less, about 80% or less, about 70% or less, about 60% or less, or about 50% or less, and about 5% or greater, about 10% or greater, about 15% or greater, about 20% or greater, about 30% or greater, about 40% or greater, or about 50% or greater, etc. of the distance between attaching point A and attaching point B.

A predefined position can have a volume, which is the entire volume of a region where an interaction complex can be present (if an interaction complex is present at only one point, this can be a volume of the interaction complex itself). The volume of a predefined position may be that by assuming an interaction complex as a sphere (e.g., sphere with a volume of molecular weight (kDa) × 1 nm³). In one embodiment, the ratio of the volume of a predefined position in the volume of a sphere with the distance between attaching points as the radius can be about 50% or less, about 40% or less, about 30% or less, etc.

Depending on the design of a structure, the orientation of an interaction complex at a predefined position can also be controlled. For example, setting the position of an attaching point in a substrate, adjusting the length of a linker, etc. limits the region where an interaction complex can be formed, which can simultaneously limit the orientation of the interaction complex that is formed. Since the orientation of an interaction complex may be associated with a function of the interaction complex or an interaction between the interaction complex and a drug, the structure of the present disclosure can be preferably used in the analysis of such an interaction complex. Controlling the orientation of an interaction complex can be beneficial in, for example, analysis of orientation with respect to the interface (such as liquid-liquid interface, liquid-oil layer interface) such as orientation of transmembrane protein with respect to the lipid bilayer, analysis of orientation with respect to a repulsive force field or electromagnetic field, etc.

### (Substrate)

The structure described herein can be used by comprising a substrate, to which a unit molecule is attached. By attaching a unit molecule to a substrate, a predefined position can be positioned as a relative position with respect to the substrate. Unless specifically noted otherwise, a description of a unit molecule attached to a substrate herein can mean that the unit molecule is attached to the substrate via a linker. If a unit molecule is attached to a substrate, a region where the unit molecule can move can be restricted in accordance with the structures of the unit molecule and linker.

Preferably, at least one of the unit molecules constituting an interaction complex is attached to a substrate. Even if only one of the unit molecules constituting an interaction complex is attached to a substrate, the interaction complex can be formed at a predefined position. For example, if one of the unit molecules is a macromolecule with a size that cannot pass through an opening of a substrate, a region where the unit molecule can move with respect to the substrate can be restricted and the position where an interaction complex is formed with the other unit molecule attached to the substrate can be limited to a specific position, even if the unit molecule is not attached to the substrate and is in a free state. In one embodiment, all of the unit molecules constituting an interaction complex are attached to a substrate. In one embodiment, two, three, four, five, or more of the unit molecules constituting an interaction complex are attached to a substrate. In one embodiment, an interaction complex is comprised of two, three, four, five, or more unit molecules.

A substrate that can be used in the structure described herein is not particularly limited, as long as it is capable of having a unit molecule attached thereto. For observation using a structure, a substrate is preferably a solid with a size that can be observed through a microscope. A predefined position can be positioned using a substrate as the baseline. Thus, when spatial information for the predefined position is used in analysis, it is preferable that the substrate is readily detected, and the positional relationship of the predefined position with respect to the substrate is fixed. Therefore, it is preferable that the substrate hardly deforms under conditions of forming an interaction complex.

Since the position of a attaching point for a unit molecule relative to a substrate can be one of the factors defining a predefined position, it is preferable that the attaching point for a unit molecule relative to a substrate can be controlled. As described herein, unless specifically noted otherwise, a attaching point for a unit molecule relative to a substrate can refer to a attaching point for a linker (attached to the unit molecule) relative to the substrate. A attaching point is not limited to that formed by a single bond by a covalent bond but also can be that formed by any form of bond such as a set of multiple covalent bonds, ionic bond, or a bond between complementary strands of a nucleic acid. An attaching point and a linker can have a combination of chemical structures that can specifically bind to each other. Such a combination of chemical structures is known to those skilled in the art. Examples thereof include complementary nucleic acids, a reaction pair of click chemistry, an antibody and a target protein thereof, etc.

Examples of substrates that can provide an attaching point at a controlled position include substrates comprised of a nucleic acid material such as DNA origami used in the Examples, etc. An attaching point can also be provided at a controlled position by micro-processing technologies such as nanolithography. Since those skilled in the art can readily design and construct a protein, a substrate comprised of a protein material is also possible. For example, various proteins having pores such as channels or proteasomes are known, which can be used as a tubular substrate. When measuring electromagnetic waves such as light, a substrate is preferably comprised of a material with little interference to electromagnetic waves and can be comprised of, for example, a nucleic acid material or a peptide material. The analysis of the present disclosure can also be performed based on results of measuring signals including signals such as light from chemical luminescence or fluorescence passing through a substance.

A substrate can have any shape such as a tubular structure, plate-like structure, or bowl-like structure (Figure **2**). A predefined position is generally positioned inside a substrate on a plane, when the substrate is viewed from one of the directions. When measuring an electromagnetic wave or particle beam passing through a predefined position, an opening of a substrate can be provided at a position where the substrate would not interfere with the electromagnetic wave or particle beam to be measured.

In many embodiments of the present disclosure, a plurality of unit molecules constituting an interaction complex is attached to a substrate. In such an embodiment, it is preferable that a contiguous portion in the substrate connecting attaching points do not deform in order to fix the relative positional relationship between attaching points corresponding to the plurality of unit molecules. For example, variation in the distance between different attaching points is preferably about 10% or less, about 5% or less, about 2% or less, about 1% or less, about 0.5% or less, about 0.1% or less, etc. under conditions upon measurement.

The method described herein can be performed by concurrently using a plurality of unit molecules (or interaction complexes). A plurality of substrates or a plurality of spots on a substrate (where unit molecules, etc. are placed) can be used therefor. To simplify the description, unless specifically noted otherwise, a description of a plurality of substrates herein refers to both the presence of a plurality of separate substrates and the presence of a plurality of spots on a substrate. Observation of a plurality of interaction complexes has advantages such as facilitating the analysis of interaction complexes placed in various directions and allowing improved precision of an observed image of an interaction complex by statistical processing, etc. When a plurality of interaction complexes is caused to be formed, substrates may have the same shape, whereby the effect of the substrates on the formation of an interaction complex can be uniform. However, all of the substrates observed at once do not need to have the same shape. The substrates can be formed to be readily distinguishable in terms of their shape, etc. Thus, embodiments of causing the formation of respectively different interaction complexes in two or more types of substrates and simultaneously observing the complexes to distinguish each interaction complex (or each substrate) at the analysis stage, etc. are possible. Information showing that substrates have the same shape can be used in the analysis of an interaction complex, which for example, allowing distinguishing which direction an interaction complex was observed from. To facilitate the direction of observation to be distinguished, substrates may be configured to have asymmetric shapes.

A substrate can also be designed to interact with another substrate, whereby a cluster of substrates can be formed. For example, a cluster can be formed wherein substrates are arranged in the same direction to one another, as shown in Figure **13**, etc.. Regularly arranging substrates can facilitate automated processing of the observed image thereof. A structure forming a cluster can also improve the handling when using a structure, e.g., when used as a site for an enzymatic reaction.

A substrate may comprise a plurality of attaching points for attaching a unit molecule. Each attaching point is at different positions on a substrate. The attaching points may have the same chemical structure or different chemical structures from one another. Examples of the same chemical structure include the same base sequence, portion involved in streptavidin binding to biotin, etc., where linkers or unit molecules having the same structure can be (specifically) attached thereto. If one substrate comprises a plurality of attaching points with the same chemical structure with one another, the same unit molecules can be brought into proximity from different directions to study the formation of an interaction complex. Attaching points having different chemical structures from each other are useful for attaching different unit molecules to respective attaching point. In one embodiment, the distance between two attaching points (especially attaching points for attaching different unit molecules) on a substrate can be selected from about 1 nm, about 2 nm, about 5 nm, about 10 nm, about 20 nm, about 50 nm, about 100 nm, about 150 nm, about 200 nm, about 250 nm, about 300 nm, about 350 nm, about 400 nm, about 450 nm, about 500 nm, about 750 nm, about 1 µm, about 2 µm, about 5 µm, about 10 µm, about 20 µm, about 50 µm, about 100 µm, a range between two of said values, or longer.

In one embodiment, a substrate can also be configured to comprise a cap (also see Figure **3**). A cap can be attached to an opening of a substrate. For example, a cap can be a polymer with a mesh structure or a linear structure. In one embodiment, a cap is structured so as not to completely block electromagnetic waves or particle beams. An attaching point of a unit molecule may be provided on the cap portion of a substrate. For example, a linear molecule comprising a complementary nucleic acid sequence on both ends attached to a tubular structure constructed with DNA origami can be a cap. When the linear molecule has a length that does not allow free movement (no slack) in a tubular structure and has a functional group, the functional group can be an attaching point on a substrate described herein. In one embodiment, an interaction complex formed inside a tubular substrate can be captured inside the substrate by providing a cap to the substrate. A substate may have any modification. For example, a system wherein a moiety of promoting a reaction (e.g., donor, acceptor, and quencher for a FRET reaction, coenzyme, etc.) is attached to a substrate, etc. is envisioned.

### (Linker)

A linker is a portion connecting a unit molecule and a substrate. An attaching point between the linker and the unit molecule and a attaching point between the linker and the substrate can be formed, which are the portions that are not intended to be directly involved in the function of a unit molecule (e.g., formation of a desired interactive molecule). When the presence/absence of the formation of an interaction complex is determined by the position where a unit molecule is present, or when it is preferable that a state where an interaction complex is formed and a state where an interaction complex is not formed are both present, it is preferable that a linker is designed to provide a degree of freedom in movement to a unit molecule to secure a position where the unit molecule can be present other than a predefined position. If, for example, a linear carbon chain having consecutive double bonds is used as a linker, the linker cannot have a folded structure, so that the degree of freedom in movement of a unit molecule is low. Thus, it is preferable that a linker comprises a single bond. While many rings do not provide a degree of freedom in movement to a unit molecule, a spiro ring allows a rotational movement within a molecule and thus can be present as a portion providing a degree of freedom in movement to a unit molecule. Since it is preferable that a linker is not cleaved under conditions of causing the formation of an interaction complex, it is preferable that an unstable group such as an -O-O-bond is not contained in the main chain. The linker structure is not particularly limited, as long as a unit molecule can move freely. For example, a bond other than a covalent bond such as an ionic bond, a bond due to a specific interaction (such as bond forming a attaching point described herein), a coordinate bond, etc. may be contained within a linker. A linker may comprise any number of single bonds, double bonds, and triple bonds. A linker may comprise any element such as C, N, O, S, or Si, or an isotope thereof.

Since the length of a linker can affect a predefined position, the length can be adjusted in accordance with the design of an attaching point on a substrate and/or design of a unit molecule. The length of a linker herein can typically refer to the distance between both ends of a linker moiety when the linker moiety is placed in an extended manner (placement achieved as a result of natural movement without adding any tensile force). The length of a linker can be expressed as the part from an attaching point between the linker and a unit molecule to a attaching point between the linker and a substrate. The length of a linker can be approximated from the number of bonds on the shortest path between both attaching points (group of atoms connecting both attaching points with each other with the smallest number of atoms). The portion comprised of atoms on the shortest path may be referred to as a main chain. The length of a linker can certainly be calculated more precisely by taking the linker structure into consideration. In one embodiment, there are 1, about 5, about 10, about 15, about 20, about 25, about 30, about 35, about 40, about 45, about 50 chemical bonds (covalent bonds, ionic bonds, etc.), or at a number within the range of two numbers thereamong, or greater on the shortest path between a attaching point between a linker and a unit molecule and a attaching point between the linker and a substrate. In this regard, each of a single bond, double bond, and triple bond is counted as one bond. In one embodiment, at least one linker can have a length of about 0.15 nm, about 0.2 nm, about 0.5 nm, about 1 nm, about 2 nm, about 5 nm, about 10 nm, about 15 nm, about 20 nm, about 50 nm, about 100 nm, about 150 nm, about 200 nm, about 250 nm, about 300 nm, about 350 nm, about 400 nm, about 450 nm, about 500 nm, about 1 µm, about 2 µm, about 5 µm, about 10 µm, about 20 µm, about 50 µm, about 100 µm, or a length within the range of two numbers thereamong, or greater. In one embodiment, the distance between a attaching point on a substrate and a binding surface between unit molecules forming an interaction complex (or binding point that is the center of gravity thereof) can be about 1 nm, about 2 nm, about 5 nm, about 10 nm, about 15 nm, about 20 nm, about 50 nm, about 100 nm, about 150 nm, about 200 nm, about 250 nm, about 300 nm, about 350 nm, about 400 nm, about 450 nm, about 500 nm, about 1 µm, about 2 µm, about 5 µm, about 10 µm, about 20 µm, about 50 µm, about 100 µm, a value within the range of two values thereamong, or greater. The technical scope of the present disclosure encompasses embodiments using a structure that uses two very short linkers, this is, for example, a system wherein there are two unit molecules attached to a substrate, and a complex is formed at a predefined position when an additional certain molecule bridging these two unit molecules is present.

For an interaction complex with three or more components, a plurality of unit molecules constituting the interaction complex may be attached by a single linker. A linker can comprise a functional moiety. For example, many structures corresponding to a desired functionality are known as a functional moiety, such as a moiety that is cleavable under a specific condition (such as moiety that is cleavable by light irradiation, reductively cleavable -S-S- bond), fluorescent moiety, luminescent moiety, or moiety capable of specifically binding to a specific target. A functional moiety can be appropriately chosen by those skilled in the art. For example, a portion constituting a second interaction complex that is different from the desired interaction complex (it can be considered that there is a second set of unit molecules) may be attached to a linker, which for example, allowing detection of a signal (such as light) indicating that a second interaction complex is formed based on that the second interaction complex has enzymatic activity, or to reduce the formation and activity of the first interaction complex by competition with the formation of the second interaction complex. For example, by designing a linker to comprise a fluorescent moiety near a attaching point to a unit molecule, generation of fluorescence in the vicinity of a predefined position can be deemed to mean that an interaction complex is formed. In such a case, those skilled in the art can define the vicinity of a predefined position from the design of a linker and the predefined position. As another example of a functional moiety, if for example a third component such as a coenzyme is required for the formation of an interaction complex of interest, said component may be included in a linker to assist the formation of an interaction complex. In such a case, the third component can be also considered as a unit molecule.

Even if a suitable linker is unknown, an interaction complex can be formed. For example, by using a substrate having a plurality of linker immobilization points on a single substrate or a plurality of substrates with different positions of linker immobilization points from one another, and by each immobilization point having the same chemical structure, unit molecules can form an interaction complex via a linker attached to a probabilistically selected suitable immobilization point.

A linker is essentially a portion that provides a degree of freedom in movement of a unit molecule. Thus, at the minimum, a linker can be a single bond (such as carbon-carbon bond). A linker can also be expressed as a portion having a primary or secondary structure that is generally used in a biomolecule such as a protein. In such a case, a unit molecule (in a state of forming an interaction complex) and a substrate can be expressed as a tertiary or quaternary structure. When three different points in the structure of each of a unit molecule and substrate are designated and the three-dimensional coordinates of atoms (other than hydrogen atoms) are identified as the relative positions with respect to these three points, atoms may be determined to belong to the unit molecule or substrate if the coordinates of the atoms move within a certain range of tolerance (e.g., within 1Å) due to thermal fluctuation, etc. and to belong to a linker if the coordinates move beyond the range. In a state where an interaction complex is formed, a unit molecule does not need to be able to move freely. Thus, a portion providing a degree of freedom in movement of the unit molecule in absence of a corresponding unit molecule which is a binding partner may be a linker. Thus, in one embodiment, a linker can comprise a portion comprised of the same material as a unit molecule and/or substrate. If, for example, a unit molecule or substrate is comprised of a protein or a nucleic acid, it is known to those skilled in the art that these substances can form a portion that freely moves (e.g., linking moiety between domains or a single stranded nucleic acid which is not forming a specific three-dimensional structure), so that a moiety that functions as a linker while being continuous as the same protein or nucleic acid material as the unit molecule or substrate is envisioned to be present. In some cases, the entire portion protruding out from a substrate other than a binding surface (binding point) with another unit molecule upon forming an interaction complex may be a linker. In one embodiment, a linker is a portion between an attaching point between the linker and a unit molecule and a attaching point between the linker and a substrate. In such a case, each of the unit molecule, linker, and substrate can be distinguished as separate portions in a state before being attached. For example, if a triazole ring is present in an integrated structure of a unit molecule, linker, and substrate, those skilled in the art can readily understand two molecular structures comprising alkyne and azide respectively before the formation of the triazole ring as separate portions. In addition, a linker may be a portion between a single bond that is the closest from a portion where a unit molecule binds to one of other components of an interaction complex (attaching point between the unit molecule and the linker) and a single bond that is in a portion that has a chemical structure different from the material constituting a substrate and closest to the substrate (attaching point between the substrate and the linker). For example, when a biotin is attached to a substrate comprised of a nucleic acid material via a PEG linker, a linker can be the portion surrounded by a square frame in the following diagram.

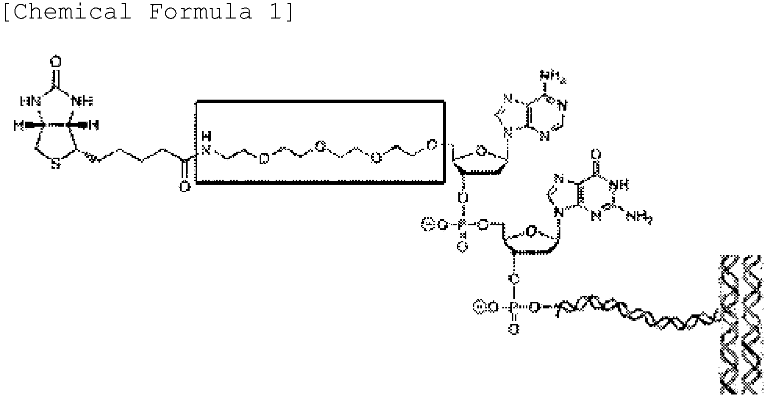

In this regard, optionally, a portion where a unit molecule binds to one of other components of an interaction complex can be the smallest portion that enables the formation of the interaction complex, such as a portion where the probability of forming an interaction complex does not decrease 99% or more when the remaining portion is removed from the unit molecule. When a chemical group known as a linker such as PEG is used, when a unit molecule is a protein and a linker is a long straight chain, etc., the portion corresponding to the linker can be readily identified by those skilled in the art. Thus, a strict definition of a linker portion such as those described above is not necessarily required. The portion from free DNA double strands branched from a substrate to a carbon atom adjacent to S of biotin can also be considered as a linker in the above diagram. It is intended that the requirements (such as length) for a linker described herein are meant to fall under the requirements for a linker portion corresponding to one of the explanations.

### (Unit molecule)

A unit molecule can be any substance of interest for those skilled in the art performing the method described herein. The structure described herein is useful in the observation or use of a unit molecule forming an interaction complex, while the structure can also be useful in the use of a unit molecule that does not form an interaction complex. When forming an interaction complex, it is not essential that entire unit molecule interacts with other unit molecule. In many cases, only a portion of the unit molecules are involved in the formation of an interaction complex. A unit molecule can be, in many cases, a protein, a lipid, a nucleic acid or other biomolecules, a small molecular weight compound (such as drug), etc., but can be any other substance that is desirable for analysis or use. Generally, at least one unit molecule constituting an interaction complex of interest is a protein. The analysis described herein can be promoted by modifying a substance of interest or using a complexed substance as a unit substance as needed (e.g., by introducing a portion that facilitates attachment to a linker). Such a modification can be appropriately applied by those skilled in the art. The analysis described herein can be performed for any study on interaction such as a detailed study on the form of interaction of an interacting pair of interest or identification of a substance that interacts with a substance of interest. In one embodiment, a unit molecule or interaction complex has a molecular weight of about 1 kDa, about 2 kDa, about 5 kDa, about 10 kDa, about 20 kDa, about 50 kDa, about 100 kDa, about 200 kDa, about 500 kDa, about 1 MDa, about 2 MDa, about 5 MDa, about 10 MDa, about 20 MDa, about 50 MDa, about 100 MDa, about 200 MDa, about 500 MDa, about 1000 MDa, a molecular weight within the range of two of said molecular weights, or greater.

A substrate, a linker, and/or a unit molecule may be integrally prepared with one another or may be prepared separately and then assembled. A structure may be provided as a set of components for assembling the structure (components may not be attached to one another). The components may be sub-members of the substrate, linker, and/or unit molecule (members that form the substrate, linker, and/or unit molecule by combining a plurality of the members). As used herein, unless specifically noted otherwise, a description of a substrate, a linker, or a unit molecule is also a reference to a set of sub-members that are combined to form the substrate, linker, or unit molecule.

At least one of a substrate, a linker, and a unit molecule can be provided separately from other components to provide a versatile platform that enables the formation of an interaction complex for various unit molecules. For example, a substrate comprising a linker attaching point can be prepared separately from a linker to allow various structures comprising various linkers to be readily prepared and a suitable linker to be experimentally chosen for the formation of an interaction complex of interest. When a linker is provided separately from a substrate and/or a unit molecule, the linker can comprise a capture moiety for specifically attaching to the substrate and/or unit molecule. This attachment is not limited to a single covalent bond and can be a bond of any form, such as a set of a plurality of covalent bonds, ionic bond, or a bond between complementary strands of a nucleic acid. Those skilled in the art can choose any suitable means for forming a specific bond, such as click chemistry or antibody. Since a carboxyl group activated by adding N-succinimide exhibits reactivity specific to an amino group, this is also included in examples of specific bonds.

Examples of preparing a substrate, linker, and unit molecule integrally with one another include a protein prepared by fusing a foundation part (substrate), movable part (linker), and an interaction pair (unit molecules), which are obtained from known proteins, by a biotechnological method.

A predefined position for an interaction complex consisting of three or more unit molecules can be a region of overlap between predefined positions, each predicted and determined from the relationship between two unit molecules. A predefined position may also be experimentally determined from an observed image obtained by performing the analysis described herein.

In order to reduce the probability that an object is present at a predefined position when there is no formation of interaction complex, a substrate may have a portion that is (weakly) bound by a unit molecule at a position different from a predefined position.

The construct of the present disclosure can be constructed to facilitate analysis of interaction between unit molecules. In one embodiment, complexity in analysis based on variation of interactions can be reduced by using a construct having a single structure (for substrate, linker, unit molecule, attaching relationship therebetween, etc.). In one embodiment, an analysis model based on compartmentalization of unit molecules can be applied by using a construct designed so that a unit molecule attached to a certain construct does not interact with a unit molecule attached to another construct. For example, those skilled in the art can readily design the configuration of a construct so that at least one unit molecule is not placed on the outside of a substrate by adjusting the substrate structure, the length of a linker, and/or attaching position between a linker and a substrate, etc. Even for a design of a construct where a unit molecule can be placed on the outside of a substrate, those skilled in the art can readily make a configuration wherein unit molecules attached to different constructs do not interact with each other, e.g., by linking substrates in regularized manner.

### (Analysis of interaction complex)

In one aspect, the present disclosure provides analysis of an interaction complex (also referred to as the "analysis described herein"). By constructing a system wherein at least one unit molecule, which is a component of an interaction complex of interest, can move within a certain region (defined by the length of a linker, etc.) if the interaction complex of interest is not formed, and the unit molecule is only in a more limited region (predefined position) in the certain region if the interaction complex of interest is formed, the presence of the unit molecule at the predefined position can be deemed to mean the formation of the interaction complex of interest. For this reason, an interaction complex of interest can be analyzed by measuring a signal obtained from a predefined position.

A state where an interaction complex of interest is formed is not necessarily the target of analysis. A state where an interaction complex of interest is not formed can also be a target of analysis. For example, a high ratio of the time or frequency of a state where an interaction complex of interest is not formed can indicate that the interaction complex of interest is not readily formed or the interaction between unit molecules is weak. When, for example, an interaction complex between a receptor and a ligand thereof is analyzed, the ability of a drug to promote or suppress the formation of an interaction complex can be studied by testing the formation of an interaction complex in the presence of the drug.

In one embodiment, the present disclosure provides a method of analyzing an interaction complex, wherein a system for causing the formation of an interaction complex at a predefined position is used. A system is a collection of components for achieving a specific purpose, and the components may or may not be attached to one another. If, for example, a linker and a unit molecule are attached upon measurement, the linker and the unit molecule that are separated before being attached to each other can also be components of the system. A representative example of a system for causing the formation of an interaction complex at a predefined position comprises a substrate comprising a first linker immobilization point and a second linker immobilization point, wherein a relative positional relationship between the first immobilization point and the second immobilization point is fixed. A first linker and a second linker can be attached to or are attached to the first linker immobilization point and the second linker immobilization point, respectively. A first unit molecule and a second unit molecule can be attached to or are attached to the first linker and the second linker, respectively. The first unit molecule and the second unit molecule bind to each other to form an interaction complex at a predefined position. A representative embodiment measures a particle beam or an electromagnetic wave from a predefined position of a structure optionally with irradiation with a particle beam or an electromagnetic wave. In many embodiments, a particle beam or an electromagnetic wave from a region other than the predefined position is also simultaneously measured. In such a case, information from measurement of a particle beam or an electromagnetic wave from a predefined position may be extracted by analysis. An interaction complex can be analyzed by analyzing data on a particle beam or an electromagnetic wave from a predefined position.

### (Use/Method)

The structure described herein can be used in various uses, such as those described hereinafter. Those skilled in the art will appreciate that a method of using a structure is equivalent to being described herein based on the descriptions of use of the structure. The structure described herein can also have an effect of stabilizing a unit molecule captured inside. This effect can be beneficial in various applications. The structure described herein can prevent the formation of an aggregate (unit molecules that are different from the interaction complex of interest bound to one another in a disorderly manner, etc.) by segregating the captured unit molecule. For this reason, the structure described herein can enhance the concentration of unit molecules (ratio of volumes of unit molecules occupying the space) to a level that is generally unachievable due to the formation of an aggregate in a solution, etc. while maintaining the function of the unit molecules. Since a unit molecule immobilized to a substrate is segregated from other unit molecules, it is not necessary to standardize and handle the molecular behavior in the entire system, such as the conventional formation of a complex between molecules in a free state in a solution, which can enable measurement reflecting individual complex formation events in more detail. In one embodiment, an interaction complex may account for at least about 5%, at least about 10%, at least about 15%, at least about 20%, at least about 30%, at least about 40%, or at least about 50% of the volume (may be calculated as molecular weight of interaction complex (kDa) × 1 nm³) of the internal volume of the structure (e.g., volume of the largest sphere that comprises the center of gravity of a substrate inside but does not comprise any atom constituting the substrate inside) in the structure described herein.

In one embodiment, the structure described herein is used for analyzing an interaction complex. In one embodiment, an object at a predefined position is detected for analysis. An object at a predefined position can be detected by detecting an electromagnetic wave or particle beam passing through the predefined position or an electromagnetic wave or particle beam generated from the predefined position. In one embodiment, an electromagnetic wave or particle beam is irradiated to measure the diffracted, reflected, or transmitted electromagnetic wave or particle beam. Since properties such as the wavelength of irradiated electromagnetic wave or particle beam can change due to interference by an interaction complex, the irradiated electromagnetic wave or particle beam and the measured electromagnetic wave or particle beam may not be the same. Those skilled in the art can suitably choose the electromagnetic wave or particle beam that is measured. Even if an electromagnetic wave or particle beam is not irradiated, chemical luminescence, etc. generated from a predefined position can be detected. In such a case, a signal can be quantitatively measured. A system where a signal is generated when a desired interaction complex is present at a predefined position and a signal is not generated when present at a predefined position is also possible. Detection means is not particularly limited, as long as the predefined position can be distinguished, such as detection of a reaction product or detection through sound waves.

In one embodiment, the presence/absence of an object at a predefined position is detected for analysis. More detailed analysis, in addition to the presence/absence of an objective at a predefined position, is also possible. For example, the shape of an interaction complex can be observed and detailed form of interaction can be analyzed in measurement using a cryogenic electron microscope. More information collected can allow for more detailed analysis of an interaction complex. However, highly precise analysis of an interaction complex is not necessarily required when simply measuring the binding strength, etc. Since improvement in the precision of analysis increases the amount of information required and the time required for obtaining the information of interest, those skilled in the art can perform the analysis described herein at a precision in accordance with the purpose to improve efficiency.

Most information is obtained from the observed surface in many measuring means. Since the analysis described herein can also handle large molecules such as proteins, measurement of a predefined position from a variety of directions can be advantageous for the analysis of the entire interaction complex. If a plurality of substrates are present in a sample, a predefined position can be measured from a variety of directions even in measurement from one direction of a detector, but the predefined position may be measured from a plurality of directions with a detector. An electromagnetic wave or particle beam may be irradiated from a plurality of directions. Information from measurement of interaction complexes can be increased by increasing the number of interaction complexes measured and/or by measuring interaction complexes over time. The number of interaction complexes measured can be increased by using a plurality of substrates to cause the formation of a plurality of structures.

Data obtained from measurement can be appropriately analyzed by those skilled in the art in accordance with the objective. For simple analysis, it can be sufficient to simply distinguish whether an object is present at a predefined position. It can be advantageous for an analysis to distinguish measurement information derived from a predefined position from measurement information derived from another region. A predefined position can be identified as a relative position from a substrate region (e.g., opening at the center of a circular substrate region) by identifying measurement information derived from the substrate region. Analysis can be performed even without spatial information on the predefined position. For example, analysis can be performed by using a signal that distinguishes a state where an interaction complex is formed from a state where an interaction complex is not formed. Possible examples thereof include a method of using the progression of an enzymatic reaction (e.g., detection of a product of the enzymatic reaction such as luminescence) as an indicator of the formation of an interaction complex by using a combination of unit molecules exerting an enzymatic function only if an interaction complex is formed, a method of using fluorescence intensity at a predefined wavelength as an indicator of the formation of an interaction complex by using a combination of unit molecules with a FRET donor and acceptor attached to positions that can be brought into proximity only if an interaction complex is formed, etc. The binding strength (may be binding affinity or binding frequency) of interaction complexes can also be measured by measuring the signal intensity. The competitive formation of interaction complexes can also be analyzed. A competing molecule may be added in a free state or linked to a substrate via a linker. The intensity of competition can be regulated by changing the linker position or linker length.

In one embodiment, the structure described herein can be used for determining a binding dissociation constant (K_{d}) between unit molecules of an interaction complex. Specifically, a method of determining K_{d} is provided in the Examples of the present application in detail. In the same system using the structure described herein, K_{d} between unit molecules with unknown K_{d} can be determined by referring to the signal intensity upon using unit molecules having known K_{d}. In one embodiment, a binding dissociation constant is determined based on calibration curves created from results of a reference unit molecule with a known binding dissociation constant. Typically, a unit molecule having unknown K_{d} and a unit molecule with a known K_{d} are tested in parallel and signal intensities are measured to determine the unknown K_{d}, but if a system with high accuracy of signal intensities measured can be constructed, K_{d} may be determined only by measuring a unit molecule having unknown K_{d}.

As described above, the structure described herein can achieve a high concentration of unit molecules, so that a binding dissociation constant (K_{d}) of weak interaction that is difficult to measure with a conventional method can also be determined. In one embodiment, the structure described herein can determine a binding dissociation constant (K_{d}) between unit molecules of an interaction complex of about 10⁻¹ to 10⁻¹⁵ M, such as about 10⁻¹ M, about 10⁻² M, about 10⁻³ M, about 10⁻⁴ M, about 10⁻⁵ M, about 10⁻⁶ M, about 10⁻⁷ M, about 10⁻⁸ M, about 10⁻⁹ M, about 10⁻¹⁰ M, about 10⁻¹¹ M, about 10⁻¹² M, about 10⁻¹³ M, about 10⁻¹⁴ M, about 10⁻¹⁵ M, or a value within the range between any two of said values.

For example, in order to determine the binding dissociation constant (K_{d}) between a first unit molecule and a second unit molecule, the first unit molecule and the second molecule are initially chemically modified to attach a linker or to be introduced with a attaching point to a linker (such as one of the pair of functional groups that can induce a click chemistry reaction). For the chemical modification, a known chemical reaction, such as an activated carboxyl group (such as NHS esterified carboxyl group) that can form a bond with NH₂ of a lysine residue can be utilized. In substrates with the same shape, the same type of physical quantity (such as light intensity) is measured for an interaction complex of interest by a first unit molecule and a second unit molecule, and a reference unit molecule with a known binding dissociation constant. The binding dissociation constant for the reference unit molecule and the physical quantity measured are associated, and the binding dissociation constant for the interaction complex of interest is calculated based thereon from the measured physical quantity for the interaction complex of interest.

K_{d} can be determined more accurately with improved accuracy of simulation on the behavior of a unit molecule. By detecting the respective signal intensities in a plurality of constructs having different D from one another, the behavior of a unit molecule (such as shape of unit molecule, probability of the orientation of the unit molecule, degree of freedom in movement of a linker, position of an interaction point between unit molecules) can be corrected to improve the accuracy of simulation.

In one embodiment, the analysis described herein can comprise the step of distinguishing a structure comprising an interaction complex from a structure free of an interaction complex. In one embodiment, the analysis described herein analyzes the binding force between unit molecules. In one embodiment, the analysis described herein can comprise analyzing results of measurement over time. For example, changes over time such as protein folding or successive reactions can also be analyzed by using the construct of the present disclosure.

If the position and/or orientation of a bond of a target biomolecule to another molecule is of interest, analysis can be facilitated by using a substrate having immobilization points for a unit molecule (target biomolecule and/or another molecule) at different positions. One substrate may have a plurality of immobilization points or a plurality of substrates with immobilization points at different positions from one another may be used. Labels causing the generation of different signals may be introduced to unit molecules or linkers attached to different immobilization points. The inhibition or promotion of the formation of an interaction complex can also be analyzed. For example, analysis using an optical signal is possible. In one embodiment, a system is constructed, wherein a first linker or a first unit molecule comprises a first functional group, a second linker or a second unit molecule comprises a second functional group, and light is generated or light with a specific wavelength is generated by an interaction between the first functional group and the second functional group. Typically, a structure is constructed so that a first functional group and a second function group interact when an interaction complex of interest is not formed, as a flip-flop detection mechanism. In one embodiment, a structure is constructed wherein a first linker or a first unit molecule, or a second linker or a second unit molecule, comprises a first functional group, a third linker comprising a second functional group is immobilized to a third linker immobilization point on a substrate, and light is generated or light with a specific wavelength is generated by an interaction between the first functional group and the second functional group. Typically, a structure is constructed so that a first functional group and a second functional group interact when an interaction complex of interest is not formed, as a switch detection mechanism.

In the analysis described herein, the formation of an interaction complex can be promoted by attaching a unit molecule to a substrate. Thus, an interaction complex which generally entails challenges in causing the formation, can also be readily formed and can be directly measured.

The analysis described herein such as those described above enables the development and design of a drug. If a complex of a target biomolecule such as a protein and a candidate drug molecule is caused to be formed, the method described herein can readily determine the binding form of the candidate drug molecule to the target biomolecule. Information on the binding strength can also be obtained concurrently therewith. For example, the position and orientation of a bond of a candidate drug molecule to a target biomolecule, etc. is information that can be readily obtained even with relatively low precision of analysis. Since a bound and free portions in a candidate drug molecule can be relatively easily distinguished, a pharmacophore can be extracted from the structure of the bond portion of the candidate drug molecule. If a plurality of candidate drug molecules that can interact with a target biomolecule can be identified, identification of a pharmacophore is further promoted. Information for further optimizing the drug structure can be accumulated by designing a variant with a modified binding portion in a candidate drug molecule and further performing the analysis described herein. Especially when a target biomolecule is known, analysis that takes into consideration the structure of the target biomolecule is possible. Analysis can be further facilitated by introducing a label to a portion in a candidate drug molecule that is not associated with the binding. If a lead compound that binds to a target biomolecule is obtained, those skilled in the art can design and synthesize related compounds based thereon. Meanwhile, the analysis described herein can provide useful information for determining the directionality of the design of a drug.

If the position and/or orientation of a bond of a candidate drug molecule to a target biomolecule is of interest, analysis can be facilitated by using a substrate having immobilization points for a unit molecule (target biomolecule and/or candidate drug molecule) at different positions. One substrate may have a plurality of immobilization points or a plurality of substrates with different immobilization point positions from one another may be used. Labels causing the generation of different signals may be introduced to unit molecules or linkers attached to different immobilization points.

In one embodiment, the method described herein can be used for screening for a drug. Screening for any drug associated with the formation of an interaction complex is possible. For example, drugs for inhibiting or promoting the formation of an interaction complex in vivo, drugs (glue drugs) that form an interaction complex via binding to different molecules in vivo, etc. can be screened. In one embodiment, screening for a drug comprises comparing states of the formation of an interaction complex (such as ratio of substrates comprising an interaction complex) between a condition under which a candidate drug is present and a control condition (such as condition under which a candidate drug is not present, condition under which a control drug is present, condition under which candidate drug is present at different concentration). In a typical embodiment of drug screening, measurement is taken in a state where a component (such as target protein) of an interaction complex other than the candidate drug molecule is attached to a substrate. An embodiment using a candidate drug molecule attached to a substrate and an embodiment using a free candidate drug molecule are both possible. For example, introducing a common functional group to a plurality of candidate drug molecules (especially to a portion that is not involved in the binding with a target molecule) and placing a moiety that specifically captures the functional group (capture moiety) on an attaching point on a substrate or linker attached to the substrate enables efficient screening for a drug in a state where the candidate drug molecule is attached to the substrate. Multiplex analysis, which uses a candidate drug molecule introduced with a plurality of types of functional groups such as those from a DNA-encoded library and a substrate comprising a capture moiety corresponding to each functional group, is also possible. In a typical embodiment of drug screening using a free candidate drug molecule, measurement is taken in a state where only a component (such as target protein) of an interaction complex other than the candidate drug molecule is attached to a substrate. A drug that inhibits or promotes the formation of an interaction complex can be screened based on signal intensity.

For example, a screening system using an optical signal is intended. Representative examples thereof include a flip-flop detection mechanism and switch detection mechanism described above.

In one embodiment, the analysis described herein can also screen for a drug by using a sample comprising a plurality of candidate drugs or a sample for which it is unknown whether a candidate drug is contained. A candidate drug that is present as a part of an interaction complex can be identified by obtaining measurement data that is sufficient to enable analysis of the formed interaction complex in detail.

More specific exemplary embodiments and advantageous of the analysis described herein will be described hereinafter to facilitate the understanding of the technologies of the present disclosure.

In one embodiment, the analysis described herein can find a candidate drug from a DNA-encoded library, identify a pharmacophore from the candidate drug, and design an additional drug. For example, a substrate is constructed using DNA origami, a single stranded DNA moiety (functional group) is provided as a linker attaching point on the substrate, and a target protein is attached via another linker attaching point on the substrate. By preparing a group of compounds from a DNA-encoded library with a DNA complemental to the single-stranded DNA moiety as candidate drugs, the candidate drugs can be screened using the same substrate (attached to a target protein). A candidate drug with an interaction complex formed at a high frequency can be identified as a compound bound to the target protein, and data for the formation of an interaction complex can be further collected by using the same system (optionally by applying a modification such as optimization of the linker length), whereby the binding form of the candidate drug to the target protein can be analyzed in more detail. The binding to the target protein may involve not only the portion of compound before modification with a DNA, but also a portion to which the DNA is added. The analysis described herein can be useful in understanding such a binding region in a candidate drug. It may be investigated whether an interaction complex is formed when a substrate of DNA origami is redesigned, the position of a linker attaching point is changed, and a candidate drug is brought into proximity to the target protein from another direction. By analyzing the binding form between a candidate drug and a target protein in this manner, a pharmacophore in a candidate drug can be identified, a candidate drug with a modification can be designed and made based thereon, and the binding affinity of such a variant to the target protein can be studied through the analysis described herein.

In one embodiment, the analysis described herein enables screening for an allosteric modulator. Since the analysis described herein can readily determine the presence/absence of the formation of an interaction complex, this can be suitable for such analysis. For example, a plurality of substrates of the same shape with a known protein and a known ligand thereof attached via respective linkers are prepared, and a signal originating from a predefined position is observed in the presence of each candidate compound. If the signal intensity decreases in the presence of a candidate compound relative to the signal intensity under a control condition, the compound can be determined as an allosteric inhibitor.

In one embodiment, the analysis described herein enables analysis of the binding form of a multi- binding molecule. While development of a multi-binding drug such as PROTAC (proteolysis targeting chimera) comprising a portion binding to a target protein to be degraded and a portion binding to ubiquitin E3 ligase has been attempted, the formation and analysis of an interaction complex requiring combining three or more components are more difficult. In the analysis described herein, the formation of an interaction complex can be promoted by attaching a unit molecule to a substrate. Thus, an interaction complex which generally entails difficulty in causing the formation thereof can also be readily formed and directly measured. Thus, the analysis described herein can efficiently provide information supporting the development of a multi-binding drug (glue drug) (also see Example 13).

In one embodiment, the structure described herein is used for performing an immobilized enzymatic reaction. The luminescence reaction in the Examples of the present application is due to an enzymatic reaction. This result can be interpreted such that the structure described herein affects the efficiency of an enzymatic reaction. Since the structure described herein can promote the formation of a complex, the formation of an enzymatic complex and the function thereof can be controlled by the design of the structure. A biosynthesis system and metabolic system of an organism produce a metabolic complex (biosynthesis complex) known as metabolon by weak interaction of enzymes with one another. This serves an important role in efficient intermediate substrate channeling between enzymes in some cases. For this reason, continuous enzymatic reactions can be efficient by utilizing the structure described herein and placing an enzyme at a predefined position to stabilize metabolon formation. For an enzyme that generally does not form a complex, the degree of promotion or inhibition of an enzymatic reaction can be controlled by placing an enzymatic reaction promoter or inhibitor in the structure described herein together with the enzyme. For example, in a system producing a substance of interest via a multi-stage enzymatic reaction, etc., flux of each enzymatic reaction step can affect the yield of the final substance of interest, so that it can be useful to control enzymatic reactions utilizing the structure described herein.

In one embodiment, the structure described herein can be used to capture a substance of interest (including proteins other than an interaction complex, etc.). Since the structure described herein has an internal space that can be designed, capturing a substance of interest can be promoted by adjusting the internal space to the size of the substance of interest. For example, a substance of interest can be selectively captured from a cell lysate comprising a plurality of proteins, mixture of agents, etc. A captured substance can be purified. For example, the structure described herein can be designed with a substance of interest as a unit molecule. In one embodiment, a structure has a plurality of linkers that can be bound to a substrate of interest through a form of bond other than a covalent bond and captures the substate of interest at a position (predefined position) where a large number of linkers can bind to the substance of interest. If, for example, such linkers are placed distributed equally inside a structure, a substance of interest can gradually move to the inside of the structure while repeating binding and dissociation.

### (Kit)

In one aspect, the present disclosure provides a kit for preparing the structure described herein. A kit comprises at least one component of a system for causing the formation of an interaction complex at a predefined position. In one embodiment, a kit comprises a substrate and optionally a linker of a system. In one embodiment, a kit comprises a user manual for performing the method described herein.

### (Other embodiments)

As described above, the present invention has been described while showing preferred embodiments to facilitate the understanding. While the present invention is described hereinafter based on Examples, the above descriptions and the following Examples are provided for the sole purpose of exemplification, not to limit the present invention. Thus, the scope of the present invention is not limited to the embodiments and Examples specifically described herein and is limited only by the scope of claims.

### [Examples]

Examples will be described hereinafter. For reagents, the specific products described in the Examples were used. However, the reagents can be substituted with an equivalent product from another manufacturer (Sigma-Aldrich, Fujifilm, Wako Pure Chemical, Nacalai Tesque, R & D Systems, USCN Life Science Inc., Kanto Chemical, Funakoshi, Tokyo Chemical Industry, Merck, etc.).

### (Example 1: Theoretical concept of interaction complex formation)

The technology of the present disclosure uses a member of a complex in a state of being attached to a substrate instead of a free state in order to cause the formation of a complex at a predefined position. A theory was constructed for handling the complex formation behavior in such a case.

### (Introduction of crossing index)

A system in which two types of particles A and B are connected to a substrate via a linker and bind by an interaction will be contemplated. Experimentally, there is a need for the probability of these particles binding to be at a certain level or higher. A design guideline for this purpose will be established by the theory discussed hereinafter.

### Binding probability and mean crossing rate of two particles

An increase in the binding probability in a system with particles connected via a linker will be quantitatively estimated. Since particles are in random thermal motion, the position of the particles at a certain time cannot be determined in this design. If an initial value is determined, classical simulation is possible, but the initial value thereof also cannot be determined. In this regard, even if the position cannot be determined, a probability distribution by time average can be considered. Thus, the binding probability is quantitatively calculated hereinafter by probabilistically handling the position of a particle.

Particles A and B are assumed to follow the probability density functions f_{A}(x) and f_{B}(x) for position x. In this system, the probability of the two particles binding will be considered. If the coordinates of the two particles are nearly the same, the particles will be deemed to be bound. In other words, if the distance is r or less, the particles will be deemed to be bound. This r is referred to as the interaction distance, and the binding probability of two particles is defined below. One-dimensional system is initially considered for simplicity.

If particle A is at position x, the probability of particle B being within distance r is given by the following:
[Numerical Formula 2] ${\int }_{x - r}^{x + r} dy {f}_{B} \left(y\right)$

If this is multiplied by f(x) and integrated with respect to x, the probability that the two particles are within distance r is calculated.
[Numerical Formula 3] ${\int }_{- ∞}^{∞} dx {f}_{Λ} \left(x\right) {\int }_{x - r}^{x + r} dy {f}_{B} \left(y\right)$

Since this can be written in a symmetric form with respect to x and y as described below if a rectangular function
[Numerical Formula 4] $rect \left(x\right) = \left\{\begin{matrix}0 & \left(\left|x\right|>1/2\right) \\ 1 / 2 & \left(\left|x\right|=1/2\right) \\ 1 & \left(\left|x\right|<1/2\right)\end{matrix}\right.$ is used, this will be used as the definition.

### Definition 1 (probability of two particles binding)

It is assumed that particles A and B follow the probability density functions f_{A}(x) and f_{B}(x) for position x. Then, the probability of particles A and B binding is defined as follows:
[Numerical Formula 5] $p = {\int }_{- ∞}^{∞} dx {\int }_{- ∞}^{∞} dy {f}_{A} \left(x\right) {f}_{B} \left(y\right) rect \left(\left(x-y\right)/2r\right)$ wherein r is the interaction distance.

It is known that a rectangular function, when modified to be narrower and longer, has a property similar to a delta function.
Theorem 1.

If
[Numerical Formula 6] ${ϕ}_{a} \left(x\right) = \frac{1}{a} rect \left(x/a\right)$ [Numerical Formula 7] ${\int }_{- ∞}^{∞} f \left(x\right) {ϕ}_{a} \left(x\right) dx \to f \left(0\right) \left(a\to 0\right)$ would hold for any function f(x) (satisfying a suitable property).

Using this, rect((x-y)/2r) within formula (4) can be deemed to be nearly equal to 2rδ(x-y) when r is sufficiently small. In other words, binding probability p can be expressed as
[Numerical Formula 8] $p = 2 r {\int }_{- ∞}^{∞} dx {\int }_{- ∞}^{∞} dy {f}_{A} \left(x\right) {f}_{B} \left(y\right) δ \left(x-y\right)$ by using a delta function.

By expanding the aforementioned discussion to three dimensions, the binding probability is given by the following formula by replacing the integral with a triple integral and 2r with v = 4πr³/3:
[Numerical Formula 9] $p = v \int {d}^{3} x \int {d}^{3} y {f}_{A} \left(x\right) {f}_{B} \left(y\right) {δ}^{3} \left(x-y\right)$ wherein v is referred to as the interaction volume. In this regard, the part other than v, i.e.,
[Numerical Formula 10] $\int {d}^{3} x \int {d}^{3} y {f}_{A} \left(x\right) {f}_{B} \left(y\right) {δ}^{3} \left(x-y\right)$ is not dependent on r or v and is a quantity that is automatically determined once the probability density functions f_{A}(x) and f_{B}(x) are given. This quantity will be considered. In view of the properties of δ functions, this can be transformed as follows: $\begin{matrix}\int {d}^{3} x \int {d}^{3} y {f}_{A} \left(x\right) {f}_{B} \left(y\right) {δ}^{3} \left(x-y\right) \left(10\right) \\ = \int {d}^{3} x {f}_{A} \left(x\right) {f}_{B} \left(x\right) \left(11\right)\end{matrix}$ This formula, in view of its form, multiplies the probability density functions for when particles A and B are at the same location and takes the integral thereof. Thus, this quantity is referred to as the mean crossing rate d. The probability density functions f_{A}(x) and f_{B}(x) can be calculated through a simulation from the attaching point between a substrate and a linker, length of the linker, estimated volumes of particles A and B, and information on the binding point between particles A and B.

Thus, the binding probability p defined initially can be expressed simply as $p = vd$ by using the interaction volume v and mean crossing rate d.

### Specific examples of mean crossing rate

In this section, the mean crossing rate is calculated for specific probability density functions. While a one-dimensional system will be contemplated for simplicity, the obtained results can be extended to a three-dimensional system in view of the same discussion.

### 1. Uniform distribution with offset positions

The following case wherein f_{A}(x) and f_{B}(x) are given with a uniform distribution in a finite section but the position thereof is offset will be discussed.

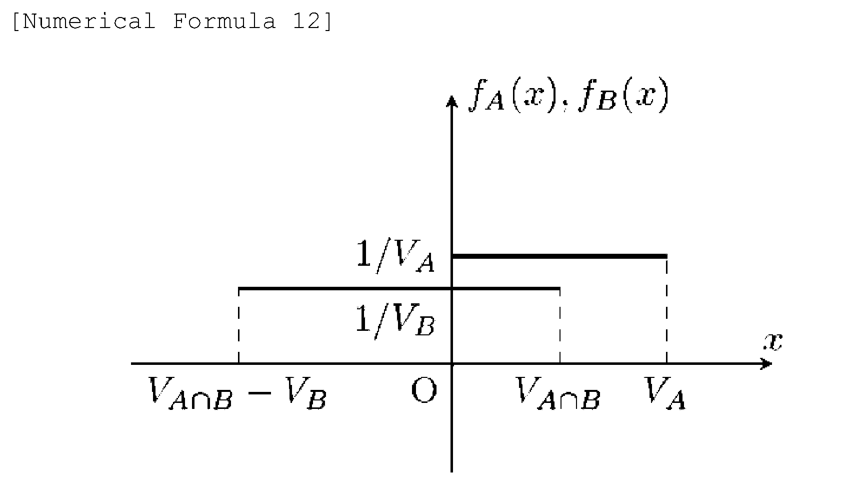

The sizes of the finite sections are V_{A} and V_{B}, and the size of the overlap in said sections is V_{A∩B}.

In this regard, f_{A}(x) and f_{B}(x) are uniformly distributed in the spaces of volumes V_{A} and V_{B}, so that the mean crossing rate can be calculated. $\begin{matrix}d = {\int }_{0}^{{V}_{A}} dx {\int }_{{V}_{A ∩ B} - {V}_{B}}^{{V}_{A ∩ B}} dy {f}_{A} \left(x\right) {f}_{B} \left(y\right) δ \left(x-y\right) \left(13\right) \\ = {\int }_{0}^{{V}_{A ∩ B}} dx {f}_{A} \left(x\right) {f}_{B} \left(x\right) \left(14\right) \\ = \frac{{V}_{A ∩ B}}{{V}_{A} {V}_{B}} \left(15\right)\end{matrix}$ Using formula (12) again, binding probability p is given by
[Numerical Formula 14] $p = \frac{{vV}_{A ∩ B}}{{V}_{A} {V}_{B}}$ While a one-dimensional system was used above for the calculation, the same conclusion is reached when extended to a three-dimensional system.

### Chemical equilibrium and mean crossing rate

In the discussion thus far, a bond was assumed to be determined only by its position without considering the binding energy between the particles. Meanwhile, a bond may provide benefit in terms of energy. Thus, hereafter binding energy will also be taken into consideration by considering statistical thermodynamic parameters. To this end, it will be confirmed hereinafter that the mean crossing rate still serves an important role even if the binding probability is calculated using a canonical distribution and binding energy for particles is considered. The ratio of particles that are bound is calculated by using canonical distribution. The number of unbound states and bound states and energy are given in the following table.

**[Table 1]**

| | **Number of states** | **Energy** |
|---|---|---|
| **Unbound** | (V(*A*)/*ν*) × (*V*(*B*)/*ν*) | 0 |
| **Bound** | *V*(*A* ∩ *B*)/*ν* | -*ε*_{AB} |

wherein ε_{AB} (>0) is the binding energy between particles A and B.

By using a canonical distribution, the binding probability can be calculated as 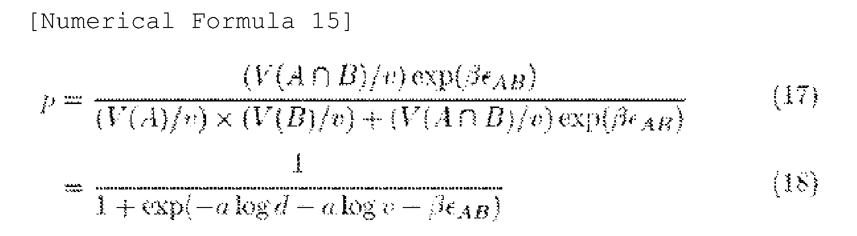 wherein a = 1/loge = 2.30 ...

In this regard, binding index X and crossing index D are defined by the following formulas.
[Numerical Formula 16] $X = - a log d - a log v - β {ϵ}_{AB}$ Then, the binding probability p is expressed as
[Numerical Formula 17] $p = \frac{1}{1 + exp \left(X\right)}$

Since the term -alogd in the formula for binding index X is a quantity that can be adjusted by the design of a structure, crossing index D is defined as follows: $D = - logd$ Since d can be any real number that is 0 or greater, the range of the values of crossing index D is any real number from - ∞ to ∞.

Plotting crossing index D on the horizontal axis and binding probability p on the vertical axis results in the following relationship. 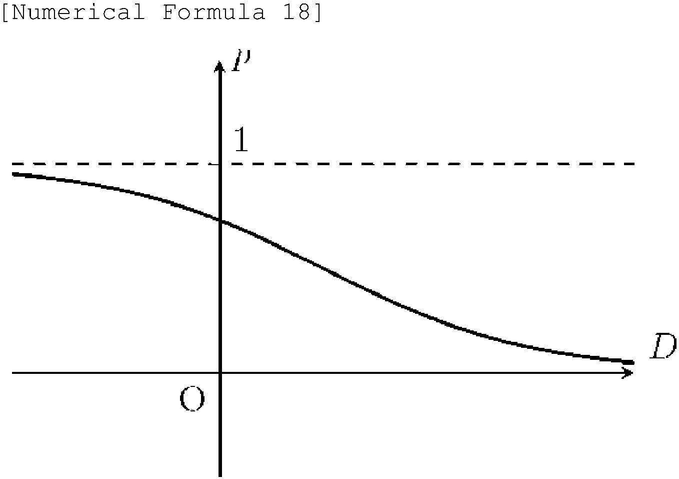
[Numerical Formula 18]
P
1
D
O
 wherein p at D = 0 can be expressed as follows:

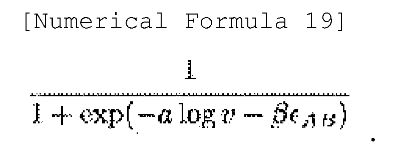

### Specific example of crossing index

### 1. Method of calculating the crossing index of particles attached to a substrate with a cylindrical internal space via a linker

A system with particles attached to the inside of a cylindrical space via a linker will be contemplated. It is assumed that the particles are spherical, and the point of interaction with another particle is located on the opposite side of the attaching point of the linker (Figure **4A**). In this regard, the probability distribution of an interactive point can be calculated through numerical simulation by determining the length of the linker and the radius of the particles.

Simulation of protein particles attached via a linker to the inside of DNA origami was performed through the following procedure.
1. The volume per one residue of amino acid was calculated as 0.141 nm³ from the mean value of the volume of all amino acids. The volume of the particle was calculated therefrom, and the particle size was calculated by assuming that the particle is spherical.
2. The size of the substrate and the length of the linker were determined.
3. The orientation of the particle was set to be random, and the linker wrapping around the particle and a wall of a cylindrical substrate were taken into consideration to calculate the probability distribution of possible positions of the interaction point for the particle (particle **1**) connected to the linker.
4. The same processing was also applied to the particle on the opposite side (particle **2**) attached to a attaching point at the same height to calculate the probability distribution.
5. The means cross rate d and the crossing index D were calculated using formula (11) and formula (21) by using the probability distributions for particle **1** and particle **2**.
6. The size of the substrate and the length of the linker were changed in 2. to repeat the calculations in 3.-5.

*In this simulation, the distance between a linker attaching point on the substrate and a contact point between the linker and the particle excluding the wrapping around portion of the linker was deemed to be distributed at a constant probability from length 0 to the length of the linker excluding the wrapped around portion. It was assumed that the particle was oriented to any direction at the same probability. Any D described herein may be determined under these conditions. Simulation that sets the range of movement of the particles in accordance with the detailed structure of the substrate was also possible.

As one example, the probability distribution at the cross-section of a cylindrical substrate at the height of a attaching point when the radius of the cylindrical substrate was 6.5 nm, the radius of the particle was 2 nm, and the length of the linker was 4 nm was as shown in Figure **4B**. The probability distribution at the cross-section of a cylindrical substrate at the height of a attaching point when another particle was immobilized to the opposite side of the cylindrical substrate via a linker, and the radius of the particle was 4 nm and the length of the linker was 4 nm, was as shown in Figure **4C**. Crossing index D in this regard, calculated using formula (11) and formula (21), was 2.91.

### Design guidelines

It is understood that a bond cannot be observed if the binding probability for an experiment is too low. In this regard, if the minimum value of binding probability at which bonds can be experimentally observed is assumed to pₘᵢₙ, the following inequality must hold:
[Numerical Formula 20] $\frac{1}{1 + exp \left(X\right)} \geq {p}_{min}$ By solving for crossing index D, this can be modified to
[Numerical Formula 21] $D \leq log v + \frac{β {ϵ}_{AB}}{a} + \frac{1}{a} log \left(\frac{1 - {p}_{min}}{{p}_{min}}\right)$

Thus, there is maximum value Dₘₐₓ for a certain D, and the system needs to be designed so that D is Dₘₐₓ or less in actual design. However, it is not necessary to design a substrate configuration each time in order to obtain a desired D. For example, the value of D can be changed widely by changing the length of a linker or the attaching position of the linker while using the same substrate. The range of the values of D that can be materialized by adjusting the length of a linker would be wider if the linker is attached to asymmetric positions in a substrate.

### (Example 2: Construction of substrate for causing the formation of an interaction complex)

In order to observe an interaction complex in the method of the present disclosure, a substrate for attaching a unit molecule and a linker was designed and constructed.

A complex of biotin and streptavidin was initially contemplated as an interaction complex, and a structure for structural observation thereof was studied. The binding dissociation constant between biotin and streptavidin is understood to be about K_{d} = 10⁻¹⁵ M, and the size of streptavidin (tetramer) is about 6 nm. Since the presence of biotin and streptavidin in a bound state in almost all structures is preferable for the observation of a complex, structures wherein biotin and streptavidin were in a bound state at a probability of 99% or higher were studied. As a result, it was considered that it is preferable to design a structure achieving D ≤ 13. If an interaction complex is formed at a position away from a wall of a structure, analysis focusing on streptavidin forming the interaction complex is considered to be facilitated, and thus it was preferable to design a structure achieving 0 ≤ D. In the following Examples, a cylindrical structure with an opening at the bottom portion was designed and used to enable observation from the bottom side of the structure where D = 3 within such a range of 0 ≤ D ≤ 13. The similar observation would be possible even if D is configured to be greater than 3 by shortening the linker length, etc.

### Synthesis of DNA origami substrate

### 1. Method of synthesis of CP52 monomers A and B, CP74, and CP76

Each of 500 µL of 400 nM template DNA (p7560 was used for CP52 monomers A and B and p8064 was used for CP74 and CP76) and staple DNA (217 and 219 types for CP52 monomers A and B, respectively, 238 types for CP74, and 248 types for CP76) was added at 10 equivalents to template DNA. See K. F. Wagenbauer, et al., ChemBioChem 2017, 18, 1873-1885. for the structure of such DNA origami. 5 mM Tris, 1 mM EDTA, and 5 mM NaCl buffer were used for dilution so that the final concentration of template DNA was 20 nM, the final concentration of each stable DNA was 200 nM, and the concentration of MgCl₂ was 20 mM. The reaction was conducted under controlled temperature by using a PCR thermal cycler. After the temperature was raised to 65°C and maintained for 15 minutes, the temperature was reduced to 60°C and allowed to slowly decrease 1°C per hour to 45°C. After the completion of the reaction, the solution was cooled to room temperature. The solution was centrifuged and concentrated while removing excessive staple DNA by using a 100 kDa Amicon tube.

After concentration of the reaction solution, the solution was purified by agarose electrophoresis. 1.5% agarose gel was used as the separation gel, and 0.5× TBE and 6 mM Mg(OAc)₂ were used as the electrolytic solution. When purifying CP76, 0.5× TBE and 2 mM Mg(OAc)₂ were used as the electrolytic solution. After the electrophoresis, the agarose gel portion containing the DNA origami of interest was cut out, then the DNA origami of interest was extracted from the agarose gel by using a Montage Gel Extraction Kit (Merck). After repeating the extraction operation three times with 5 mM Tris, 1 mM EDTA, 5 mM NaCl, and 5 mM MgCl₂ (2 mM MgCl₂ for CP76) solutions, the extracted solution was centrifuged and concentrated using 100 kDa Amicon^{®}. Measurements were taken on the absorbance of DNA origami solution obtained using Nanodrop^{®}, and the DNA origami concentration was determined from the value at A260. The DNA origami of interest was obtained at a yield of about 30 to 45%.

### 2. Method of synthesizing CP52

CP52 monomers A and B were mixed at equal amounts, and 5 mM Tris, 1 mM EDTA, and 5 mM NaCl buffer were used to adjust the final concentration of each DNA origami to 10 nM and the concentration of MgCl₂ to 20 mM. A tubular structure CP52 formed by the association between monomer A and monomer B was quantitatively obtained through a 24-hour reaction at 25°C. The formation of a structure was confirmed by performing electrophoresis with 0.5× TBE, 6 mM Mg(OAc)₂ buffer by using 1.5% agarose gel, then applying fluorescent staining to nucleic acids with SYBRGold (Figure **5**).

### (Example 3: Construction of unit molecules and linkers)

Unit molecules and linkers were designed and constructed in the following manner.

The following organic molecule-PEGX-BCN was synthesized in one step from a commercially available synthetic reagent and examined with TLC after an operation to separate solutions. The organic molecule-PEGX-BCN was then used in a click reaction with DNA without purification. For Halo-tag, total synthesis was performed based on the genetic information registered at NCBI (GenBank Accession number: AY773970).
*Biotin-PEG12-BCN 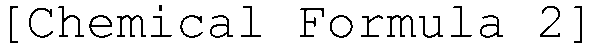 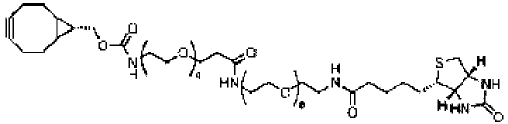
*Fmoc-GGG-PEG13-BCN 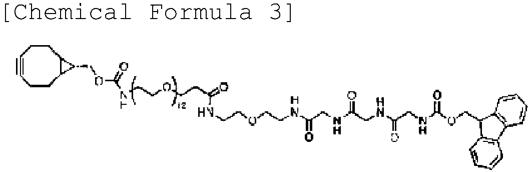
*HaloTag attaching molecule-BCN 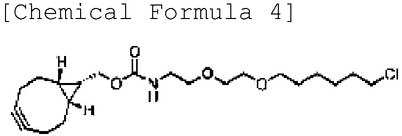
*HaloTag attaching molecule-PEG12-BCN 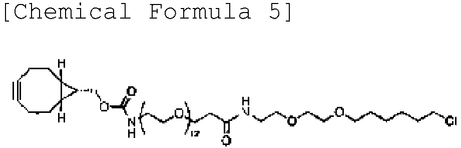
*BCN-PEG17-BCN 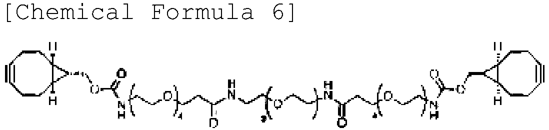
*BCN-PEG22-BCN

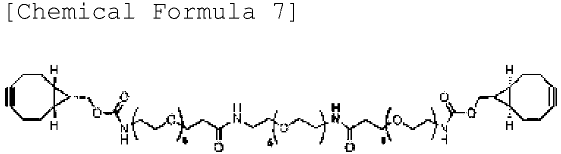

The following organic molecule-PEGX-N₃ was organically synthesized and examined through 1H NMR.
*HaloTag attaching molecule-N₃

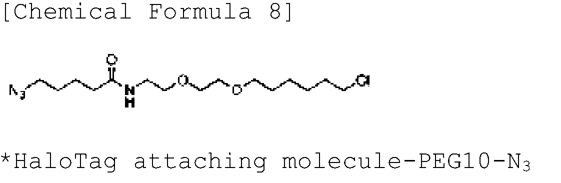

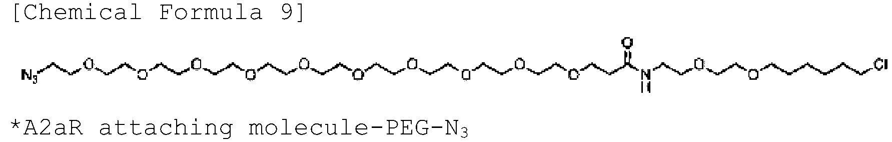

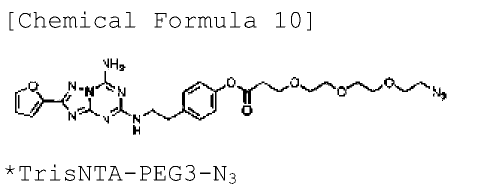

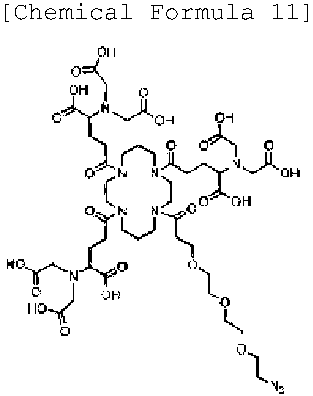

### 1. Synthesis of molecularly modified oligo-DNA by one-step click reaction

Organic molecule-PEGX-ssDNA was synthesized by linking organic molecule-PEGX-BCN and a single-stranded DNA (ssDNA) with an azide group introduced to the 3' or 5' terminus by a click reaction. 4 µL of 10 mM organic molecule-PEGX-BCN solution dissolved in DMSO or DMF and 40 µL of an aqueous 100 µM azide-modified DNA solution were mixed and reacted for 2 hours at 60°C to quantitatively obtain the organic-molecule-PEGX-ssDNA of interest. The formation of organic molecule-PEGX-ssDNA of interest was confirmed via electrophoresis using 20% polyacrylamide gel or electrophoresis using 5% agarose gel (PrimeGel Agarose PCR-Sieve HRS, Takara) in a TBE electrolytic solution. The molecules of interest in the gel can be made visible by detecting fluorescence from a fluorescent dye introduced into a single-stranded DNA (CY3 or CY5) or fluorescence from SYBR Gold bound on a single-stranded DNA from staining with SYBR Gold. Upon synthesis of Fmoc-GGG-PEG13-DNA, after linkage by a click reaction, piperidine was added to the reaction solution so that the final concentration was 5 v/v% and the mixture was incubated for one hour at room temperature for deprotection of Fmoc. After the deprotection reaction, an aqueous 0.5 M HCl solution was added at an amount equal to the added piperidine to neutralize and subsequently purify the reaction product. The synthesized organic molecule-PEGX-ssDNA was purified by one of the following two approaches.
1) Organic molecule-PEGX-ssDNA was directly extracted and purified from the reaction solution by using a commercially available DNA purification kit (NucleoTrap, Takara). After diluting the reaction solution with a binding buffer in a kit, silica bead suspension was added. The silica beads bound to the organic molecule-PEGX-ssDNA were washed once with binding buffer and twice with washing buffer, then the molecule of interest was extracted from the silica beads with a 5 mM Tris pH 8.5 buffer.
2) The post-reaction solution was subjected to agarose electrophoresis to separate the product of interest from unreacted raw materials, etc., and gel comprising the product of interest identified by fluorescence was cut out. The cut out gel was melted by heating, then extracted and purified with a commercially available DNA purification kit (NucleoTrap, Takara). A silica bead suspension was added to an aqueous solution with the melted gel. The silica beads bound to the organic molecule-PEGX-ssDNA were washed once with binding buffer and twice with washing buffer, then the molecule of interest was extracted from the silica beads with a 5 mM Tris pH 8.5 buffer.

Measurements were taken on the absorbance of the aqueous solution comprising the organic molecule-PEGX-ssDNA after purification, and the concentration of organic molecule-PEGX-ssDNA in the solution was determined from the value at A260 (Figure **6**).

### 2. Synthesis of molecularly modified oligo-DNA by two-step click reaction

BCN-PEGX-ssDNA was synthesized by linking BCN-PEGX-BCN and a single-stranded DNA (ssDNA) with an azide group at the 3' or 5' terminus by a click reaction. 4 µL of 10 mM BCN-PEGX-BCN solution dissolved in DMSO or DMF and 40 µL of an aqueous 100 µM azide-modified DNA solution were mixed and reacted for 2 hours at 60°C to quantitatively obtain the BCN-PEGX-ssDNA of interest. The formation of BCN-PEGX-ssDNA of interest can be confirmed via electrophoresis using 20% polyacrylamide gel or electrophoresis using 5% agarose gel (PrimeGel Agarose PCR-Sieve HRS, Takara) in a TBE electrolytic solution. The molecules of interest in the gel can be made visible by detecting fluorescence from a fluorescent dye modifying a single-stranded DNA (CY3 or CY5) or fluorescence from SYBR Gold bound on the single-stranded DNA from staining with SYBR Gold. The synthesized BCN-PEGX-ssDNA was purified by one of the following two approaches.
1) BCN-PEGX-ssDNA was directly extracted and purified from the reaction solution by using a commercially available DNA purification kit (NucleoTrap, Takara). After diluting the reaction solution with a binding buffer in a kit, silica bead suspension was added. The silica beads bound to BCN-PEGX-ssDNA were washed once with attaching buffer and twice with washing buffer, then the molecule of interest was extracted from the silica beads with a 5 mM Tris pH 8.5 buffer.
2) The post-reaction solution was subjected to agarose electrophoresis to separate the product of interest from unreacted raw materials, etc., and gel comprising the product of interest identified by fluorescence was cut out. The cut out gel was melted by heating, then extracted and purified with a commercially available DNA purification kit (NucleoTrap, Takara). A silica bead suspension was added to an aqueous solution with the melted gel. The silica beads bound to BCN-PEGX-ssDNA were washed once with binding buffer and twice with washing buffer, then the molecule of interest was extracted from the silica beads with a 5 mM Tris pH 8.5 buffer.

Measurements were taken on the absorbance of the aqueous solution comprising the BCN-PEGX-ssDNA after purification, and the concentration of BCN-PEGX-ssDNA in the solution was determined from the value at A260.

25 to 30 equivalents of N₃-organic molecules of BCN-PEGX-ssDNA were added to the resulting aqueous BCN-PEGX-ssDNA solution as a DMSO or DMF solution, and the mixture was reacted for 2 hours at 60°C to quantitatively obtain the organic molecule-PEGX-ssDNA of interest. The formation of organic molecule-PEGX-ssDNA of interest can be confirmed via electrophoresis using 20% polyacrylamide gel or electrophoresis using 5% agarose gel (PrimeGel Agarose PCR-Sieve HRS, Takara) in a TBE electrolytic solution. The molecules of interest in the gel can be made visible by detecting fluorescence from a fluorescent dye introduced into a single-stranded DNA (CY3 or CY5) or fluorescence from SYBR Gold bound on a single-stranded DNA from staining with SYBR Gold. The synthesized organic molecule-PEGX-ssDNA was purified by one of the following two approaches.
1) Organic molecule-PEGX-ssDNA was directly extracted and purified from the reaction solution by using a commercially available DNA purification kit (NucleoTrap, Takara). After diluting the reaction solution with a binding buffer in a kit, silica bead suspension was added. The silica beads bound to organic molecule-PEGX-ssDNA were washed once with binding buffer and twice with washing buffer, then the molecule of interest was extracted from the silica beads with a 5 mM Tris pH 8.5 buffer.
2) The post-reaction solution was subjected to agarose electrophoresis to separate the product of interest from unreacted raw materials, etc., and gel comprising the product of interest identified by fluorescence was cut out. The cut out gel was melted by heating, then extracted and purified with a commercially available DNA purification kit (NucleoTrap, Takara). A silica bead suspension was added to an aqueous solution with the melted gel. The silica beads bound to organic molecule-PEGX-ssDNA were washed once with binding buffer and twice with washing buffer, then the molecule of interest was extracted from the silica beads with a 5 mM Tris pH 8.5 buffer.

Measurements were taken on the absorbance of the aqueous solution comprising the organic molecule-PEGX-ssDNA after purification, and the concentration of organic molecule-PEGX-ssDNA in the solution was determined from the value of A260 (Figure **7**).

### (Example 4: Synthesis of protein (peptide)-DNA linked form) 1. Synthesis of protein-DNA linked molecule utilizing a HaloTag attaching reaction

5 equivalents of recombinant protein prepared from fusing a HaloTag to the C- or N-terminus of a target protein was added to 5 µM of HalogTag attaching molecule-PEGX-ssDNA, and the mixture was incubated on ice for one hour. The reaction solution was adjusted with 50 mM Tris, 100 mM NaCl, and pH 7.5 buffer. The protein, DNA, and protein-DNA linked molecule in the reaction solution were separated in a molecule size dependent manner by electrophoresis using 4 to 20% polyacrylamide gel, then fluorescence was detected from a fluorescent dye (CY3 or CY5) modified on ssDNA and the protein was stained with CBB stain to track the reaction. The reaction progressed quantitatively, consuming all of the HaloTag attaching molecule-PEGX-ssDNA, and the generation of corresponding protein-DNA linked molecule was able to be confirmed. The resulting solution was directly used in the protein encapsulation described below (Figure **8**).

### 2. Synthesis of protein (peptide)-DNA linked molecule via the enzyme sortase

20 equivalents (100 equivalents for a peptide) of recombinant protein (SmBit) prepared from introducing LPETGHHHHHH (SEQ ID NO: 10) to the C-terminus was added to 5 µM of GGG-PEG13-DNA, and the mixture was adjusted with 20 mM CaCl₂, 50 mM Tris, 100 mM NaCl, and pH 7.5 buffer. Lastly, 3 equivalents of modified sortase (Srt5M) were added to GGG-PEG13-DNA and the mixture was incubated on ice for 45 minutes. The reaction was quenched by adding EDTA or Ni-NTA agarose to the reaction solution. The protein, DNA, and protein-DNA linked molecule in the reaction solution was separated in a molecule size dependent manner by electrophoresis using 4 to 20% polyacrylamide gel, then fluorescence was detected from a fluorescent dye (CY3 or CY5) modified on ssDNA and the protein was stained with CBB stain to track the reaction. GGG-PEG13-ssDNA was consumed, and the generation of corresponding protein-DNA linked molecule was able to be confirmed. The resulting solution was directly used in the subsequent protein inclusion.

When synthesizing a peptide-DNA linked molecule, peptide-PEG13-ssDNA was directly extracted and purified from the reaction solution by using a commercially available DNA purification kit (NucleoTrap, Takara). After diluting the reaction solution with a binding buffer in a kit, silica bead suspension was added. The silica beads bound to peptide-PEG13-ssDNA were washed once with binding buffer and twice with washing buffer, then the molecule of interest was extracted from the silica beads with a 5 mM Tris pH 8.5 buffer. Measurements were taken on the absorbance of the aqueous solution comprising the peptide-PEG13-ssDNA after purification, and the concentration of peptide-PEG13-ssDNA in the solution was determined from the value at A260 (Figure **9**).

### (Example 5: Immobilization onto DNA origami)

1. Immobilization of streptavidin onto DNA origami by utilizing biotin
   10 equivalents of biotin-PEG12-ssDNA were added to DNA origami (CP52, CP74, or CP76), and the mixture was adjusted with 5 mM or 20 mM MgCl₂ (2 mM for CP76), 50 mM Tris, and 100 mM NaCl buffer and incubated at room temperature for two hours. After the completion of the reaction, excessive biotin-PEG12-ssDNA was removed by repeating centrifugation using a 100 kDa Amicon filter five times. Measurements were taken on the absorbance of the resulting solution, and the concentration of DNA origami in the solution was determined from the value at A260.
   5 equivalents of streptavidin were added to the resulting DNA origami (CP52, CP74, or CP76), and the mixture was adjusted with 5 mM or 20 mM MgCl₂ (2 mM for CP76), 50 mM Tris, and 100 mM NaCl buffer and incubated at room temperature for two hours. After the completion of the reaction, excessive streptavidin was removed by repeating centrifugation using a 100 kDa Amicon filter five times.
2. Immobilization of protein onto DNA origami by utilizing His-tag bond
   5 equivalents of NiCl₂ were added to the synthesized NTA-PEGX-ssDNA, and the mixture was adjusted with 5 mM Tris buffer and incubated at room temperature for one hour to prepare Ni-NTA-PEGX-ssDNA. 10 equivalents of Ni-NTA-PEGX-ssDNA were added to DNA origami (CP52, CP74, or CP76), and the mixture was adjusted with 5 mM or 20 mM MgCl₂ (2 mM for CP76), 50 mM Tris, and 100 mM NaCl buffer and incubated at room temperature for two hours. After the completion of the reaction, excessive Ni-NTA-PEGX-ssDNA was removed by repeating the centrifugation using a 100 kDa Amicon filter five times. Measurements were taken on the absorbance of the resulting solution, and the concentration of DNA origami in the solution was determined from the value at A260.
   2.5 equivalents of recombinant protein tagged with His-tag were added to the resulting DNA origami (CP52, CP74, or CP76), and the mixture was adjusted with 5 mM or 20 mM MgCl₂ (2 mM for CP76), 50 mM Tris, and 100 mM NaCl buffer and incubated on ice for two hours.
3. Immobilization of protein-DNA linked molecule onto DNA origami

10 equivalents of the synthesized protein (peptide)-ssDNA linked molecule were added to DNA origami (CP52, CP74, or CP76), and the mixture was adjusted with 5 mM or 20 mM MgCl₂ (2 mM for CP76), 50 mM Tris, and 100 mM NaCl buffer and incubated on ice for two hours. After the completion of the reaction, excessive protein (peptide)-ssDNA linked molecule was removed by repeating centrifugation using a 100 kDa Amicon filter five times. Measurements were taken on the absorbance of the resulting solution, and the concentration of DNA origami in the solution was determined from the value at A260.

### (Example 6: Measurement)

### TEM measurement

### 1. General method

A 2 nm carbon membrane coated 200 (R = 1.2/1.3) Cu TEM grid was hydrophilized, then 3 µL of aqueous solution comprising DNA origami was added onto the grid, left standing for 30 seconds, then removed with filter paper. 3 µL of 25% EM stainer solution was then added, left standing for one minute, then removed with filter paper, which were repeated twice (negative staining). The created TEM grid was dried for 60 minutes or longer, then the nanostructure was observed through a transmission electron microscope.

### 2. Method of obtaining a structure from a liquid-liquid bilayer interface under 2D sheet observation of CP76

A reaction solution prepared in 2-1 was added onto a heptacosafluorotributylamine solution to prepare a bilayer solution. A 2D sheet of CP76 was concentrated in the vicinity of the liquid-liquid interface by centrifugation. A hydrophilized TEM grid produced by the same method as 1 described above was inserted, to the heptacosafluorotributylamine solution. The grid was slowly moved, so as to scoop the top of the bilayer interface, from the heptacosafluorotributylamine layer to the aqueous solution, and a single-phase DNA origami sheet was transcribed onto the TEM grid. The resulting TEM grid was negatively stained by the same approach described above, dried for 60 minutes or longer, then the nanostructure was observed through a transmission electron microscope.

### Cryo-EM measurement

After hydrophilizing a 200 (R=0.6) or 300 (R=0.6) Cu TEM grid, 2.7 µL of aqueous solution comprising a DNA origami sheet or protein encapsulating DNA origami was added onto the grid and Vitrobot was used for rapid cooling with liquid ethane to create thin ice on the TEM grid. Thin ice forming conditions were optimized for each sample to be measured by considering the sample solution concentration, blotting time, and the presence/absence of addition of digitonin. Nanostructures were observed with a cryo-transmission electron microscope. Captured images were analyzed by using an open-source software Relion (https://github.com/3dem/relion). Correction of electron beam induced drifts, CTF estimation, particle picking, cutting out particle images, 2D class averaging, and 3D reconstruction were performed using the function within the software. As a result, a construct matching the size and shape of the introduced protein molecule was observed at a predefined position as designed, as shown in Figures **10** and **11**. The construct would not be observed unless designed properly.

### (Example 7: Formation of 2D sheet of DNA origami)

### 2D sheet forming reaction for CP76

Eight (total of 16) protruding ssDNAs with difference sequences from one another are placed on each of the top and bottom surfaces of DNA origami of CP76 (see the left side of Figure **12**). DNA origamis can be disposed regularly by adding crosslink wires having two types of sequences that would link corresponding protruding ssDNAs of adjacent CP76s at the termini. ssDNA-PEGX-ssDNA with asymmetric sequences were synthesized by a two-stage reaction in the same manner as organic molecule-PEGX-ssDNA as the crosslink wires. Commercially available BCN-C2-BCN, BCN-PEG2-BCN, and BCN-PEG4-BCN were used to synthesize the respective crosslink wires. For example, the synthesis method for ssDNA-PEG4-ssDNA is the following: 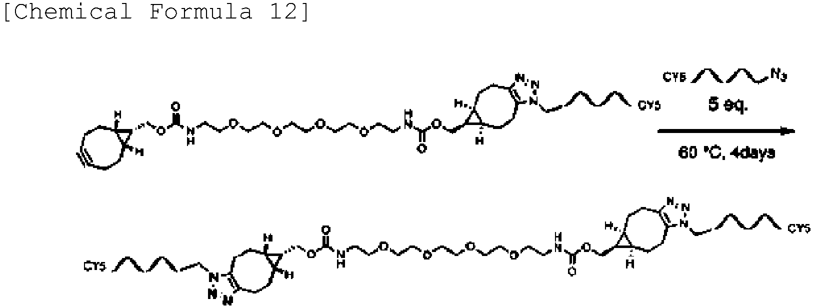 After synthesis, gel electrophoresis was performed for extraction and purification of ssDNA-PEG4-ssDNA of interest. Eight types of crosslink wires prepared in this manner were mixed at equal amounts with CP76, and the mixture was adjusted with 5 mM Tris, 1 mM EDTA, and 5 mM NaCl buffer so that the final concentration of CP76 would be 42 nM and the final concentration of MgCl₂ would be 10 mM. The solution was reacted for 2 days at 25°C while stirring in a microtube shaker. As a result, a regularly arranged DNA origami cluster was obtained (Figure **13**). By constructing DNA origami having ssDNA strands protruding out from the side surface to give a suitable complementarity between protruding ssDNA strands as shown on the right side of Figure **12**, a regularly arranged DNA origami cluster can also be formed without crosslink wires.

### (Example 8: Enzymatic reaction by a complex)

### Enzymatic luminescence reaction in a space within DNA origami by LgBit and SmBit

LgBit and SmBit were introduced into DNA origami (CP76) in the same manner as Example 5-3. With each of LgBit and SmBit, total synthesis of the gene was performed based on information in the following article: Dixon AS, et al., NanoLuc Complementation Reporter Optimized for Accurate Measurement of Protein Interactions in Cells. ACS Chem Biol. 2016 Feb 19; 11(2): 400-8. Each molecule can be immobilized at a determined position in a space within DNA origami by the sequence selectivity of ssDNA with LgBit and SmBit attached thereto. DNA origami was adjusted with 2 mM MgCl₂, 50 mM Tris, and 100 mM NaCl buffer so that the final concentration would be 1 nM based on the DNA origami concentration calculated from the value at A260. 50 µL of the adjusted aqueous 1 nM DNA origami solution and 50 µL of the aqueous solution from diluting a luminescent substrate (NanoGlo Luciferase Assay System, Promega) 100-fold with 2 mM MgCl₂, 50 mM Tris, and 100 mM NaCl buffer were mixed within a white plate and incubated at room temperature for 3 minutes. The luminescent intensity was measured using a plate reader (Infinite 500, Tecan).

For the pair of LgBit and SmBit with K_{d} = 190 µM, substantial luminescence was detected when both LgBit and SmBit were immobilized to DNA origami at 1 equivalent. Meanwhile, when one of LgBit and SmBit was added at 1 equivalent in a free state instead of being immobilized to DNA origami, the luminescence intensity was very low. The luminescence intensity was also very low even when the free LgBit was increased to 10000 equivalents.

While the volume of interaction complex in a volume of a space within a construct can be deemed as the concentration, the concentration of interaction complex in a construct made in this Example is very high relative to the normal concentration of protein dissolved in a solution. For this reason, the system provided in this Example can handle proteins that are stable at a high concentration, which cannot be achieved in other systems.

This Example shows that interactions between proteins with a weak binding at K_{d} = 190 µM can also be analyzed. There are many important biological phenomena to which interaction between proteins having a weak binding and a short duration are involved, such as transport of neurotransmitters. Thus, the present disclosure can also enable studying interactions between proteins with a weak binding.

The sequence of ssDNA attached to SmBit was then changed, and SmBit was introduced to a different location within DNA origami. Specifically, LgBit was immobilized at position B in Figure **14**, and SmBit was immobilized at position A, C, D, or E in Figure **14**. The results are shown on the right side of Figure **14**. The luminescence intensity changed depending on the position where SmBit was immobilized.

To study the effect of the length of a linker, LgBit or SmBit was linked to ssDNA (16 nt) with a PEG spacer of various lengths (1 nm, 4 nm, 7.6 nm, 9 nm, 10.7 nm, 12.5 nm, or 14 nm). The results are shown in Figure **15**. The luminescence intensity changed depending on the length of the linker. The luminescence intensity changed when the attaching member was switched between LgBit and SmBit under conditions of the same immobilization position on DNA origami and spacer length (Figure **16**).

The simulation disclosed in Example 1 was revised to set a space further reflecting the structure of a wall of DNA origami in place of a cylindrical space and to set the position of a linker attaching point to a position reflecting the actual linker attaching point. D was calculated and plotted using the results of Figures **14** to **16** (Figure **17**). Since the luminescence intensity and D are roughly correlated, the binding strength of an interaction complex of interest can be quantitatively estimated based on the design of a structure and measured luminescence intensity. Further, obtaining a large number of plots just like Figure **15** enables the adjustment of simulation used in the calculation of D and improvement in the precision of simulation in the system used.

As disclosed in Example 1, interaction can be quantitatively handled in the configuration of the present disclosure. Thus, in one aspect of the present disclosure, the binding force (K_{d}) between unknown interacting pair can also be provided. If, for example, binding probability p is experimentally calculated, K_{d} can be estimated from ${K}_{d} = \frac{1}{{ve}^{β {ϵ}_{AB}}}$ and formula (19) and formula (20) described above.

Specifically, this is derived in the following manner.

### A reaction expressed by

Reaction formula A + B ↔ AB
will be contemplated. Since this is a system with a variable number of particles, this is considered in terms of a grand canonical distribution. When the number of particles for particles A, B, and AB are expressed as N_{A}, N_{B}, and N_{AB}, respectively, and chemical potentials are expressed as µ_{A}, µ_{B}, and µ_{AB}, respectively, the grand partition function would be 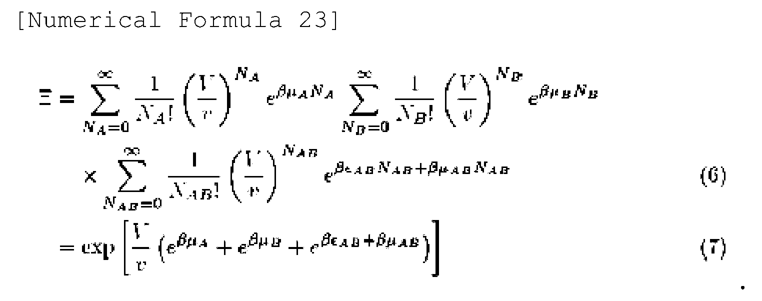 This is used to calculate the expected value of the number of particles to provide . 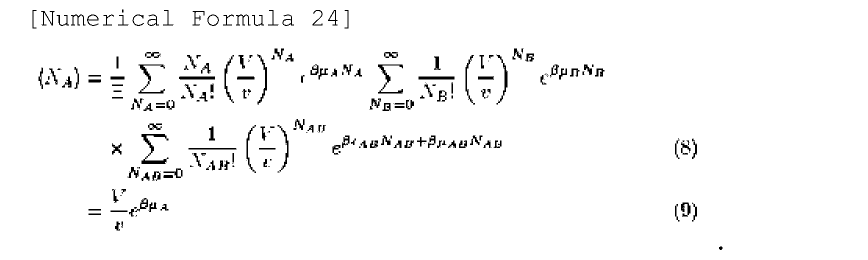 Likewise, the below is provided

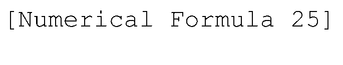

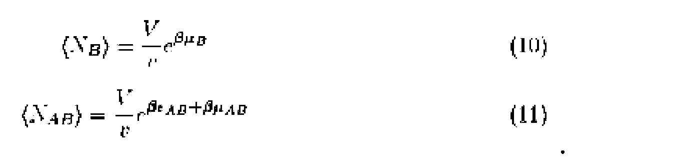

The dissociation constant K_{d} is defined as
[Numerical Formula 26] ${K}_{d} = \frac{\left[A\right] \left[B\right]}{\left[AB\right]}$ by using particle densities [A], [B], and [AB] in an equilibrium state. Since [A] = <N_{A}>/V, [B] = <N_{B}>/V, and [AB] = <N_{AB}>/V, substitution thereof together with formulas (9), (10), and (11) results in 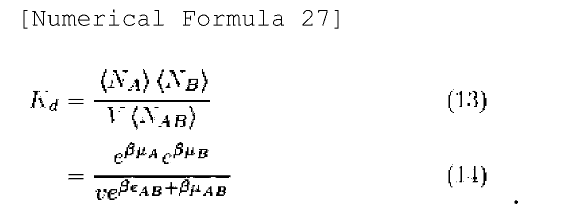 Since µ_{A} + µ_{B} = µ_{AB} must hold in an equilibrium state,
[Numerical Formula 28] 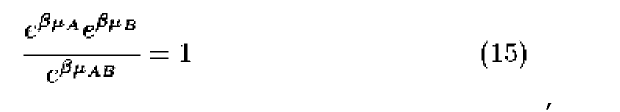 and this results in
[Numerical Formula 29]

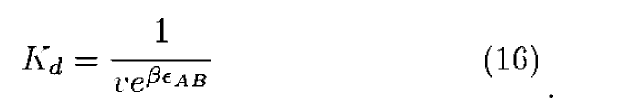

The construct of the present disclosure can analyze the interaction in a state where a unit molecule is captured in a specific compartment. Use of this characteristic enables the construction of a model that is far simpler than conventional analysis of measurement results for interaction between free molecules, drastic reduction in the computational load for analysis, and more detailed analysis thereby. The outline of simplification of the model is disclosed hereinafter.

The reaction rate in a two-component system is expressed by the following general formula.
[Numerical Formula 30] 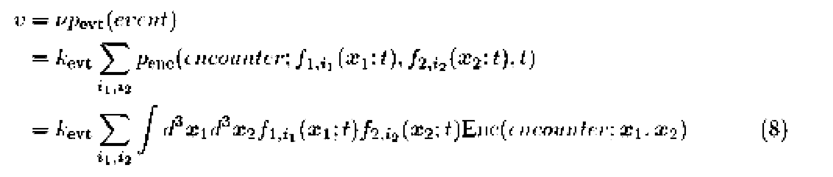 wherein i₁ and i₂ are labels for particles and encounter determining function Enc(encounter;x₁, x₂) is a function with a value of 0 or greater and 1 or less.

Particles are separated for each compartment, and the label of a particle is expressed as a set of (m, i) by using both m representing the index of a compartment and i representing the index of a particle within the compartment. By changing the labeling in this manner, formula (8) is expressed as follows: 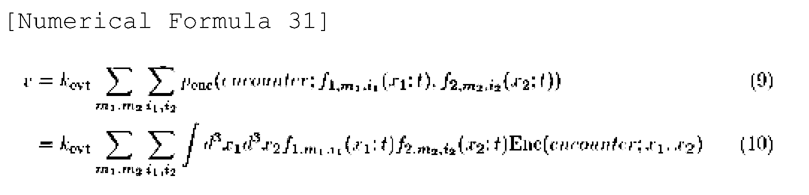 wherein ${f}_{1 , {m}_{1} , {i}_{1}} {f}_{2 , {m}_{2} , {i}_{2}}$ represent the probability distributions of the i₁th particle **1** within the m₁th compartment, and the probability distribution of the i₂th particle **2** within the m₂th compartment, respectively.

The formulas can be further modified to separate the contribution from a particle pair within the same compartment and the contribution from a particle pair between different compartments.

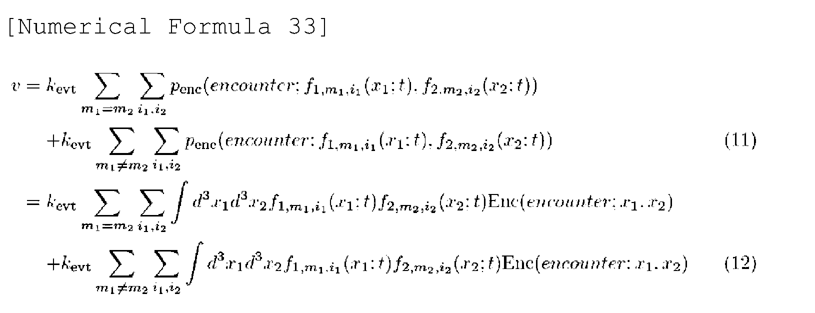

In particular, when considering a system with one each of particle 1 and particle 2 within a single compartment, this would be 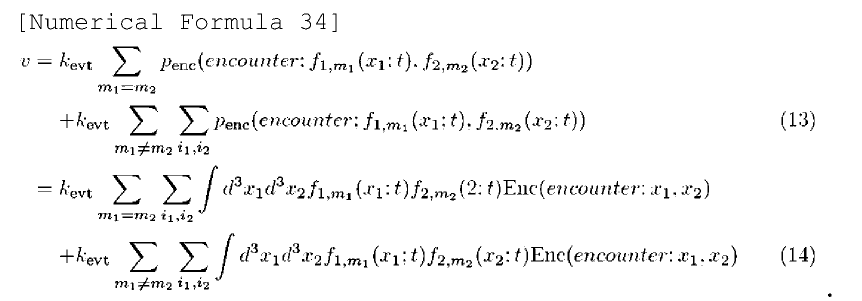 If the number of compartments is N, the number of terms in the first term is N, and the number of terms in the second term would be N² - N in formulas (13) and (14). Compartmentalization can limit interacting particles to be within the same compartment, whereby, if m₁ ≠ m₂,
[Numerical Formula 35] ${p}_{enc} \left(encounter; {f}_{1 , {m}_{1} , {i}_{1}}; {f}_{2 , {m}_{2} , {i}_{2}}\right) = 0$ would hold. Thus, the sum for m₁ and m₂ can be simplified, and the reaction rate is expressed as follows: 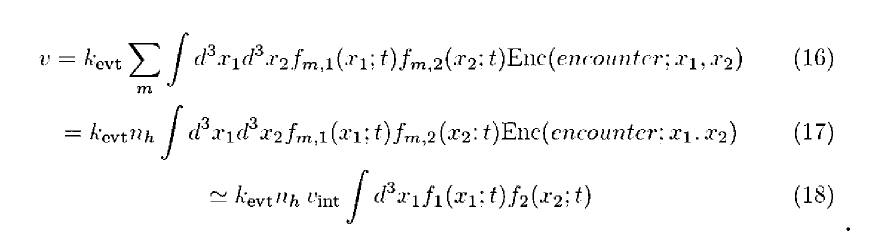 (On the second line, it was assumed that there are nₕ copies of the same structure as a compartment. On the last line, a method of approximating Enc with a δ function was used.)

In this manner, calculation resources can be drastically saved in comparison to embodiments considering the interaction between free molecules in free motion.

### (Example 9: Inhibition experiment using a construct)

DarkBit prepared from SmBit by replacing R with A in the sequence has the ability to bind to LgBit but does not promote a luminescent reaction, so that DarkBit functions as a competitive inhibitor that obstructs a luminescent reaction between LgBit and SmBit. DarkBit was used to conduct an inhibition experiment within a designed space.

For a pair of LgBit and SmBit with K_{d} = 190 µM, DarkBit was added at various concentrations in the four different arrangements shown in Figure **18** to evaluate the inhibition based on luminescence intensity. As a result, it was again observed that luminescence intensity between LgBit-SmBit varied in accordance with the type of arrangement, but IC₅₀ of DarkBit was constant at about 1.1 µM in any arrangement.

For a pair of LgBit and SmBit with K_{d} = 180 nM, DarkBit was added at various concentrations in the three different arrangements shown in Figure **19** to evaluate the inhibition based on luminescence intensity. As a result, IC₅₀ of DarkBit was constant at about 132 µM in any arrangement. In view of these results, if a construct of the present disclosure is used to cause the formation of a complex therein, the effect of the construct on the function/property of the complex would be low, and allow for biomolecular analysis mimicking a natural state, etc.

Inhibition by DarkBit was evaluated for SmBit with different K_{d} under the same arrangement. SmBit used and K_{d} thereof were the following: SmBit114 (K_{d} = 190 µM), SmBit104 (K_{d} = 2.5 µM), SmBit99 (K_{d} = 180 nM), SmBit78 (K_{d} = 3.4 nM), and SmBit86 (K_{d} = 0.7 nM). The results are shown in Figure **20**. It was found that a high concentration of DarkBit is required in order to inhibit the function of SmBit with low K_{d} and high binding strength.

### (Example 10: Derivation of K_{d} using a construct)

This Example shows that K_{d} can be derived by a method of the present disclosure based on measurement results for multiple substances (SmBit) with a known K_{d} value. The outline of the construct used in this Example is shown in Figure **21**.

### (Plots on a log-log graph)

The binding probability of particles A and B is given by $\begin{matrix}{p}_{AB} = \frac{{c}_{eff} / {K}_{d}}{1 + {c}_{eff} / {K}_{d}} & \left(1\right) \\ = \frac{{c}_{eff}}{{K}_{d} + {c}_{eff}} & \left(2\right)\end{matrix}$ by using the local effective concentration c_{eff} (nearly the same form as the Michaelis-Menten equation).

If c_{eff} <<K_{d,}
[Numerical Formula 38] ${p}_{AB} ≃ {c}_{eff} / {K}_{d}$ Taking the log of both sides results in
[Numerical Formula 39] ${log}_{10} {p}_{AB} ≃ {log}_{10} {c}_{eff} - {log}_{10} {K}_{d}$ With K_{d} on the horizontal axis and p_{AB} on the vertical axis, this would be on a straight line with a slope of -1 on a log-log graph. In this regard, if K_{d} is known, c_{eff} can also be calculated by fitting.

If c_{eff} >> K_{d}, $\begin{matrix}{p}_{AB} = 1 - \frac{{K}_{d} / {c}_{eff}}{1 + {K}_{d} / {c}_{eff}} \left(5\right) \\ ≃ 1 - {K}_{d} / {c}_{eff} \left(6\right) .\end{matrix}$ Thus, taking the log of both sides results in $\begin{matrix}{log}_{10} {p}_{AB} ≃ {log}_{10} \left(1-{K}_{d}/{c}_{eff}\right) \left(7\right) \\ = \frac{ln \left(1-{K}_{d}/{c}_{eff}\right)}{ln 10} \left(8\right) \\ ≃ - \frac{{K}_{d} / {c}_{eff}}{ln 10} \left(9\right) .\end{matrix}$ With K_{d} on the horizontal axis and p_{AB} on the vertical axis, this would be on a straight line on a semi-log graph.

### (The case where Hill coefficient is not 1)

Generally, the Hill coefficient is often not 1. In such a case, if the Hill coefficient is r, the equation is expressed as
[Numerical Formula 42] ${p}_{AB} = \frac{{\left({c}_{eff}/{K}_{d}\right)}^{r}}{1 + {\left({c}_{eff}/{K}_{d}\right)}^{r}}$ Thus, if c_{eff} << K_{d,}
[Numerical Formula 43] ${p}_{AB} ≃ {\left({c}_{eff}/{K}_{d}\right)}^{r}$ Thus, taking the log of both sides results in
[Numerical Formula 44] 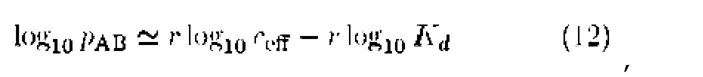 so that this would be on a straight line with a slope of -r on a log-log graph.

If c_{eff} >> K_{d}, 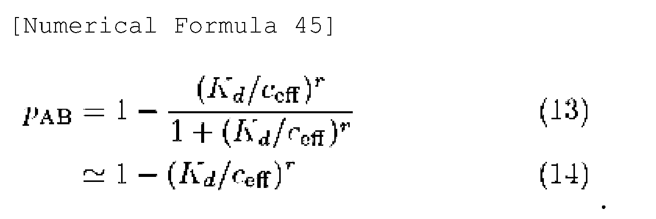 Thus, taking the log of both sides results in 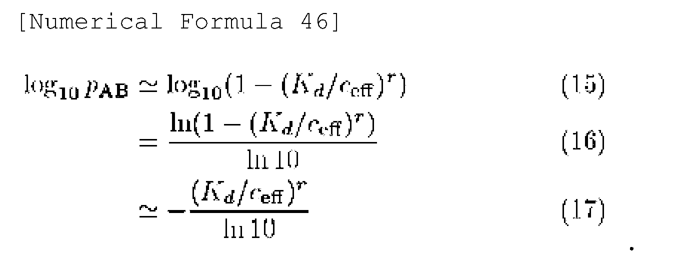 Thus, this would not be on a straight line when r = 1.

### (Results of fitting)

With x = log₁₀K_{d} on the horizontal axis and y = log₁₀(experimentally obtained luminescence value) = log₁₀p_{AB} + (constant) on the vertical axis, fitting was conducted as y = Ax + B. The binding dissociation for SmBit99 was calculated from the luminescence value for SmBit99 (K_{d} = 1.8 × 10⁻⁷ M) based on the corresponding relationship of binding dissociation with the luminescence values for SmBit114 (K_{d} = 1.90 × 10⁻⁴ M), SmBit104 (K_{d} = 2.5 × 10⁻⁶ M), SmBit78 (K_{d} = 3.4 × 10⁻⁷ M), and SmBit86 (K_{d} = 7.0 × 10⁻⁸ M). The calculation provided 2.3 × 10⁻⁷ M (linker length: 7.6 nm + 7.6 nm), 1.2 × 10⁻⁷ M (linker length: 1.0 nm + 7.6 nm), and 2.4 × 10⁻⁷ M (linker length: 14.2 nm + 7.6 nm), respectively, for the cases using three different structures. The results of plotting the luminescence values for SmBit99 on the vertical axis and the known binding constant K_{d} = 1.8 × 10⁻⁷ M thereof on the horizontal axis are also shown (Figure **22**). These results show that the method of the present disclosure was able to measure K_{d} that is very close to the known binding constant.

Design of a construct suitable for the measurement of K_{d} can also be envisioned. Since an approximate value of K_{d} for an interaction complex of interest is generally estimated, the range of D suitable for the measurement of actual K_{d} can be determined based on the estimated K_{d}.

In the most preferred embodiment, approximation with a straight line on a long-log graph is assumed upon fitting for the following discussion, but those skilled in the art can appreciate that fitting is also possible without necessarily using linear approximation.
From $p = \frac{d}{{K}_{d} + d} ,$ $d = \frac{{pK}_{d}}{1 - p} ,$ and if the lower limit of detectable binding rate p is set to pₘᵢₙ and the upper limit to pₘₐₓ in accordance with the detection method and characteristics of the analyzer, $d \geq \frac{{p}_{min}}{1 - {p}_{min}} {K}_{d} and \frac{{p}_{max}}{1 - {p}_{max}} {K}_{d} \geq d$ must hold. Combining them together results in $\frac{{p}_{max}}{1 - {p}_{max}} {K}_{d} \geq d \geq \frac{{p}_{min}}{1 - {p}_{min}} {K}_{d} .$ Definitional equation D = -logd results in $- log \left(\frac{{p}_{max}}{1 - {p}_{max}}{K}_{d}\right) \leq D \leq - log \left(\frac{{p}_{min}}{1 - {p}_{min}}{K}_{d}\right) .$

For the construct used in this Example, the simulation disclosed in Example 1 was altered so as to set a space better reflecting the structure of a wall of DNA origami instead of a cylindrical space and to set the position of a linker attaching point to a position reflecting the actual linker attaching point to calculate D, which yielded D in the range of approximately 4 to 5. Since the dynamic range of a detector was expected to be about 7 digits in the system used in this Example, it was confirmed that the design of the construct used in this Example was appropriate for providing the fitting results in Figure **22** which assumed a case of measuring an interaction complex of interest with K_{d} = about 1 × 10⁻⁷ M. In this manner, a construct that is suitable for measurement can be designed and chosen based on estimated K_{d} of the subject of measurement.

### (Example 11: Construction of a structure having luminescence intensity changing by addition of an inhibitor)

A structure that can detect a change in interaction between Talin 1 (TLN1) and integrin subunit beta (ITGB) upon addition of an inhibitor was constructed.

Two types of systems, i.e., flip-flop detection mechanism and switch detection mechanism, were constructed by using HaloTag, LgBit, SmBit, TLN1, and ITGB. Each of TLN1 (Uniprot ID: Q9Y490) and ITGB1 (Uniprot ID: P05556) was cloned from the registered genetic information. HaloTAg has three chemically modifiable locations at a ligand recognition site, C-terminus, and N-terminus. In this Example, a single-stranded DNA for attaching to DNA origami was connected to a ligand recognition site of HaloTag, a subunit of an interaction complex was connected to the C-terminus, and LgBit or SmBit was connected to the N-terminus.

Hereinafter, FIYM10 (KFEKEKMNCKWMT: SEQ ID NO: 11) was used as a drug for inhibiting binding between TLN1 and ITBG and FIYM46 (KAEKEKMNCKAMT: SEQ ID NO: 12) which is a variant that has lost said effect was used as a control drug.

### 1: Flip-flop detection mechanism

The outline of the system is disclosed in Figure **23**. When TLN1 and ITBG are in a bound state in this system, LgBit and SmBit are at spatially separated positions from each other, with a HaloTag interposed therebetween. In this regard, if an inhibitor is added, complex formation of TLN1 and ITBG is inhibited. As a result, a LgBit-SmBit complex can be formed, and a luminescence reaction progresses.

The results are shown in Figure **23**. When 40 µM of FIYM10 was added, an increase in the luminescence intensity was confirmed, but a change in the luminescence intensity was not observed upon addition of 40 µM of FIYM46. Thus, FIYM10 can be confirmed to function as an inhibitor.

### 2: Switch detection mechanism

The outline of this system is disclosed in Figure **24**. SmBit was placed at a position that is spatially away from other members in this sytem. When TLN1 and ITBG are in a bound state, LgBit and SmBit cannot bind. If an inhibitor is added, complex formation of TLN1 and ITBG is inhibited. As a result, a LgBit-SmBit complex can be formed, and a luminescence reaction progresses.

The results are shown in Figure **24**. In the same manner as a flip-flop detection mechanism, when 40 µM of FIYM10 was added, an increase in the luminescence intensity was confirmed, but a change in the luminescence intensity was not observed upon addition of 40 µM of FIYM46 in this system. Thus, FIYM10 can be confirmed to function as an inhibitor.

### 3: FRET/BRET detection mechanism

A detection mechanism utilizing a system wherein energy transfer occurs upon bringing a fluorescent protein or agent with a different excitation wavelength into proximity was also constructed. When TLN1 and ITGB are in a bound state in this system, a CFP fluorescent protein and an mCherry fluorescent protein are at proximal positions with a HaloTag interposed therebetween. In this regard, if an inhibitor is added, complex formation of TLN1 and ITGB is inhibited. As a result, the CFP fluorescent protein and mCherry fluorescent protein are sufficiently distanced, so that a FRET reaction is attenuated.

### (Example 12: Inhibitor screening)

An experiment for screening for an agent that inhibits interactions between Talin 1 (TLN1) and integrin subunit beta (ITGB) was conducted.

As a candidate drug for inhibiting the binding between TLN1 and ITBG, peptide inhibitors consisting of 13 amino acids prepared by modifying amino acids in an intracellular region of ITGB3 have been designed (Shen B. et al., A directional switch of integrin signaling and a new antithrombotic strategy. Nature 503, 131-135 (2013)). IC₅₀ values for the peptides shown in the following table were calculated by using the flip-flop detection mechanism described in Example 11 for optimizing the sequence. In this manner, the effect of a large number of compounds on the interaction with a target protein can be readily analyzed in the system of the present disclosed.

**[Table 2-1]**

| **No. (SEQ ID NO.)** | **Name** | **Inhibitory activity** | **Amino acid sequence** |
|---|---|---|---|
| 1 (11) | FIYM1 | 1050 = 708.7 uM | KFEKEKMNAKWDT |
| 2 (12) | FIYM2 | no inhibition | KAEKEKMNAKADT |
| 3 (13) | FIYM3 | no inhibition | ENMKEKKDWAKFT |
| 4 (14) | FIYM4 | no inhibition | KWDTGENPIYK |
| 5 (15) | FIYM5 | no inhibition | EFAKFEKEKMNAKWDT |
| 6 (16) | FIYM6 | no inhibition | KFEKEKMNAKWDTGENPIYK |
| 7 (17) | FIYM7 | no inhibition | EFAKFEKEKMNAKWDTGENPIYK |
| 8 (18) | FIYM8 | no inhibition | KFEKEKMNCKWDT |
| 9 (19) | FIYM9 | no inhibition | KFEKEKMNAKWMT |
| 10 (20) | FIYM10 | IC50 = 5.7 uM | KFEKEKMNCKWMT |
| 11 (21) | FIYM11 | no inhibition | KFEKEKMNCKWMK |
| 12 (22) | FIYM12 | no inhibition | KFEKEKMNCKWMR |
| 13 (23) | FIYM13 | no inhibition | KFEKEKMNCKWMpT |
| 14 (24) | FIYM14 | no inhibition | KFEKEKMNCKWMpS |
| 15 (25) | FIYM15 | IC50 = 41.2 uM | Ace-KFEKEKMNCKWMT-Me |
| 16 (26) | FIYM16 | no inhibition | RFEREKMNCRWMT |
| 17 (27) | FIYM17 | IC50 = 143.7 uM | Ace-PKFEKEKMNCKWMT |
| 18 (28) | FIYM18 | no inhibition | KF(-CI)EKEKMNCKWMT |
| 19 (29) | FIYM19 | IC50 = 211.5 uM | KF(-Me)EKEKMNCKWMT |
| 20 (30) | FIYM20 | no inhibition | KFERMNCKWMT |
| 21 (31) | FIYM21 | IC50 = 38.08 uM | 5-FAM-KFEKEKMNCKWMT |
| 22 (32) | FIYM22 | IC50 = 471.5 uM | KFEKEKMNCKWM |
| 23 (33) | FIYM23 | IC50 = 444.2 uM | FEKEKMNCKWMT |
| 24 (34) | FIYM24 | no inhibition | FEKEKMNCKWM |
| 25 (35) | FIYM25 | no inhibition | FERMNCKWMT |
| 26 (36) | FIYM26 | IC50 = 76.91 uM | RFEKEKMNCKWMT |
| 27 (37) | FIYM27 | IC50 = 94.70 uM | KFEREKMNCKWMT |

**[Table 2-2]**

| | | | |
|---|---|---|---|
| 28 (38) | FIYM28 | no inhibition | KFEKERMNCKWMT |
| 29 (39) | FIYM29 | no inhibition | KFEKEKMMCKWMT |
| 30 (40) | FIYM30 | no inhibition | RFEKERMNCKWMT |
| 31 (41) | FIYM31 | IC50 = 1044 uM | RFEKEKMMCKWMT |
| 32 (42) | FIYM32 | no inhibition | KFEREKMMCKWMT |
| 33 (43) | FIYM33 | no inhibition | KFEKERMMCKWMT |
| 34 (44) | FIYM34 | no inhibition | KFEKEKMNTKWMT |
| 35 (45) | FIYM35 | no inhibition | KFEKEKMNMKWMT |
| 36 (46) | FIYM36 | no inhibition | KFEKEKMNpSKWMT |
| 37 (47) | FIYM37 | IC50 = 140 uM | KFEKEKMNSeKWMT |
| 38 (48) | FIYM38 | no inhibition | KFEKEKMNC(Acm)KWMT |
| 39 (49) | FIYM39 | IC50 = 131.1 uM | KFEKEKMNC(Cam)KWMT |
| 40 (50) | FIYM40 | IC50 = 109.5 uM | KFEKEKMNC(tBu)KWMT |
| 41 (51) | FIYM41 | no inhibition | KFEKEKMNC(pMeBzl)KWMT |
| 42 (52) | FIYM42 | no inhibition | KFEKEKMNC(pMeOBzl)KWMT |
| 43 (53) | FIYM43 | no inhibition | KFEKEKMNC(Bzl)KWMT |
| 44 (54) | FIYM44 | IC50 = 1066 uM | KFEKEKMNC(Me)KWMT |
| 45 (55) | FIYM45 | IC50 = 313.7 uM | KFEEERARAKWDT |
| 46 (56) | FIYM46 | 1C50 = 474 uM | KAEKEKMNCKAMT |
| 47 (57) | FIYM47 | IC50 = 71.4 µM | MFEKEKMNCKWMT |
| 48 (58) | FIYM48 | 1050 = 291.5 µM | MKFEKEKMNCKWMT |
| 49 (59) | FIYM49 | 1050 = 107.2 µM | MHHHHHHKFEKEKMNCKWMT |
| 50 (60) | FIYM50 | 1050 = 1855 µM | KYEKEKMNAKWDT |
| 51 (61) | FIYM51 | 1050 = 1831 µM | KYEKEKMNCKWMT |
| 52 (62) | FIYM52 | 1C50 = 8127 *µ*M | DFEKEKMNCKWMT |
| 53 (63) | FIYM53 | 1C50 = 42045 *µ*M | EFEKEKMNCKWMT |
| 54 (64) | FIYM54 | no inhibition | PKFEKEKMNCKWMT |
| 55 (65) | FIYM55 | no inhibition | GPKFEKEKMNCKWMT |
| 56 (66) | FIYM56 | no inhibition | KFEKEKMNCKWRVTQENPIYK |

### (Example 12: Additional experiment)

While the substrate used in Figure 13, etc. is DNA origami having a nearly square opening, DNA origami (DB01) having a nearly rectangular opening was also studied to see whether a structure having a structure of interest was formed through a cryo-transmission electron microscope (Figure **25**).

A system of measuring the luminescence intensity similar to Example 9 was further investigated in DNA origami DB01 by using LgBit and SmBit with different K_{d}. The SmBit used and their K_{d} were the following: SmBit230 (did not bind), SmBit114 (K_{d} = 190 µM), SmBit104 (K_{d} = 2.5 µM), SmBit99 (K_{d} = 180 nM), SmBit78 (K_{d} = 3.4 nM), and SmBit86 (K_{d} = 0.7 nM). An embodiment wherein LgBit and SmBit were immobilized to DB01 via a linker (4 nm) was compared to an embodiment using them in a free state. The concentrations of LgBit and SmBit in the entire solution were configured to be the same under each condition (0.5 nm for both LgBit and SmBit). As a result, an improvement in luminescence intensity was observed in the immobilized state relative to the free state. It was observed that luminescence intensity was higher for higher binding strength of SmBit in an immobilized state (Figure **26**).

Since DB01 has a rectangular opening, the luminescence intensity can be adjusted by utilizing the difference in the lengths of the long side and short side of the rectangle. The difference in the luminescence intensity was studied by immobilizing LgBit and SmBit via a linker with a length of 4 nm or 9 nm at the positions of the pair of near midpoints of the two long sides or the pair of near midpoints of the two short sides in the opening of DB01. As a result, the actual measurement value of luminescence intensity exhibited a relative value similar to the predicted value in accordance with the theory in Example 1 (Figure **27**).

In the same manner as Example 9, inhibition experiment using DarkBit was also conducted in DB01. LgBit and SmBit were immobilized via a linker with a length of 4 nm or 9 nm at the positions of the pair of near midpoints of the two long sides in the opening of DB01, and luminescence intensity was measured in the presence of DarkBit at various concentrations. IC₅₀ of DarkBit as an inhibitor was calculated for each combination of linker lengths based on the measurement results, and the similar IC₅₀ value was obtained for each combination of linker lengths (Figure **28**). Although the absolute value of the luminescence intensity varied in accordance with the substrate and linker configurations, the resulting IC₅₀ value was similar. Thus, the physical structure of the construct in the measurement method of the present disclosure such as the substrate and linker would have little effect on the molecular property of DarkBit as an inhibitor. Simply adjusting the linker length would enable measurement under conditions that are suitable for a measurement system (conditions under which measurement values in a range with high quantitative reliability for the analytic instrument are obtained). Figure **29** shows the results of tracking the time-dependent transition in the inhibition curve upon addition of free DarkBit (inhibitor) from the outside to DB01 with LgBit and SmBit (ligands) attached thereto. The center column of Figure **29** shows the change in theoretical relative luminescence intensity when assuming each relationship between reaction rate (dissociation rate) constants for the inhibitor and ligand. For any of the relationships for reaction rate constants, the signal intensity when an equilibrium state is not achieved immediately after the addition of an inhibitor is different from the signal intensity when an equilibrium state is reached, but when the dissociation rate constant for an inhibitor is greater, it takes more time to reach an equilibrium state and the difference in signal intensity due to the difference in the timing of measurement can be greater. As demonstrated by the actual measurement results on the right side of Figure **29**, the time until reaching an equilibrium state for binding of an inhibitor molecule was observed to affect the calculated inhibition strength (IC50). A system using the construct of the present disclosure can analyze interactions even if such real-time measurement conditions are set while the required sample is a small amount and can also detect a small change in the interactive state.

### (Example 13: Example of an additional screening system)

While Example 11 discloses an example of the configuration of a construct in a measurement method of the present disclosure that can be used in screening for a drug, an additional configuration of a construct was studied (Figure **30**).

In this system, a molecule (glue molecule) promoting the formation of a complex of TLN1 and ITBG can be screened. A moiety comprising both ITBG and LgBit is immobilized inside the opening of a substrate via a linker (arm 1), and a linker attached to SmBit (arm 2) and a linker attached to TLN1 (arm 3) are immobilized to another location inside the opening of the substrate. Arms 1 to 3 are placed so that only the pair of LgBit and SmBit or the pair of TLN1 and ITBG can be simultaneously contacted, whereby the formation of a complex of TLN1 and ITBG can be promoted and luminescence from a complex of LgBit and SmBit can be reduced in the presence of a glue molecule. In the same manner as calculating an IC₅₀ value for an inhibitor, the intensity of a glue molecule promoting the formation of a complex of TLN1 and ITBG can also be quantitatively evaluated.

The structure disclosed in Figure **30a** was constructed and the luminescence intensity upon adding multiple peptides (Pep1, Pep2, and Pep3) as a candidate glue molecule at a concentration of 40 µM was actually measured. As a result, a change in luminescence intensity was not found by adding Pep1, whereas Pep2 and Pep3 were observed to result in a decrease in luminescence intensity (Figure **30b**). Further, a concentration-dependent decrease in luminescence intensity was observed upon changing the concentration of Pep3 added (Figure **30c**). Thus, the method of the present disclosure enables novel screening, which is screening for a glue molecule that promotes the formation of a complex.

Furthermore, Figure **31** shows the results from changing the binding strength between LgBit and SmBit by changing the type of SmBit. Through such an adjustment, a screening system capable of identifying a glue molecule having a desired level of activity at a particularly high sensitivity can be designed. A system can be adjusted by changing the structure (such as linker length, attaching position) of a construct without changing the molecule used.

A system for identifying a glue molecule based on the enhancement of luminescence intensity is also possible, as shown in Figure **32**.

### (Example 14: Modification of a linker)

While Example 8, etc. immobilized LgBit or SmBit to a substrate by using a HaloTag, a system using a linker (following structure: AccuLinker) comprising a SpyTag with less molecular weight was also made.

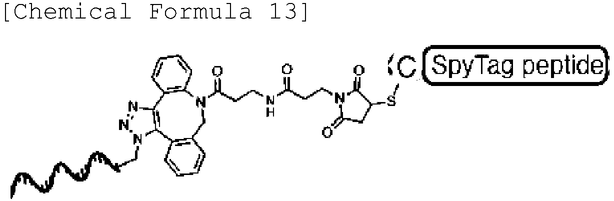

A linker comprising a photocleavable group with the following structure was then made.

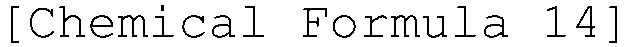

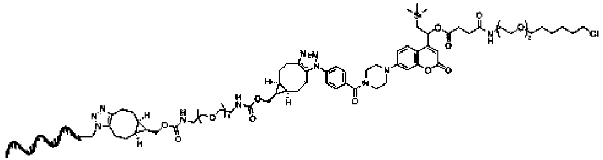

A photocleavable group-containing linker with SmBit-SpyTag attached thereto and a linker with LgBit-SpyTag attached thereto were immobilized to DB01 to test whether a linker is cleaved by light irradiation. As a result, as shown in Figure **33**, luminescence intensity decreased with longer light irradiation time when a photocleavable group-containing linker was used, but luminescence intensity did not change with light irradiation when a linker free of a photocleavable group was used. Thus, it was confirmed that a unit molecule which has been immobilized to a substrate can be subsequently detached by using a photocleavable group.

### (Example 15: Three-way competition system)

The similar configuration in Example 12 was constructed in a form where DarkBit was also further attached to the substrate via a linker instead of adding free DarkBit.

LgBit, SmBit, and DarkBit were attached to DB01 by using AccuLinker as shown in Figure **34**. As a result, the luminescence intensity changed by changing the linker length of DarkBit (Figure **34**). In this manner, the binding ability of a molecule in a substate can be adjusted to any degree with an inhibitor by choosing the linker length.

Furthermore, a system in accordance with the objective can be designed by changing the attaching position of a ligand (SmBit) or inhibitor (DarkBit) and linker length (Figure **35**).

### (Example 15A: Theory of construction of a three-way competition system)

The theory of designing a construct that is suitable for measurement in the three-way competition system described above will be contemplated.
It is assumed that
?the crossing index is D_{A} and binding dissociation constant is Kₐ for the LgBit-SmBit pair
?the crossing index is D_{B} and binding dissociation constant is K_{b} for LgBit-DarkBit pair.

In a three-way competition system, the binding rate p₁ for SmBit is given by
[Numerical Formula 52] ${p}_{1} = \frac{{d}_{A} / {K}_{a}}{1 + {d}_{A} / {K}_{a} + {d}_{B} / {K}_{b}}$ wherein d_{A} is the mean cross rate for the LgBiT-SmBiT pair and d_{B} is the mean cross rate for the LgBiT-DarkBiT pair. When the contributions of d_{A}/Kₐ and d_{B}/K_{b} are close, it is deemed suitable for experimental measurement. Thus, the ratio thereof as r_{AB} is defined by
[Numerical Formula 53] ${r}_{AB} = \frac{{d}_{A} / {K}_{a}}{{d}_{B} / {K}_{b}}$ . The crossing ratio index R is defined as follows:
[Numerical Formula 54] $R = log {r}_{AB}$

r_{AB}, when expressed using crossing indices D_{A} and D_{B}, is
[Numerical Formula 55] ${r}_{AB} = \frac{exp \left(-{D}_{A}\right) / {K}_{a}}{exp \left(-{D}_{B}\right) / {K}_{b}}$ Thus, providing
[Numerical Formula 56] $R = {D}_{B} - {D}_{A} + log \left({K}_{b}/{K}_{a}\right)$

In the absence of DarkBit, the formula
[Numerical Formula 57] ${p}_{1} = \frac{{d}_{A} / {K}_{a}}{1 + {d}_{A} / {K}_{a}}$ is given so that the ratio rₚ₁ of formula (1) to formula (6) (i.e., the retention rate of the binding rate of SmBiT in the presence of DarkBiT) is expressed as follows:

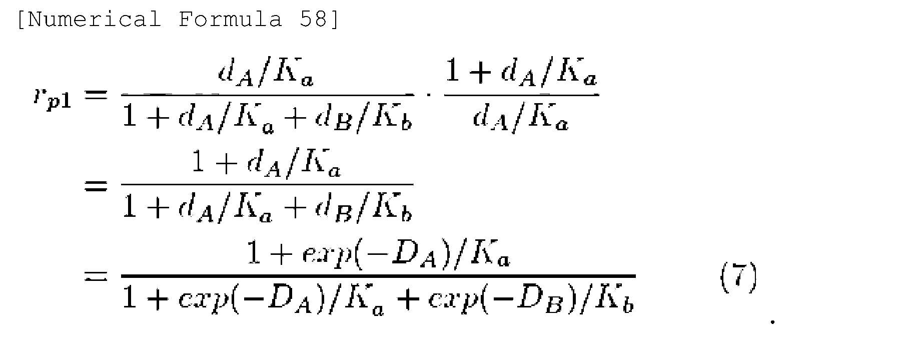

From formula (7), crossing index D_{B} for DarkBiT (inhibitor) to reduce the binding rate of SmBiT (ligand) placed in the configuration of specific crossing index D_{A} by x% can be calculated, i.e., this provides what configuration the inhibitor should be placed in when it is desirable to limit the activity of ligand at a specific ratio.

For example, even if an inhibitor is used whose binding dissociation constant K_{b} is not accurately studied and only the approximate value thereof is known, a configuration of a construct that allows the inhibitor to exert an inhibitory function (e.g., 1-99% inhibition) can also be set from formula (7). This concept can also be applied in screening for inhibitors.

### (Example 16: Fluorescence detection system)

While the formation of an interaction complex is detected by luminescence in the Examples described above, a system for detection using fluorescence was similarly constructed.

LgBiT, SmBiT99 (K_{d} = 180 nM), and HiBiT (K_{d} =700 pM) were connected within DNA origami DB01 by AccuLinker utilizing HaloTag. SmBiT99, HiBiT, 6-TAMRA, and BHQ1 were connected using 1 nm AccuLinker. For LgBiT, a rigit 20 bp dsDNA (about 7 nm) was connected within DB01 and LgBiT was connected to the tip thereof by using a 1 nm AccuLinker.

The outline of the system is disclosed in Figure **36**. A unit molecule (LgBiT) and AlexF488 of FRET donor were attached near the center of the long side of the inner cavity of DNA origami DB01, and the other unit molecule SmBit99 was attached near the center of the short side of the inner cavity via a linker. In the case of forming a complex between LgBiT and SmBit99, FRET accepter 6-TAMRA and quencher BHQ1 were attached to a position in contact with AlexF488. Constructs wherein SmBit99 is attached to both (C) or one (6-TAMRA side: A, BHQ1 side: B) of the short sides or not attached (Cont) were made (top part of Figure **37**). Fluorescence was measured at an excitation wavelength of 450 nm and detection wavelength of 580 nm.

The results are shown in the bottom part of Figure **37**. Higher fluorescence intensity was observed in the constructs of A and C compared to Cont, and lower fluorescence intensity was observed in the construct of B. Fluorescence intensity from the construct of A was higher than that from the construct of C. Since fluorescence intensity is deemed to be proportional to the probability of AlexF488 and 6-TAMRA being at proximal positions, it can be understood that localization of LgBiT within the construct is affected based on the interaction between LgBiT and SmBit. When fluorescence was measured by replacing SmBit99 with HiBiT, the difference in the fluorescence intensity ratios between the constructs of A and B widened, as disclosed in Figure **38**. Thus, it can be understood that this can be utilized to deduce K_{d} even with measurement of fluorescence.

While it is not necessary to construct a system wherein luminescence can also be detected at the same time detecting fluorescence, LgBiT and SmBit were used as unit molecules in this example, so that luminescence intensity was also measured (bottom part of Figure **37**). Since luminescence is generated by the complex formation between LgBiT and SmBit, it exhibited a behavior that is different from fluorescence based on the interaction between AlexF488 and 6-TAMRA. When luminescence was measured by replacing SmBit99 with HiBiT (K_{d} = 700 pM) (bottom right portion of Figure **37**), the luminescence intensity increased. Thus, it can be understood that a certain ratio of SmBit99 molecules is present in a free state in this configuration. In this manner, a system that detects various interactions separately can also be designed.

### [Industrial Applicability]

The present disclosure can be unutilized in the formation of an interaction complex, etc.
*SEQ ID NO: 1: complementary nucleic acid sequence
   GCAATCAATCAACAAC
*SEQ ID NO: 2: complementary nucleic acid sequence
   CTTCTACTTACACCAC
*SEQ ID NO: 3: complementary nucleic acid sequence
   CTTCTCCATTTTCCAG
*SEQ ID NO: 4: complementary nucleic acid sequence
   GTGCGTTCTATCTTCC
*SEQ ID NO: 5: complementary nucleic acid sequence
   GGTGATGATGGTTTGG
*SEQ ID NO: 6: complementary nucleic acid sequence
   CACGTAACAAGATCCC
*SEQ ID NO: 7: complementary nucleic acid sequence
   GTGATTTGAAAGTTGC
*SEQ ID NO: 8: complementary nucleic acid sequence
   TAATATGGACGGGAAA
*SEQ ID NO: 9: complementary nucleic acid sequence
   TTTTCTACTCTTCACG
*SEQ ID NO: 10: amino acid sequence introduced with a His-tag
   LPETGHHHHHH
*SEQ ID NO: 11: FIYM10
   KFEKEKMNCKWMT
*SEQ ID NO: 12: FIYM46
   KAEKEKMNCKAMT

## Claims

1. A method of making a structure for causing the formation of an interaction complex,
wherein the structure comprises a substrate comprising a first linker immobilization point and a second linker immobilization point,
wherein the first linker immobilization point and the second linker immobilization point can bind to or are attached to a first linker and a second linker, respectively, and the first linker and the second linker can bind to or are attached to a first unit molecule and a second unit molecule, respectively,
wherein a relative positional relationship between the first linker immobilization point and the second linker immobilization point is fixed,
wherein the first unit molecule and the second unit molecule can together form an interaction complex, and
wherein the method comprises a step of designing the structure based on the interaction complex.

2. The method of claim 1, wherein the step of designing the structure comprises designing the structure which is structured so that a crossing index for the first unit molecule and the second unit molecule is -5 *≤* D *≤* 15.

3. The method of claim 1, wherein the step of designing the structure comprises designing the structure based on sizes of the first unit molecule and the second unit molecule.

4. The method of claim 1, wherein the step of designing the structure comprises:
designing the structure based on a predicted value of a binding dissociation constant between the first unit molecule and the second unit molecule.

5. The method of claim 1, wherein the step of designing the structure comprises determining lengths of the first linker and the second linker based on at least one of: sizes of the first unit molecule and the second unit molecule; and a binding dissociation constant between the first unit molecule and the second unit molecule.

6. The method of claim 1, wherein the step of designing the structure comprises determining a distance between the first linker immobilization point and the second linker immobilization point based on at least one of: sizes of the first unit molecule and the second unit molecule; and a binding dissociation constant between the first unit molecule and the second unit molecule.

7. The method of claim 1, wherein the step of designing the structure comprises choosing the substrate from a plurality of options based on sizes of the first unit molecule and the second unit molecule.

8. The method of claim 1, wherein the step of designing the structure comprises:
choosing the substrate from a plurality of options;
determining a distance between the first linker immobilization point and the second linker immobilization point; and
determining lengths of the first linker and the second linker.

9. A method of analyzing an interaction complex, comprising the steps of:
making the structure according to the method of claim 1;
preparing a structure comprising the first unit molecule attached to the first linker immobilization point via the first linker and the second unit molecule attached to the second linker immobilization point via the second linker; and
analyzing the interaction complex.

10. The method of claim 9, of determining a binding dissociation constant between the first unit molecule and the second unit molecule.

11. The method of claim 10, wherein the binding dissociation constant (K_{d}) is within a range of 10⁻² to 10⁻⁶ M.

12. The method of claim 10, of determining the binding dissociation constant based on calibration curves created from a result for a reference unit molecule with a known binding dissociation constant.

13. The method of claim 9, of determining a structure of the interaction complex.

14. A method of conducting an immobilized enzymatic reaction, comprising the steps of:
making the structure according to the method of claim 1; and
contacting the structure with a substrate for an enzymatic reaction;
wherein the interaction complex is an enzyme.

15. A structure for causing the formation of an interaction complex,
wherein the structure comprises a substrate comprising a first linker immobilization point and a second linker immobilization point,
wherein the first linker immobilization point and the second linker immobilization point can bind to or are attached to a first linker and a second linker, respectively, and the first linker and the second linker can bind to or are attached to a first unit molecule and a second unit molecule, respectively,
wherein a relative positional relationship between the first linker immobilization point and the second linker immobilization point is fixed, and
wherein the first unit molecule and the second unit molecule can together form an interaction complex.

16. The structure of claim 15, wherein the structure is structured so that a crossing index for the first unit molecule and the second unit molecule is -5 *≤* D *≤* 15.

17. The structure of claim 15 made according to the method of claim 1.

18. A method of analyzing an interaction complex, comprising the steps of:
(A) providing a structure for causing the formation of an interaction complex at a predefined position, the structure comprising:
a substrate comprising a first linker immobilization point and a second linker immobilization point;
a first linker and a second linker attached to the substrate via the first linker immobilization point and the second linker immobilization point, respectively; and
a first unit molecule and a second unit molecule attached to the first linker and the second linker, respectively;
wherein a relative positional relationship between the first linker immobilization point and the second linker immobilization point is fixed,
wherein the first unit molecule and the second unit molecule can together form an interaction complex,
wherein the predefined position is defined as a relative position with respect to the substrate, and
wherein the interaction complex is formed at the predefined position;
(B) causing the formation of the interaction complex; and
(C) analyzing data derived from the interaction complex at the predefined position to analyze the interaction complex.

19. The method of claim 18, further comprising the step of irradiating a particle beam or an electromagnetic wave at the predefined position.

20. The method of claim 18, further comprising the step of collecting a particle beam or an electromagnetic wave from the interaction complex.

21. The method of claim 20, wherein the step of collecting an irradiated particle beam or an electromagnetic wave comprises using a cryogenic electron microscope.

22. The method of claim 18, of analyzing a three-dimensional structure of the interaction complex.

23. The method of claim 18, wherein data collected from a plurality of the structures is used to analyze a three-dimensional structure of the interaction complex.

24. The method of claim 18, further comprising the step of distinguishing the structures comprising the interaction complex and the structures free of the interaction complex.

25. The method of claim 24, of analyzing a binding force between the first unit molecule and the second unit molecule.

26. The method of claim 18, further comprising the step of:
attaching the first unit molecule to the first linker; and/or
attaching the second unit molecule to the second linker.

27. The method of claim 18,
wherein the substrate further comprises a third linker immobilization point, a third linker is attached to the substrate via the third linker immobilization point, and a third unit molecule is attached to the third linker,
wherein a relative positional relationship between the first linker immobilization point, the second linker immobilization point, and the third linker immobilization point is fixed,
wherein the first unit molecule, the second unit molecule, and the third unit molecule together form the interaction complex at the predefined position.

28. The method of claim 18, wherein the linker comprises a nucleic acid material.

29. The method of claim 18, wherein at least one of the first linker and the second linker has a capture moiety that non-covalently binds to the first unit molecule and/or the second unit molecule.

30. The method of claim 18, wherein the substrate is comprised of a nucleic acid material.

31. The method of claim 18, wherein the substrate comprises an opening.

32. The method of claim 31, wherein the predefined position is located at the opening.

33. The method of claim 18, wherein the substrate has a tubular structure or a plate-like structure.

34. The method of claim 18, wherein the substrate comprises a cap.

35. The method of claim 34, wherein the cap is a polymer with a mesh structure.

36. The method of claim 35, wherein at least one of the first linker immobilization point and the second linker immobilization point is on the cap.

37. The method of claim 18, wherein the first unit molecule and the second unit molecule are linked by a linker.

38. The method of claim 18, wherein the structure is structured so that a crossing index for the first unit molecule and the second unit molecule is 0 *≤* D *≤* 10.

39. A method of screening for a drug, comprising the steps of:
(A) providing a structure for causing the formation of an interaction complex at a predefined position, the structure comprising:
a substrate comprising a first linker immobilization point and a second linker immobilization point;
a first linker and a second linker attached to the substrate via the first linker immobilization point and the second linker immobilization point, respectively; and
a first unit molecule and a second unit molecule attached to the first linker and the second linker, respectively;
wherein a relative positional relationship between the first linker immobilization point and the second linker immobilization point is fixed,
wherein the first unit molecule and the second unit molecule can together form an interaction complex,
wherein the predefined position is defined as a relative position with respect to the substrate, and
wherein the interaction complex is formed at the predefined position;
(B) causing the formation of the interaction complex in the presence of a candidate drug; and
(C) analyzing data derived from the interaction complex at the predefined position to analyze an interaction between the candidate drug and the interaction complex.

40. A method of screening for a drug, comprising the steps of:
(A) providing a structure for causing the formation of an interaction complex at a predefined position, the structure comprising:
a substrate comprising a first linker immobilization point and a second linker immobilization point;
a first linker and a second linker attached to the substrate via the first linker immobilization point and the second linker immobilization point, respectively; and
a first unit molecule and a second unit molecule attached to the first linker and the second linker, respectively;
wherein the first unit molecule comprises a candidate drug,
wherein a relative positional relationship between the first linker immobilization point and the second linker immobilization point is fixed,
wherein the first unit molecule and the second unit molecule can together form an interaction complex,
wherein the predefined position is defined as a relative position with respect to the substrate, and
wherein the interaction complex is formed at the predefined position;
(B) causing the formation of the interaction complex; and
(C) analyzing data derived from the interaction complex at the predefined position to analyze the interaction complex.

41. The method of claim 40, comprising the steps of:
attaching the first unit molecule to a linker comprising a capture moiety capable of attaching to the first linker immobilization point; and
attaching the first linker attached to the first unit molecule to the first linker immobilization point to form the structure.

42. The method of claim 39 or 40, further comprising the step of collecting a particle beam or an electromagnetic wave from the interaction complex.

43. The method of claim 39 or 40, wherein a state where the interaction complex is formed is distinguished from a state where the interaction complex is not formed.

44. The method of claim 39 or 40, wherein the first linker or the first unit molecule comprises a first functional group, the second linker or the second unit molecule comprises a second functional group, and an interaction between the first functional group and the second functional group generates light or light of a specific wavelength.

45. The method of claim 39 or 40, wherein the first linker or the first unit molecule, or the second linker or the second unit molecule, comprises a first functional group, a third linker comprising a second functional group is immobilized to a third linker immobilization point on the substrate, and an interaction between the first functional group and the second functional group generates light or light of a specific wavelength.

46. The method of claim 39 or 40, of screening for a drug that inhibits or promotes the formation of the interaction complex.

47. A method of designing a drug, comprising the steps of:
(A) providing a structure for causing the formation of an interaction complex at a predefined position, the structure comprising:
a substrate comprising a first linker immobilization point and a second linker immobilization point;
a first linker and a second linker attached to the substrate via the first linker immobilization point and the second linker immobilization point, respectively; and
a first unit molecule and a second unit molecule attached to the first linker and the second linker, respectively;
wherein a relative positional relationship between the first linker immobilization point and the second linker immobilization point is fixed,
wherein the first unit molecule and the second unit molecule can together form an interaction complex,
wherein the predefined position is defined as a relative position with respect to the substrate,
wherein the interaction complex is formed at the predefined position, and
wherein the first unit molecule comprises a starting drug molecule;
(B) causing the formation of the interaction complex;
(C) analyzing data derived from the interaction complex at the predefined position to extract a pharmacophore of the starting drug molecule; and
(D) designing a drug by modifying the pharmacophore.

48. The method of claim 47, wherein the substrate comprises the first linker immobilization point at a plurality of different positions.

49. The method of claim 47, wherein the substrate comprises a plurality of types of substrates comprising the first linker immobilization point at positions that are different from each other.

50. The method of claim 49, further comprising the step of classifying data for the particle beam or electromagnetic wave by each substrate having a linker attached thereto via the first linker immobilization point at different positions.
